(19) Europäisches Patentamt
European Patent Office
Office européen des brevets

(11) EP 4 335 840 A1

(12) EUROPEAN PATENT APPLICATION

(43) Date of publication:
13.03.2024 Bulletin 2024/11

(21) Application number: 22194915.9

(22) Date of filing: 09.09.2022

(51) International Patent Classification (IPC):
*C07D 257/02* (2006.01) *C07F 5/00* (2006.01)
*A61K 51/04* (2006.01)

(52) Cooperative Patent Classification (CPC):
C07D 257/02; A61K 49/106

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: **Bayer Aktiengesellschaft**
**51373 Leverkusen (DE)**

(72) Inventor: **The designation of the inventor has not yet been filed**

(74) Representative: **BIP Patents**
**c/o Bayer Intellectual Property GmbH**
**Alfred-Nobel-Straße 50**
**40789 Monheim am Rhein (DE)**

# (54) NEW CONTRAST AGENTS FOR USE IN DIAGNOSTIC IMAGING

(57) The present invention relates to a new class of compounds of general formula (I), to the metal chelates with a metal ion suitable for computed tomography thereof, to the $Gd^{3+}$ chelate complexes thereof, to methods of preparing said compounds, and to the use of said compounds as contrast agents in diagnostic imaging, such as in magnetic resonance imaging (MRI) and computed tomography (CT).

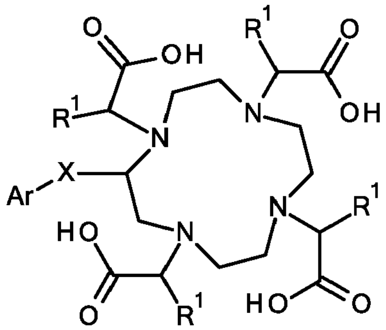

(I)

EP 4 335 840 A1

## Description

### FIELD OF THE INVENTION

The present invention relates to the items characterized in the patent claims, i.e. to new gadolinium metal chelate compounds, particularly metal chelate compounds suitable for computed tomography, to methods of preparing said compounds, to the use of said compounds as contrast agents in diagnostic imaging such as magnetic resonance imaging (MRI) or computed tomography (CT) and to their use in a mammalian body.

### BACKGROUND

#### 1. Introduction

The Magnetic Resonance Imaging (MRI) technique is non-invasive and can provide information on the anatomy, function and metabolism of tissues in vivo. Unenhanced MRI scans of tissue anatomy and function make use of the hydrogen atoms in water to generate the image. Apart from differences in the local water content, the basic contrast in the MR image mainly results from regional differences in the intrinsic relaxation times T1 and T2, each of which can be chosen to dominate image contrast. However, the intrinsic contrast provided by the water T1 and T2 and changes in their values brought about by tissue pathology are often too limited to enable a sensitive and specific diagnosis. To overcome these limits the proton relaxation times can be influenced by the presence of paramagnetic ions. Commercial Gadolinium-based Contrast Agents (GBCAs) contain at least one paramagnetic ion of the rare earth metal Gadolinium ($Gd^{3+}$), which possesses the highest number of unpaired electrons of any stable ion (seven), creating a high magnetic moment that is effective at enhancing proton relaxation.

Paramagnetic contrast media shorten the T1 (longitudinal) and T2 (transversal) relaxation times of surrounding water protons to indirectly produce a signal-enhancing effect. The efficacy of an agent to shorten relaxation times is called relaxivity (r1 and r2), which is dependent on the ligand surrounding the $Gd^{3+}$ ion and influenced by extrinsic factors including temperature, magnetic field strength and the matrix (water, solid tissue, or blood) (Lauffer RB et al., Paramagnetic metal complexes as water proton relaxation agents for NMR imaging: theory and design. Chem Rev. 1987;87(5):901-27; Caravan P et al., Gadolinium(III) chelates as MRI contrast agents: structure, dynamics, and applications. Chem Rev. 1999;99(9):2293-352).

Besides non-targeted agents, two linear contrast agents (i.e., contrast agents where ethe Gadolinium ion is bound to a linear ligand structure) are available for Magnetic Resonance Imaging (MRI) of the liver: Gd-BOPTA (gadobenic acid, marketed as Multihance) and Gd-EOB-DTPA (gadoxetic acid, marketed as Primovist in Europe and as Eovist in the USA). Gd-EOB-DTPA (Bayer AG) and Gd-BOPTA (Bracco) have been approved for detection and differentiation of focal liver lesions at clinical doses of 0.025 mmol/kg body weight and 0.05 mmol/kg body weight, respectively. For both agents the T1 effect dominates and yields bright contrast in the image. Both targeted agents have been used for MRI of the liver, for the detection of focal liver lesions in patients with known or suspected primary liver cancer (e.g. hepatocellular carcinoma HCC) or metastatic disease. They provide information regarding lesion vascularity in the arterial and venous phases, hepatocyte presence and function in the delayed hepatobiliary phase. The liver specific contrast agents are taken up by healthy liver cells (hepatocytes) while there is no uptake into malignant tumor tissue due to the lack of intact organic anion-transporting polypeptide (OATP) transporters. Therewith they improve the detection of focal liver lesions by increasing the lesion-to-liver contrast. The contrast-enhanced MRI (CE-MRI) provides important information for differential diagnosis. HCCs do not express the respective uptake transporters and thus do not accumulate the liver specific GBCA to an extent which is observed in healthy liver tissue.

Gd-BOPTA is secreted 3-to-5% into the bile and enables the capture of images in the liver-specific phase 1 to 2 hours after its administration (Seale MK et al. Radiographics 2009; 29: 1725-1748).

In the early 2000's, research for new contrast agents with improved tropism for liver cells led to the development of Gd-EOB-DTPA. Gd-EOB-DTPA is excreted into the bile up to about 50% of the administered dose. Gd-EOB-DTPA can be administered as a bolus, guarantees a satisfactory assessment of the vascular interstitial phase and subsequently (after 10-20 minutes), an evaluation of the hepatobiliary phase. Literature studies have confirmed that liver MRI with Gd-EOB-DTPA is able to detect and identify focal liver lesions with high specificity and sensitivity, either in patients with a healthy liver or oncologic/cirrhotic liver patients (Fidler J et al. Hepatology 2011; 53 (2): 678-82; Park Y et al. Korean J Radiol 2010; 11(4): 433-40; Bluemke DA et al. Radiology 2005; 237: 89-98).

Gd-EOB-DTPA is mainly taken up by hepatocytes via organic anion-transporting polypeptides (OATP1B1, OATP1B3) and is subsequently primarily excreted into the bile canaliculi via the multidrug resistance-associated protein 2 (MRP2, synonym cMOAT: canalicular multispecific organic anion transporter) (Ringe KI et al. American Journal of Roentgenology. 2010;195: 13-28; Leonhardt M et al. Drug Metab Dispos. 2010 Jul;38(7): 1024-8).

The molecular structure of both marketed products (Gd-EOB-DTPA and Gd-BOPTA) includes a hydrophilic

and lipophilic group. The linear DTPA-like ligand for the Gadolinium complexation comprises the hydrophilic group and the benzene (e.g. ethyl-oxy-benzyl, EOB) side chain is the lipophilic group. The lipophilic group is not only responsible for the marked biliary excretion but also results in some weak protein binding of approximately 10% (Weinmann HJ et al. Magn Reson Med 1991; 22:233-237). Compared to commercially available extracellular GBCAs, Gd-EOB-DTPA and Gd-BOPTA show higher r1 and r2 relaxivities (Rohrer M et al. Invest Radiol. 2005 Nov;40(11):715-24). The high relaxivity values depend on the affinity of the molecules with plasma proteins through the lipophilic group, which is also responsible for specific hepatocyte uptake.

Some recent reports have shown an increased signal intensity (SI) in the dentate nucleus (DN) and globus pallidus (GP) brain areas on unenhanced T1-weighted (T1w) MR images in patients with normal renal function who received multiple linear gadolinium-based contrast agent (GBCA) administrations. A visible SI increase is highly associated with linear GBCAs only. So far, this does not correlate with any clinical symptoms. However, in Europe, the European Medicines Agency (EMA) proposed to withdraw GBCAs with a linear structure from the market, except for both liver-specific market products Gd-BOPTA - if exclusively used in the liver - and Gd-EOB-DTPA. In March 2016, the Pharmacovigilance Risk Assessment Committee (PRAC) of EMA started a procedure to review linear GBCAs and recommended, in March 2017, a suspension of the marketing authorization for all multi-purpose linear GBCAs in the European Union (EU) while supporting the continued use of macrocyclic GBCAs (i.e., contrast agents where ethe Gadolinium ion is bound to a macrocyclic ligand structure). Following this decision, the GBCA market has broadly moved away from linear Gadolinium-based agents. As of early 2022, liver specific GBCAs (Gd-EOB-DTPA and Gd-BOPTA) are still accepted, because no alternative liver specific macrocyclic GBCA is commercially available. The observed increased signal intensity in the dentate nucleus was also seen after repeated administration of the linear Gd-EOB-DTPA (Kahn J et al. Radiology. 2017;282(3):708 - 716). It is known that macrocyclic GBCAs are more stable against Gd release (Frenzel et al. Invest Radiol. 2008 Dec;43(12):817-28.) Thus, there is an increased medical need for new macrocyclic liver-specific contrast agents. In particular, there is an increased medical need for new macrocyclic liver-specific contrast agents suitable for magnetic resonance imaging.

Computed tomography is a non-invasive imaging technique to visualize anatomy and function of the human body using ionizing radiation. The signal intensity bases on the X-ray attenuation characteristics of the tissue and the contrast originates from differences in the to attenuate X-rays among different tissues. Unenhanced CT offers a strong signal contrast between bones and soft tissues as the calcium in the bones attenuates X-rays effectively. A second strong CT contrast exist between soft tissue and air-containing structures as the attenuation of such structure as the respiratory system is rather low. On the other hand, the CT contrast between different soft tissue is low. Therefore, contrast media that locally increase the X-ray attenuation were used (Clauss W, Speck U. Historical development of x-ray contrast media for urography and angiography. In: Vogl T, ClaußW, Li GZ, et al., eds. Computed tomography Berlin Heidelberg, Germany: Springer; 1996:1-11).

All x-ray contrast media approved for intravascular use are iodine-containing monomeric or dimeric substances containing 1 or 2 triiodobenzene cores. These contrast media are formulated with high iodine concentration (150-400 mgl/mL) to enable sufficient attenuation and signal. They passively distribute only in the extracellular volume and are called nonspecific or extracellular contrast media.

Abdominal contrast enhanced CT with available iodinated contrast media is a major application, in especially the oncological field. For the detection and characterization of liver lesions multi-phasic contrast-enhanced CT scans are usually performed to image the distribution of contrast media in different phase of the blood circulation (e.g. arterial, portalvenous, venous). As hepatobiliary phase imaging is not possible, the diagnostic capabilities are limited to the native and dynamic contrast enhanced liver CT phases.

Targeted CT contrast media, comparable to the above-described linear liver specific GBCAs for MRI, are commercially not available. One reason is the lower contrast media sensitivity of CT; compared to MRI the amount of CM needed for a CT signal enhancement is order of magnitude higher (standard dose CT:300-600 mg Iodine/kg vs standard dose MRT: 0.025-0.1 mmol [3.9-15.7mg] Gd/kg).

Besides the differences in dosage of iodine and gadolinium based contrast agents both offer a high attenuation in the x-ray energy spectrum of CT. At identical mass-concentrations the x-ray attenuation of gadolinium is higher than that of iodine (Nowak et al. Med Phys. 2011, Dec 38(12):6469-82).

Thus, there is an increased medical need to provide new contrast agents for radiological imaging. In particular, there is an increased medical need to provide new contrast agents for magnetic resonance imaging and/or computed tomography imaging. In particular, there is a long-standing need to provide new contrast agents for computed tomography imaging which show advantageous properties similar or comparable to those of macrocyclic contrast agents for magnetic resonance imaging as described above. More particularly, there is a long-standing medical need to provide new liver-specific contrast agents for computed tomography imaging.

**2. Description of the prior art, problem to be dissolved and its solution**

WO199532741 describes bile acid conjugates claimed to be useful for imaging of liver and bile duct using magnetic resonance imaging (MRI).

WO2001082795 describes an MRI agent with a covalently bound therapeutic blocking moiety attached which elicits a change in signal intensity of said agent when therapeutic agent interacts with its intended target.

WO2007009638 discloses metal complexes containing perfluoroalkyl groups which can be used as MRI and X-ray contrast agents in particular for lymphography.

WO2004006965 discloses perfluoroalkyl containing MRI contrast agents, which exhibit micelle formation leading to high $r_1$ relaxivity, for representing intravascular thrombi. WO1997032862 describes a class of polychelates linked to alkene bridged amino groups for diagnostic imaging using magnetic resonance imaging.

WO1999005145 details a process for the preparation of tetraazamacrocycles. WO1996016677 describes metal complexes for use as X-ray contrast media for imaging of liver and bile ducts.

WO1995028392 reveals the use of amphiphilic chelates and their use for hepatobiliary imaging.

WO2013083535 describes the preparation of hyperpolarized imaging agents for MR diagnostic analysis.

Investigative Radiology 2001 (36)8:431-444; "Dy-EOB-DTPA: Tolerance and Pharmacokinetics in Healthy Volunteers and Preliminary Liver Imaging in Patients" describes the tolerance and pharmacokinetics of the X-ray contrast agent Dy-EOB-DTPA in healthy volunteers and shows initial computed tomography (CT) image data in patients with liver lesions.

Radiology 1997; 202:399-405; "Detection of Focal Liver Lesions: CT of the Hepatobiliary System with Gadoxetic Acid Disodium" describes the use of the MRI contrast Gd-EOB-DTPA to enhance liver lesions in patients with computed tomography (CT) using up to 20 fold higher doses than approved for MRI.

The medical need for CE-MRI of the liver is high and since no macrocyclic GBCA is available linear GBCAs are still being used e.g. for differential diagnosis of focal liver lesions or the detection of small liver lesions. However, currently no marketed product containing macrocyclic GBCA with liver uptake is available which displays the favorable attributes of macrocyclic GBCAs with the essential attributes of a liver imaging agent. Among the different properties desirable for compounds suitable as MRI contrast agents are e.g. high-water solubility, high relaxivity, complete and intact excretion, good tolerability and a good safety profile.

EP405704 relates to $Gd^{3+}$ complexes with derivatives of diethylentriaminopentaacetic acid (DTPA) such as Gd-EOB-DTPA and their use as contrast agents in magnetic resonance imaging of the liver, among others. However, as described above, these linear compounds and complexes have come under increased scrutiny from the health authorities in recent years and are only still being used in a clinical environment due to the lack of suitable alternatives.

Thus, there is an unmet medical need to provide macrocyclic GBCAs for diagnostic imaging, such as magnetic resonance imaging (MRI) and/or computed tomography (CT). In particular, there is an unmet need to provide macrocyclic GBCAs for magnetic resonance imaging of the liver, which combine the beneficial properties of linear liver specific agents and macrocyclic GBCAs. Also particularly, there is a long-standing need to provide contrast agents which are liver specific and that can be applied using computed tomography. Specifically, there is an unmet medical need to provide liver specific GBCAs which show as many of the below-listed criteria as possible:

- exhibit high water solubility,
- are chemically stable,
- are stable against metal release from the chelate,
- exhibit high relaxivity,
- exhibit high in vitro uptake into human transfected OATP1B1 HEK cells,
- exhibit high in vitro uptake into human transfected OATP1 B3 HEK cells,
- have low protein binding,
- show a favorable pharmacokinetic profile and dual elimination pathway,
- are fast and completely excreted,
- exhibit no long-term retention of $Gd^{3+}$ both in tissues and in organs,

- are stable against metabolic degradation,
- are well tolerated,
- are suitable for liver imaging,
- are suitable for biliary imaging,
- are suitable for the imaging of liver diseases
- and may exhibit high in vitro uptake (e.g. into rat hepatocytes).

Particularly preferred criteria for macrocyclic GBCAs which can be used in diagnostic imaging, such as magnetic resonance imaging (MRI) and/or computed tomography (CT) are those which:

- exhibit high water solubility,
- are chemically stable,
- are stable against metal release from the chelate,
- exhibit high relaxivity,
- exhibit high in vitro uptake into human transfected OATP1B1 HEK cells,
- exhibit high in vitro uptake into human transfected OATP1B3 HEK cells,
- are well tolerated,
- are suitable for liver imaging,
- are suitable for biliary imaging,
- are suitable for the imaging of liver diseases
- and may exhibit high in vitro uptake (e.g. into rat hepatocytes).

The medical need for CE-CT with a liver specific contrast agent is high. Multiphase abdominal CT is one of the most important CT applications in particular for oncological patients. In addition to diagnostics, CT offer the opportunity for image guided interventions as tissue biopsies or minimal invasive oncological treatment as RF-ablation. For these interventions tracking of the lesion during the procedure is essential. However, the available extracellular x-ray contrast media enhances the lesion only during a short time-window of the dynamic phase. A longlasting contrast between lesion and liver would make that procedure much easier and has a great potential to increase the accuracy of targeted tissue sampling (biopsy) and therapeutic interventional treatment. For that purpose, a liver specific uptake of contrast media, resulting in an enhancement of healthy liver tissue during the hepatobiliary phase would be needed.

The issue is twofold: first the lower sensitivity of CT requires higher local contrast concentrations to generate the signal enhancement and second the limited uptake efficiency of the liver specific transports at higher contrast media doses. The lower sensitivity can be partially compensated by a higher dose. Thus, a macrocyclic structure is mandatory considering the SI increases in certain brain regions after repeated application of linear contrast agents, particularly GBCAs. The second point, the limited uptake rates at higher doses can only be addressed by the molecule structure/configuration.

Thus, there is an increased medical need to provide new contrast agents for computed tomography imaging. In particular, there is a long-standing need to provide new contrast agents for computed tomography imaging which show advantageous properties similar or comparable to those of macrocyclic contrast agents for magnetic resonance imaging as described above. More particularly, there is a long-standing medical need to provide new liver-specific contrast agents for computed tomography imaging.

The state of the art described above does not disclose the compounds of general formula (I) of the present invention as defined herein, or a stereoisomer, a tautomer, an N-oxide, a hydrate, a solvate, or a salt thereof, or a mixture

of the same, as described and defined herein. Nor does the art disclose the compounds of general formula (I) of the present invention in the form of a metal complex with a metal ion suitable for computed tomography, and stereoisomers, tautomers, N-oxides, hydrates, solvates, or salts thereof, or mixtures of same, said metal ion suitable for computed tomography being a metal ion with a k-edge energy in the range of from 33 to 91 keV and/or a metal ion having at least the x-ray attenuation of iodine in the energy range of medical x-ray imaging, including preferably computed tomography.. Nor does the art disclose the compounds of general formula (I) of the present invention in the form of a complex with $Gd^{3+}$, and stereoisomers, tautomers, N-oxides, hydrates, solvates, or salts thereof, or mixtures of same. Together or separately, the compounds of general formula (I) of the present invention and the compounds of general formula (I) of the present invention in the form of a metal complex with a metal ion suitable for computed tomography, and the compounds of general formula (I) of the present invention in the form of a complex with $Gd^{3+}$, and stereoisomers, tautomers, N-oxides, hydrates, solvates, or salts thereof, or mixtures of same are hereinafter referred to as "compounds of the present invention". The compounds of general formula (I) of the present invention in the form of a metal complex with a metal ion suitable for computed tomography, and the compounds of general formula (I) of the present invention in the form of a complex with $Gd^{3+}$, and stereoisomers, tautomers, N-oxides, hydrates, solvates, or salts thereof, or mixtures of same may also be referred to as "CT-suitable compounds of the present invention" and/or "metal compounds of the present invention" and/or "$Gd^{3+}$-containing compounds of the present invention"

It has been found, and this constitutes the basis of the present invention, that the compounds of the present invention have surprising and advantageous properties.

In particular, the compounds of general formula (I) of the present invention allow for the preparation of complexes with a metal ion suitable for computed tomography. Also particularly, the compounds of general formula (I) of the present invention allow for the preparation of complexes with $Gd^{3+}$, *i.e.* compounds of general formula (I) of the present invention in the form of a metal complex with a metal ion suitable for computed tomography,and/or compounds of general formula (I) of the present invention in the form of a complex with $Gd^{3+}$, respectively, as well as stereoisomers, tautomers, N-oxides, hydrates, solvates, or salts thereof, or mixtures of same. In particular, the $Gd^{3+}$-containing compounds of the present invention display the favorable stability of macrocyclic GBCAs and a high uptake in the liver. Further, the $Gd^{3+}$-containing compounds of the present invention show high tolerability, improved relaxivity, excellent water solubility and a fast and complete excretion, making them well suited for diagnostic imaging, in particular for magnetic resonance imaging and/or computed tomography, more particularly for magnetic resonance imaging. Specifically, the $Gd^{3+}$- containing compounds of the present invention, are particularly suited for liver imaging using magnetic resonance imaging or computed tomography, preferably in magnetic resonance imaging.

## SUMMARY

The present invention describes a new class of liver-specific (non-linear) metal, particularly gadolinium chelate complexes, methods for their preparation and their use as I contrast agents, preferably as MRI contrast agents.

## DESCRIPTION OF THE INVENTION

In accordance with a first aspect, the present invention covers compounds of general formula (I),

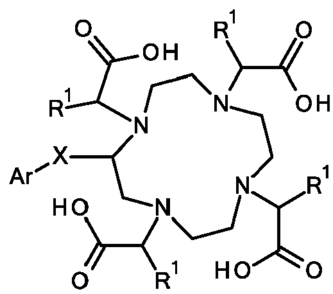

(I) ,

in which:

Ar represents a group selected from

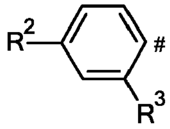 , 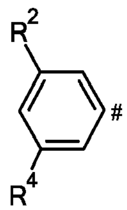 , 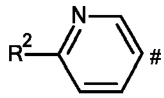 and 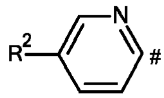 ,

wherein # indicates the point of attachment to X,

- X represents a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety;
- $R^1$ represents a hydrogen atom or a group selected from $C_1$-$C_3$-alkyl, $-CH_2OH$, $-(CH_2)_2OH$ and $CH_2OCH_3$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-;

and stereoisomers, tautomers, N-oxides, hydrates, solvates, or salts thereof, or mixtures of same.

In accordance with a second aspect, the present invention covers compounds of general formula (I), *supra,* in the form of a complex with $Gd^{3+}$, and stereoisomers, tautomers, N-oxides, hydrates, solvates, or salts thereof, or mixtures of same.

Thus, in accordance said second aspect, the present invention covers compounds of general formula (I), *supra,* in the form of a complex with $Gd^{3+}$, wherein:

Ar represents a group selected from

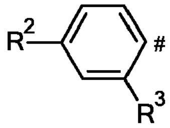 , 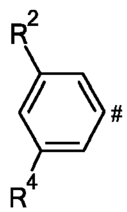 , 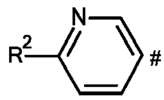 and 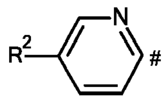 ,

wherein # indicates the point of attachment to X,

- X represents a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety;
- $R^1$ represents a hydrogen atom or a group selected from $C_1$-$C_3$-alkyl, $-CH_2OH$, $-(CH_2)_2OH$ and $CH_2OCH_3$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-;

and stereoisomers, tautomers, N-oxides, hydrates, solvates, or salts thereof, or mixtures of same.

In accordance with a third aspect, the present invention covers compounds of general formula (I), *supra*, in the form of a metal complex with a metal ion suitable for computed tomography, and stereoisomers, tautomers, N-oxides, hydrates, solvates, or salts thereof, or mixtures of same.

Thus, in accordance said third aspect, the present invention covers compounds of general formula (I), *supra,* in the form of a metal complex with a metal ion suitable for computed tomography,

wherein:

Ar represents a group selected from

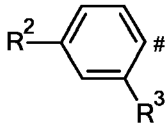, 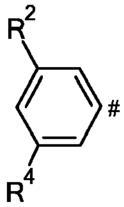, 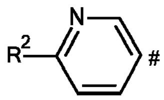 and 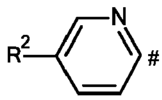,

wherein # indicates the point of attachment to X,

X represents a group selected from $CH_2$, $(CH_2)_2$, $(CH_2)_3$, $(CH_2)_4$ and $*\text{-}(CH_2)_2\text{-O-}CH_2\text{-}^{\#}$, wherein * indicates the point of attachment to Ar and # indicates the point of attachment to the acetic acid moiety;

$R^1$ represents a hydrogen atom or a group selected from $C_1$-$C_3$-alkyl, $-CH_2OH$, $-(CH_2)_2OH$ and $CH_2OCH_3$;

$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;

$R^3$ and $R^4$ represent, independently from each other, a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-;

and stereoisomers, tautomers, N-oxides, hydrates, solvates, or salts thereof, or mixtures of same.

**DEFINITIONS**

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero.

When groups in the compounds according to the invention are substituted, it is possible for said groups to be mono-substituted or poly-substituted with substituent(s), unless otherwise specified. Within the scope of the present invention, the meanings of all groups which occur repeatedly are independent from one another. It is possible that groups in the compounds according to the invention are substituted with one, two or three identical or different substituents, particularly with one substituent.

Should a composite substituent be composed of more than one parts, *e.g.* ($C_1$-$C_3$-alkoxy)-($C_2$-$C_6$-alkyl)-, it is possible for the position of a given part to be at any suitable position of said composite substituent, *i.e.* the $C_1$-$C_3$-alkoxy part can be attached to any carbon atom of the $C_2$-$C_6$-alkyl part of said ($C_1$-$C_3$-alkoxy)-($C_2$-$C_6$-alkyl)- group. A hyphen at the beginning or at the end of such a composite substituent indicates the point of attachment of said composite substituent to the rest of the molecule.

The term "comprising" when used in the specification includes "consisting of" and "consisting essentially of".

If within the present text any item is referred to as "as mentioned herein", it means that it may be mentioned anywhere in the present text.

The terms as mentioned in the present text have the following meanings:
The term "halogen atom" means a fluorine, chlorine, bromine or iodine atom, particularly a fluorine, chlorine or bromine atom.

The term "$C_1$-$C_6$-alkyl" means a linear or branched, saturated, monovalent hydrocarbon group having 1, 2, 3, 4, 5 or 6 carbon atoms, e.g. a methyl, ethyl, propyl, isopropyl, butyl, sec-butyl, isobutyl, *tert*-butyl, pentyl, isopentyl, 2-methylbutyl, 1-methylbutyl, 1-ethylpropyl, 1,2-dimethylpropyl, neo-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer or stereoisomer thereof. Particularly, said group has 1, 2, 3 or 4 carbon atoms ("$C_1$-$C_4$-alkyl"), *e.g.* a methyl, ethyl, propyl, isopropyl, butyl, sec-butyl isobutyl, or *tert*-butyl group, more particularly 1, 2 or 3 carbon atoms ("$C_1$-$C_3$-alkyl"), *e.g.* a methyl, ethyl, n-propyl or isopropyl group.

The term "$C_1$-$C_3$-haloalkyl" means a linear or branched, saturated, monovalent hydrocarbon group in which the term "$C_1$-$C_3$-alkyl" is as defined *supra,* and in which one or more of the hydrogen atoms are replaced, identically or

differently, with a halogen atom. Particularly, said halogen atom is a fluorine atom. Said $C_1$-$C_3$-haloalkyl group is, for example, fluoromethyl, difluoromethyl, trifluoromethyl, 2-fluoroethyl, 2,2-difluoroethyl, 2,2,2-trifluoroethyl, pentafluoroethyl, 3,3,3-trifluoropropyl or 1,3-difluoropropan-2-yl or other polyfluorosubstituted alkyl group.

The term "$C_2$-$C_6$-hydroxyalkyl" means a linear or branched, saturated, monovalent hydrocarbon group in which the term "$C_2$-$C_6$-alkyl" is defined *supra,* and in which one or more, preferably 1, 2 or 3 of the hydrogen atoms are replaced with a hydroxy group, *e.g.* a 2-hydroxyethyl, 3-hydroxypropyl, 2-hydroxypropyl, 1-hydroxypropan-2-yl, 2,3-dihydroxypropyl, 1,3-dihydroxypropan-2-yl, 1,3-dihydroxy-2-(hydroxymethyl)propan-2-yl, 3-hydroxy-2-methyl-propyl, 2-hydroxy-2-methyl-propyl group.

The term "$C_1$-$C_3$-alkoxy" means a linear or branched, saturated, monovalent group of formula ($C_1$-$C_3$-alkyl)-O-, in which the term "$C_1$-$C_3$-alkyl" is as defined *supra, e.g.* a methoxy, ethoxy, n-propoxy or isopropoxy group.

The term "$C_3$-$C_6$-cycloalkyl" means a saturated, monovalent, mono- or bicyclic hydrocarbon ring which contains 3, 4, 5 or 6 carbon atoms ("$C_3$-$C_6$-cycloalkyl"). Said $C_3$-$C_6$-cycloalkyl group is for example, a monocyclic hydrocarbon ring, e.g. a cyclopropyl, cyclobutyl, cyclopentyl or cyclohexyl group.

The term "$C_1$-$C_6$", as used in the present text, e.g. in the context of the definition of "$C_1$-$C_6$-alkyl" means an alkyl group having a finite number of carbon atoms of 1 to 6, *i.e.* 1, 2, 3, 4, 5 or 6 carbon atoms.

Further, as used herein, the term "$C_3$-$C_6$", as used in the present text, *e.g.* in the context of the definition of "$C_3$-$C_6$-cycloalkyl", means a cycloalkyl group having a finite number of carbon atoms of 3 to 6, *i.e.* 3, 4, 5 or 6 carbon atoms.

When a range of values is given, said range encompasses each value and sub-range within said range.

For example:

"$C_1$-$C_6$" encompasses $C_1$, $C_2$, $C_3$, $C_4$, $C_5$, $C_6$, $C_1$-$C_6$, $C_1$-$C_5$, $C_1$-$C_4$, $C_1$-$C_3$, $C_1$-$C_2$, $C_2$-$C_6$, $C_2$-$C_5$, $C_2$-$C_4$, $C_2$-$C_3$, $C_3$-$C_6$, $C_3$-$C_5$, $C_3$-$C_4$, $C_4$-$C_6$, $C_4$-$C_5$, and $C_5$-$C_6$;

"$C_1$-$C_4$" encompasses $C_1$, $C_2$, $C_3$, $C_4$, $C_1$-$C_4$, $C_1$-$C_3$, $C_1$-$C_2$, $C_2$-$C_4$, $C_2$-$C_3$ and $C_3$-$C_4$;

"$C_1$-$C_3$" encompasses $C_1$, $C_2$, $C_3$, $C_1$-$C_3$, $C_1$-$C_2$ and $C_2$-$C_3$;

"$C_2$-$C_6$" encompasses $C_2$, $C_3$, $C_4$, $C_5$, $C_6$, $C_2$-$C_6$, $C_2$-$C_5$, $C_2$-$C_4$, $C_2$-$C_3$, $C_3$-$C_6$, $C_3$-$C_5$, $C_3$-$C_4$, $C_4$-$C_6$, $C_4$-$C_5$, and $C_5$-$C_6$; and

"$C_3$-$C_6$" encompasses $C_3$, $C_4$, $C_5$, $C_6$, $C_3$-$C_6$, $C_3$-$C_5$, $C_3$-$C_4$, $C_4$-$C_6$, $C_4$-$C_5$, and $C_5$-$C_6$.

The compounds of the present invention may contain one or more asymmetric centers, depending upon the location and nature of the various substituents desired. Asymmetric carbon atoms may be present in the (R) or (S) configuration, which can result in racemic mixtures, mixtures in which one enantiomer is present in a greater amount than the other enantiomer, or single enantiomers in the case of a single asymmetric center. In the case of multiple stereogenic centers, diastereomeric mixtures, single diastereomers or single enantiomers can be synthesized. In certain instances, asymmetry may also be present due to restricted rotation about a given bond, axial chirality or coordination of the metal center.

In the context of the present invention, the compounds of formula (I), the compounds of formula (I) in the form of a $Gd^{3+}$ complex, the compounds of formula (II), the compounds of formula (III) as well as any other compounds and/or intermediates herein described may include a group X. Said group X may, *inter alia,* represent a group selected from $CH_2$, $(CH_2)_2$, $(CH_2)_3$, $(CH_2)_4$ and $*\text{-}(CH_2)_2\text{-O-}CH_2\text{-}^{\#}$, wherein * indicates the point of attachment to Ar and # indicates the point of attachment to the acetic acid moiety. One skilled in the art would recognize that the terms $CH_2$, $(CH_2)_2$, $(CH_2)_3$ and $(CH_2)_4$ refer to linear alkyl groups, *i.e.* $*CH_2\text{-}^{\#}$, $*\text{-}(CH_2)_2\text{-}^{\#}$, $*\text{-}(CH_2)_3\text{-}^{\#}$ and $*\text{-}(CH_2)_4\text{-}^{\#}$ groups, wherein * indicates the point of attachment to Ar and # indicates the point of attachment to the acetic acid moiety.

The present invention relates to compounds of formula (I) in the form of a metal complex with a metal ion suitable for computed tomography. Thus, in the context of the present invention, "a metal ion suitable for computed tomography" refers to a metal ion with a k-edge energy in the range of from 33 to 91 keV and/or to a metal ion having at least the x-ray attenuation of iodine in the energy range of medical x-ray imaging, including preferably computed tomography. Preferably, "a metal ion suitable for computed tomography" is an ion of a lanthanide or an ion of a metal selected from Bi, W, Hf and Ta.

The k-edge energy of a metal is known to the skilled person and has been described in, for example: Curry, Thomas S.; Dowdey, James E.; Murry, Robert C. (1990). "Attenuation". Christensen's Physics of Diagnostic Radiology and can be retrieved from the Physical Measurement Laboratory of the National Institute of Standards and Technology: https://physics.nist.gov/PhysRefData/XrayMassCoef/tab3.html.

The x-ray attenuation of iodine is 33.2 keV as disclosed by the Physical Measurement Laboratory of the National Institute of Standards and Technology: (https://physics.nist.gov/PhysRefData/XrayMassCoef/tab3.html) and the energy

range of medical x-ray imaging lies between 25 and 150kV, preferably between 70 and 150kV.

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g.*, chiral HPLC columns), with or without conventional derivatisation, optimally chosen to maximise the separation of the enantiomers. Suitable chiral HPLC columns are manufactured by Daicel, *e.g.*, Chiracel OD and Chiracel OJ among many others, all routinely selectable. Enzymatic separations, with or without derivatisation, are also useful. The optically active compounds of this invention can likewise be obtained by chiral syntheses utilizing optically active starting materials and/or reagents and catalysts.

In order to describe different types of isomers reference is made to IUPAC Rules Section E (Pure Appl Chem 45, 11-30, 1976).

The present invention includes all possible stereoisomers of the compounds of the present invention as single stereoisomers, or as any mixture of said stereoisomers, *e.g.* R- or S-isomers, or diastereoisomers, in any ratio. Isolation of a single stereoisomer, *e.g.* a single enantiomer or a single diastereomer, of a compound of the present invention may be achieved by any suitable state of the art method as described herein, such as chromatography, especially chiral chromatography, for example.

In particular, the compounds of general formula (I) in the form of a complex with $Gd^{3+}$, i.e. $Gd^{3+}$ complexes of the compounds of general formula (I), as described throughout this document and including their salts as described below, may exist in different stereochemical configurations (i.e., as different stereoisomers) depending on the complexation of the $Gd^{3+}$ center to the macrocyclic ring and/or on the presence of further chiral centers in the molecule. The present invention therefore includes all possible stereoisomers of the compounds of general formula (I) in the form of a complex with $Gd^{3+}$, i.e. all stereoisomers of $Gd^{3+}$ complexes of the compounds of general formula (I) either as single stereoisomers or as mixtures of two or more of said stereoisomers, in any ratio.

Further, the compounds of the present invention can exist as N-oxides, which are defined in that at least one nitrogen of the compounds of the present invention is oxidized. The present invention includes all such possible N-oxides.

The present invention also relates to useful forms of the compounds as disclosed herein, such as hydrates, solvates, salts, in particular pharmaceutically acceptable salts, and coprecipitates.

The compounds of the present invention can exist as a hydrate, or as a solvate, wherein the compounds of the present invention contain polar solvents, in particular water, methanol or ethanol for example as structural element of the crystal lattice of the compounds. The amount of polar solvents, in particular water, may exist in a stoichiometric or non-stoichiometric ratio. In the case of stoichiometric solvates, *e.g.* a hydrate, hemi-, (semi-), mono-, sesqui-, di-, tri-, tetra-, penta- *etc.* solvates or hydrates, respectively, are possible. The present invention includes all such hydrates or solvates.

Further, the compounds of the present invention can exist in the form of a salt. Said salt may be either an inorganic or organic addition salt, particularly any pharmaceutically acceptable inorganic or organic addition salt, customarily used in pharmaceutical formulations.

Further, in the context of the present invention, the compounds of general formula (I) in the form of a complex with $Gd^{3+}$, *i.e.* $Gd^{3+}$ complexes of the compounds of general formula (I), as described throughout this document, may exist as salts in the form according to formula (Ia)

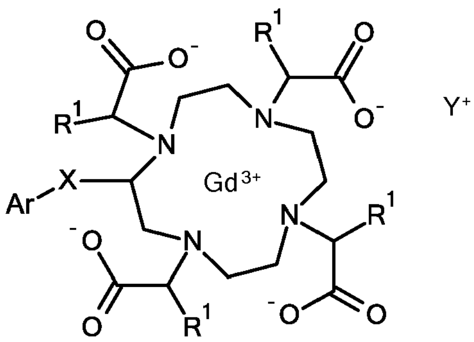

(**Ia**),

wherein:

Ar, X and $R^1$ to $R^6$ represent the groups indicated in the different aspects, embodiments, examples and any other descriptions and/or depictions of the compounds of general formula (I) in the form of a complex with $Gd^{3+}$, *i.e.* $Gd^{3+}$ complexes of the compounds of general formula (I), as described throughout this document, and $Y^+$ represents a hydrogen atom or a positively charged organic or inorganic counterion. In particularly preferred embodiments, the compounds of general formula (I) in the form of a complex with $Gd^{3+}$, *i.e.* $Gd^{3+}$ complexes of the compounds of general formula (I), as described throughout this document, may exist as salt of an alkali metal (group 1 element), such as sodium salts, *i.e.* as salts in the form according to formula (Ia), supra, wherein $Y^+$ represents a cation of an alkali metal (group 1 element), *e.g.* a positively charged sodium cation of an alkali metal (group 1 element). In more particularly preferred embodiments, the compounds of general formula (I) in the form of a complex with $Gd^{3+}$, *i.e.* $Gd^{3+}$ complexes of the compounds of general formula (I), as described throughout this document, may exist as sodium salts, *i.e.* as salts in the form according to formula (Ia), supra, wherein $Y^+$ represents a sodium cation, *i.e.* a positively charged sodium ion.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19. The production of especially neutral salts is described in US Patent No. 5,560,903.

Pharmaceutically acceptable salts of the compounds according to the invention include salts with inorganic and/or organic bases or amino acids, in particular physiologically tolerable cations of inorganic and/or organic bases or amino acids, such as, inter alia, those of primary, secondary or tertiary amines. Examples may be, without being limited thereto, salts of sodium, lithium, potassium, calcium, magnesium, arginine, lysine, ammonia, creatinine, diethanolamine, ethanol amine, morpholine, glucamine, N,N-dimethylglucamine, N-methylglucamine, ornithine, histidine, imidazole, tromethamine, meglumine and the like. Particularly preferred pharmaceutically acceptable salts of the compounds according to the invention are their corresponding sodium salts.

Those skilled in the art will further recognize that salts of the claimed compounds may be prepared by reaction of the compounds with the appropriate inorganic or organic base via any of a number of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

This applies analogously to cases in which synthesis intermediates or example compounds or salts thereof have been obtained, by the preparation and/or purification processes described, as solvates, such as hydrates with (if defined) unknown stoichiometric composition.

In accordance with a further embodiment of the first aspect, the present invention covers compounds of general formula (I), *supra,* wherein:

Ar represents a group selected from

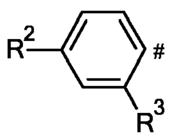 , 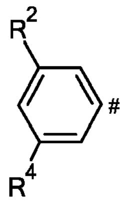 , 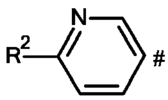 and 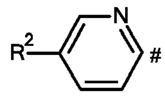 ,

wherein # indicates the point of attachment to X,

- X represents a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety;
- $R^1$ represents a hydrogen atom;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

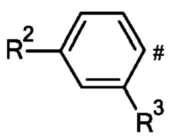 , 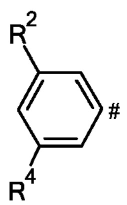 , 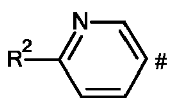 and 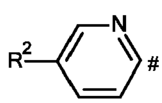

wherein # indicates the point of attachment to X,

- X represents a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety;
- $R^1$ represents a hydrogen atom or a group selected from $C_1$-$C_3$-alkyl, $-CH_2OH$, $-(CH_2)_2OH$ and $CH_2OCH_3$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ represents a hydrogen atom
- $R^4$ represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

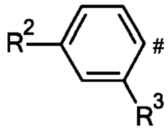, 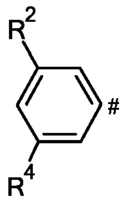, 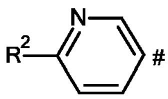 and 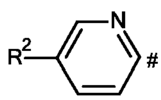,

wherein # indicates the point of attachment to X,

- X represents a group selected from $CH_2$, $(CH_2)_2$, $(CH_2)_3$, $(CH_2)_4$ and *-$(CH_2)_2$-O-$CH_2$-#, wherein * indicates the point of attachment to Ar and # indicates the point of attachment to the acetic acid moiety;
- $R^1$ represents a hydrogen atom or a group selected from $C_1$-$C_3$-alkyl, -$CH_2OH$, -$(CH_2)_2OH$ and $CH_2OCH_3$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

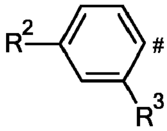 and 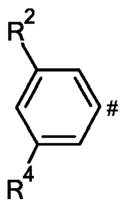,

wherein # indicates the point of attachment to X,

- X represents a group selected from $CH_2$, $(CH_2)_2$, $(CH_2)_3$, $(CH_2)_4$ and *-$(CH_2)_2$-O-$CH_2$-#, wherein * indicates the point of attachment to Ar and # indicates the point of attachment to the acetic acid moiety;
- $R^1$ represents a hydrogen atom or a group selected from $C_1$-$C_3$-alkyl, -$CH_2OH$, -$(CH_2)_2OH$ and $CH_2OCH_3$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

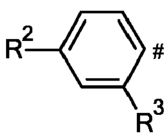 and 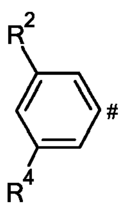,

wherein # indicates the point of attachment to X,

X represents a group selected from $CH_2$ and $(CH_2)_2$;
$R^1$ represents a hydrogen atom or a group selected from $C_1$-$C_3$-alkyl, $-CH_2OH$, $-(CH_2)_2OH$ and $CH_2OCH_3$;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

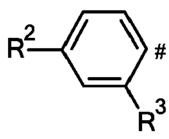
and
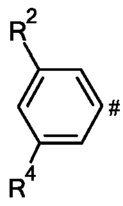
,

wherein # indicates the point of attachment to X,

X represents a group selected from $CH_2$ and $(CH_2)_2$;
$R^1$ represents a hydrogen atom or a group selected from $-CH_2OH$ and $-(CH_2)_2OH$;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

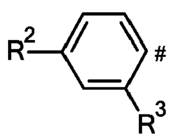
and
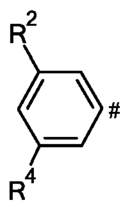
,

wherein # indicates the point of attachment to X,

X represents a $CH_2$ group;
$R^1$ represents a hydrogen atom or a group selected from $-CH_2OH$ and $-(CH_2)_2OH$;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

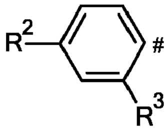 and 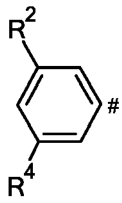 ,

wherein # indicates the point of attachment to X,

X represents a group selected from $CH_2$ and $(CH_2)_2$;
$R^1$ represents a hydrogen atom or a $-CH_2OH$ group;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

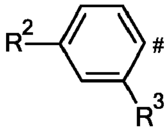 and 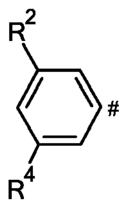 ,

wherein # indicates the point of attachment to X,

X represents a group selected from $CH_2$ and $(CH_2)_2$;
$R^1$ represents a hydrogen atom;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

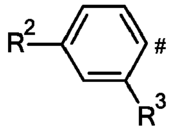 and 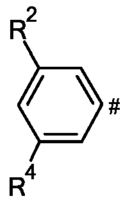 ,

wherein # indicates the point of attachment to X,

X represents a $CH_2$ group;
$R^1$ represents a hydrogen atom;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

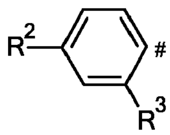 and 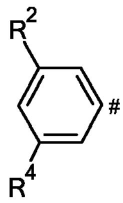 ,

wherein # indicates the point of attachment to X,

X represents a group selected from $CH_2$, $(CH_2)_2$, $(CH_2)_3$, $(CH_2)_4$ and *-$(CH_2)_2$-O-$CH_2$-#, wherein * indicates the point of attachment to Ar and # indicates the point of attachment to the acetic acid moiety;
$R^1$ represents a hydrogen atom or a group selected from $C_1$-$C_3$-alkyl, -$CH_2OH$, -$(CH_2)_2OH$ and $CH_2OCH_3$;
$R^2$ represents a group selected from C2-C4-alkoxy, ($H_3C$-$CH_2O$)-$(CH_2)_2$-O-, ($H_3C$-$CH_2O$)-$(CH_2)_2$-O-$(CH_2)_2$-O- and ($H_3C$-$CH_2O$)-$(CH_2)_2$-O-$(CH_2)_2$-O-$(CH_2)_2$-O-, wherein said $C_2$-$C_4$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

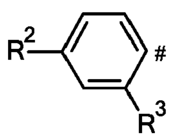 and 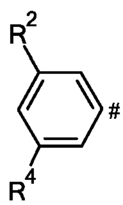 ,

wherein # indicates the point of attachment to X,

X represents a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety;

$R^1$ represents a hydrogen atom or a group selected from $C_1$-$C_3$-alkyl, $-CH_2OH$, $-(CH_2)_2OH$ and $CH_2OCH_3$;

$R^2$ represents a group selected from $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;

$R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

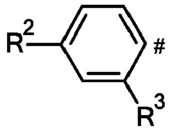 and 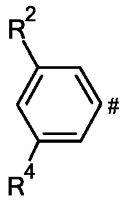 ,

wherein # indicates the point of attachment to X,

X represents a group selected from $CH_2$ and $(CH_2)_2$;

$R^1$ represents a hydrogen atom or a group selected from $C_1$-$C_3$-alkyl, $-CH_2OH$, $-(CH_2)_2OH$ and $CH_2OCH_3$;

$R^2$ represents a group selected from $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;

$R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

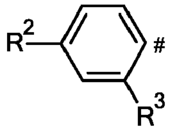 and 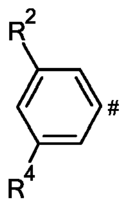 ,

wherein # indicates the point of attachment to X,

X represents a group selected from $CH_2$ and $(CH_2)_2$;

$R^1$ represents a hydrogen atom or a $-CH_2OH$ group;

$R^2$ represents a group selected from $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;

$R^3$ and $R^4$ represent, independently from each other, a hydrogen atom;

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

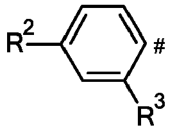 and 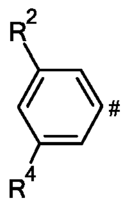 ,

wherein # indicates the point of attachment to X,

| | |
|---|---|
| X | represents a group selected from $CH_2$ and $(CH_2)_2$; |
| $R^1$ | represents a hydrogen atom; |
| $R^2$ | represents a group selected from $(H_3C\text{-}CH_2O)\text{-}(CH_2)_2\text{-}O\text{-}$, $(H_3C\text{-}CH_2O)\text{-}(CH_2)_2\text{-}O\text{-}(CH_2)_2\text{-}O\text{-}$ and $(H_3C\text{-}CH_2O)\text{-}(CH_2)_2\text{-}O\text{-}(CH_2)_2\text{-}O\text{-}(CH_2)_2\text{-}O\text{-}$; |
| $R^3$ and $R^4$ | represent, independently from each other, a hydrogen atom; |

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

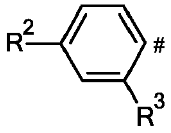 and 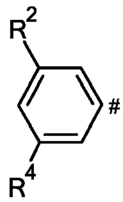 ,

wherein # indicates the point of attachment to X,

| | |
|---|---|
| X | represents a $CH_2$ group; |
| $R^1$ | represents a hydrogen atom; |
| $R^2$ | represents a group selected from $(H_3C\text{-}CH_2O)\text{-}(CH_2)_2\text{-}O\text{-}$, $(H_3C\text{-}CH_2O)\text{-}(CH_2)_2\text{-}O\text{-}(CH_2)_2\text{-}O\text{-}$ and $(H_3C\text{-}CH_2O)\text{-}(CH_2)_2\text{-}O\text{-}(CH_2)_2\text{-}O\text{-}(CH_2)_2\text{-}O\text{-}$; |
| $R^3$ and $R^4$ | represent, independently from each other, a hydrogen atom; |

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

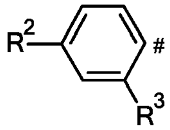 , 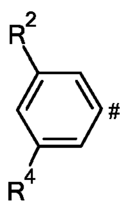 , 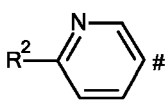 and 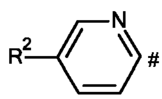 ,

wherein # indicates the point of attachment to X,

| | |
|---|---|
| X | represents a group selected from $CH_2$, $(CH_2)_2$, $(CH_2)_3$, $(CH_2)_4$ and $\text{*-}(CH_2)_2\text{-}O\text{-}CH_2\text{-}^{\#}$, wherein * indicates the point of attachment to Ar and # indicates the point of attachment to the acetic acid moiety; |
| $R^1$ | represents a hydrogen atom; |
| $R^2$ | represents a group selected from $C_2\text{-}C_5$-alkoxy, $(C_1\text{-}C_3\text{-alkoxy})\text{-}(CH_2)_2\text{-}O\text{-}$, |

$(C_1$-$C_3$-alkoxy)-$(CH_2)_2$-O-$(CH_2)_2$-O- and $(C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;

$R^3$ and $R^4$ represent, independently from each other, a hydrogen atom or a group selected from $C_2$-$C_5$-alkoxy, $(C_1$-$C_3$-alkoxy)-$(CH_2)_2$-O-, $(C_1$-$C_3$-alkoxy)-$(CH_2)_2$-O-$(CH_2)_2$-O- and $(C_1$-$C_3$-alkoxy)-$(CH_2)_2$-O-$(CH_2)_2$-O-$(CH_2)_2$-O-;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

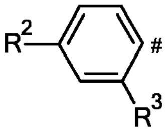 , 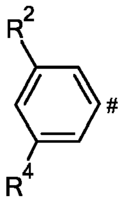 , 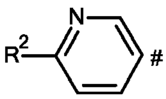 and 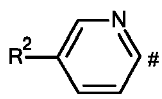 ,

wherein # indicates the point of attachment to X,

X represents a group selected from $CH_2$, $(CH_2)_2$, $(CH_2)_3$, $(CH_2)_4$ and *-$(CH_2)_2$-O-$CH_2$-#, wherein * indicates the point of attachment to Ar and # indicates the point of attachment to the acetic acid moiety;

$R^1$ represents a hydrogen atom or a group selected from $C_1$-$C_3$-alkyl, -$CH_2OH$, -$(CH_2)_2OH$ and $CH_2OCH_3$;

$R^2$ represents a group selected from $C_2$-$C_5$-alkoxy, $(C_1$-$C_3$-alkoxy)-$(CH_2)_2$-O-, $(C_1$-$C_3$-alkoxy)-$(CH_2)_2$-O-$(CH_2)_2$-O- and $(C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;

$R^3$ represents a hydrogen atom

$R^4$ represents a hydrogen atom or a group selected from $C_2$-$C_5$-alkoxy, $(C_1$-$C_3$-alkoxy)-$(CH_2)_2$-O-, $(C_1$-$C_3$-alkoxy)-$(CH_2)_2$-O-$(CH_2)_2$-O- and $(C_1$-$C_3$-alkoxy)-$(CH_2)_2$-O-$(CH_2)_2$-O-$(CH_2)_2$-O-;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

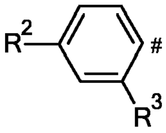 , 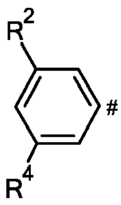 , 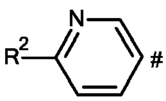 and 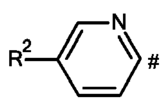 ,

wherein # indicates the point of attachment to X,

X represents a group selected from $CH_2$, $(CH_2)_2$, $(CH_2)_3$, $(CH_2)_4$ and *-$(CH_2)_2$-O-$CH_2$-#, wherein * indicates the point of attachment to Ar and # indicates the point of attachment to the acetic acid moiety;

$R^1$ represents a hydrogen atom or a group selected from $C_1$-$C_3$-alkyl, -$CH_2OH$, -$(CH_2)_2OH$ and $CH_2OCH_3$;

$R^2$ represents a group selected from $C_2$-$C_5$-alkoxy, $(C_1$-$C_3$-alkoxy)-$(CH_2)_2$-O-, $(C_1$-$C_3$-alkoxy)-$(CH_2)_2$-O-$(CH_2)_2$-O- and $(C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;

$R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

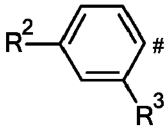 and 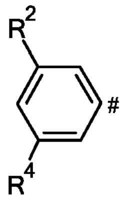 ,

wherein # indicates the point of attachment to X,

X represents a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety;

$R^1$ represents a hydrogen atom or a group selected from $C_1$-$C_3$-alkyl, $-CH_2OH$, $-(CH_2)_2OH$ and $CH_2OCH_3$;

$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;

$R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

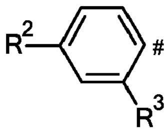 and 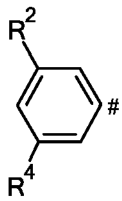 ,

wherein # indicates the point of attachment to X,

X represents a group selected from $CH_2$ and $(CH_2)_2$;

$R^1$ represents a hydrogen atom or a group selected from $C_1$-$C_3$-alkyl, $-CH_2OH$, $-(CH_2)_2OH$ and $CH_2OCH_3$;

$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;

$R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

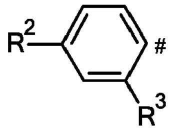 and 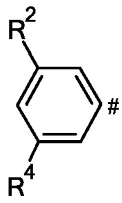 ,

wherein # indicates the point of attachment to X,

- X represents a group selected from $CH_2$ and $(CH_2)_2$;
- $R^1$ represents a hydrogen atom or a group selected from $-CH_2OH$ and $-(CH_2)_2OH$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

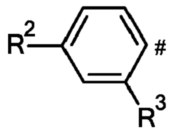 and 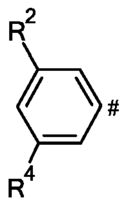 ,

wherein # indicates the point of attachment to X,

- X represents a $CH_2$ group;
- $R^1$ represents a hydrogen atom or a group selected from $-CH_2OH$ and $-(CH_2)_2OH$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

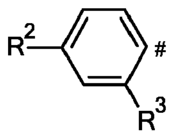 and 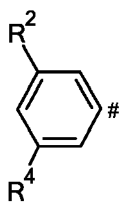 ,

wherein # indicates the point of attachment to X,

X represents a group selected from $CH_2$ and $(CH_2)_2$;
$R^1$ represents a hydrogen atom or a $-CH_2OH$ group;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

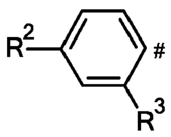 and 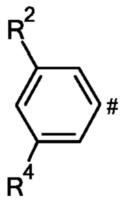 ,

wherein # indicates the point of attachment to X,

X represents a group selected from $CH_2$ and $(CH_2)_2$;
$R^1$ represents a hydrogen atom;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

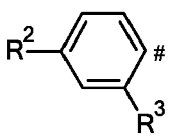 and 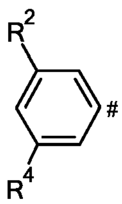 ,

wherein # indicates the point of attachment to X,

X represents a $CH_2$ group;
$R^1$ represents a hydrogen atom;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of

general formula (I), *supra,* in the form of a complex with $Gd^{3+}$, wherein:

Ar represents a group selected from

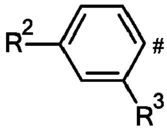 and 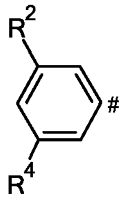 ,

wherein # indicates the point of attachment to X,

X represents a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety;

$R^1$ represents a hydrogen atom or a group selected from $C_1$-$C_3$-alkyl, $-CH_2OH$, $-(CH_2)_2OH$ and $CH_2OCH_3$;

$R^2$ represents a group selected from C2-C4-alkoxy, $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$, wherein said $C_2$-$C_4$-alkoxy groups are optionally substituted, one, two, three or four times, with a fluorine atom;

$R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

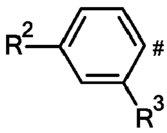 and 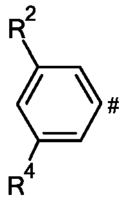 ,

wherein # indicates the point of attachment to X,

X represents a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety;

$R^1$ represents a hydrogen atom or a group selected from $C_1$-$C_3$-alkyl, $-CH_2OH$, $-(CH_2)_2OH$ and $CH_2OCH_3$;

$R^2$ represents a group selected from $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;

$R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

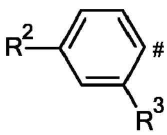 and 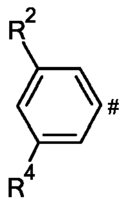 ,

wherein # indicates the point of attachment to X,

X represents a group selected from $CH_2$ and $(CH_2)_2$;
$R^1$ represents a hydrogen atom or a group selected from $C_1$-$C_3$-alkyl, $-CH_2OH$, $-(CH_2)_2OH$ and $CH_2OCH_3$;
$R^2$ represents a group selected from $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

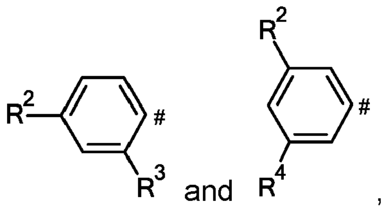

wherein # indicates the point of attachment to X,

X represents a group selected from $CH_2$ and $(CH_2)_2$;
$R^1$ represents a hydrogen atom or a $-CH_2OH$ group;
$R^2$ represents a group selected from $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

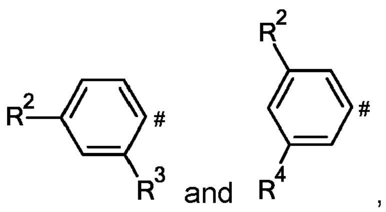

wherein # indicates the point of attachment to X,

X represents a group selected from $CH_2$ and $(CH_2)_2$;
$R^1$ represents a hydrogen atom;
$R^2$ represents a group selected from $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

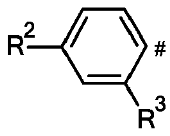
and
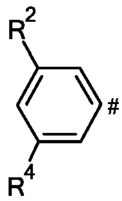
,

wherein # indicates the point of attachment to X,

| | |
|---|---|
| X | represents a $CH_2$ group; |
| $R^1$ | represents a hydrogen atom; |
| $R^2$ | represents a group selected from $(H_3C\text{-}CH_2O)\text{-}(CH_2)_2\text{-}O\text{-}$, $(H_3C\text{-}CH_2O)\text{-}(CH_2)_2\text{-}O\text{-}(CH_2)_2\text{-}O\text{-}$ and $(H_3C\text{-}CH_2O)\text{-}(CH_2)_2\text{-}O\text{-}(CH_2)_2\text{-}O\text{-}(CH_2)_2\text{-}O\text{-}$; |
| $R^3$ and $R^4$ | represent, independently from each other, a hydrogen atom; |

and stereoisomers, tautomers, N-oxides, hydrates, solvates, or salts thereof, or mixtures of same.

In accordance with a further embodiment of the second aspect, the present invention covers compounds of general formula (I), *supra,* in the form of a sodium ($Na^+$) salt of a complex with $Gd^{3+}$, wherein Ar, X, $R^1$, $R^2$, $R^3$, and $R^4$ are defined as described in any of the embodiments above, and stereoisomers, tautomers, hydrates, or solvates thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

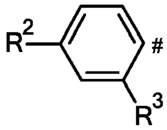
,
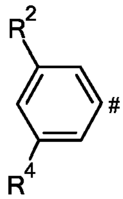
,
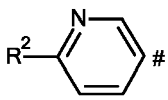
and
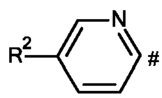
,

wherein # indicates the point of attachment to X,

| | |
|---|---|
| X | represents a group selected from $CH_2$, $(CH_2)_2$, $(CH_2)_3$, $(CH_2)_4$ and $*\text{-}(CH_2)_2\text{-}O\text{-}CH_2\text{-}^{\#}$, wherein * indicates the point of attachment to Ar and # indicates the point of attachment to the acetic acid moiety; |
| $R^1$ | represents a hydrogen atom; |
| $R^2$ | represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom; |
| $R^3$ and $R^4$ | represent, independently from each other, a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-; |

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

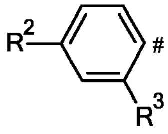 , 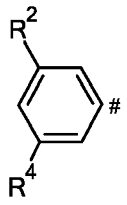 , 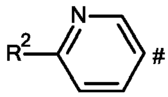 and 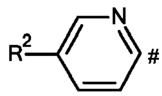 ,

wherein # indicates the point of attachment to X,

- X represents a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety;
- $R^1$ represents a hydrogen atom or a group selected from $C_1$-$C_3$-alkyl, $-CH_2OH$, $-(CH_2)_2OH$ and $CH_2OCH_3$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ represents a hydrogen atom
- $R^4$ represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

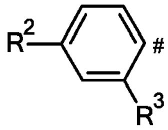 , 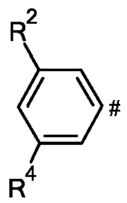 , 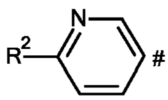 and 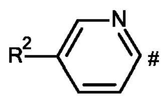 ,

wherein # indicates the point of attachment to X,

- X represents a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety;
- $R^1$ represents a hydrogen atom or a group selected from $C_1$-$C_3$-alkyl, $-CH_2OH$, $-(CH_2)_2OH$ and $CH_2OCH_3$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

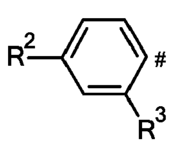 and 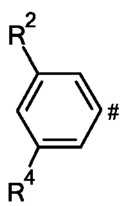 ,

wherein # indicates the point of attachment to X,

- X represents a group selected from $CH_2$, $(CH_2)_2$, $(CH_2)_3$, $(CH_2)_4$ and *-$(CH_2)_2$-O-$CH_2$-#, wherein * indicates the point of attachment to Ar and # indicates the point of attachment to the acetic acid moiety;
- $R^1$ represents a hydrogen atom or a group selected from $C_1$-$C_3$-alkyl, -$CH_2OH$, -$(CH_2)_2OH$ and $CH_2OCH_3$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

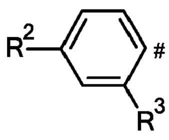 and 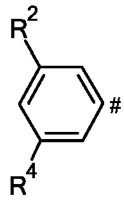 ,

wherein # indicates the point of attachment to X,

- X represents a group selected from $CH_2$ and $(CH_2)_2$;
- $R^1$ represents a hydrogen atom or a group selected from $C_1$-$C_3$-alkyl, -$CH_2OH$, -$(CH_2)_2OH$ and $CH_2OCH_3$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

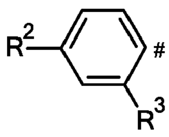 and 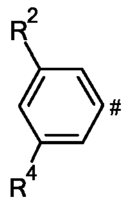 ,

wherein # indicates the point of attachment to X,

- X represents a group selected from $CH_2$ and $(CH_2)_2$;
- $R^1$ represents a hydrogen atom or a group selected from -$CH_2OH$ and -$(CH_2)_2OH$;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

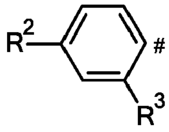 and 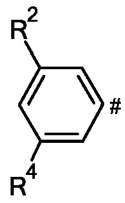 ,

wherein # indicates the point of attachment to X,

X represents a $CH_2$ group;

$R^1$ represents a hydrogen atom or a group selected from $-CH_2OH$ and $-(CH_2)_2OH$;

$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;

$R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

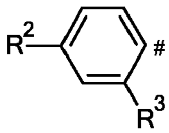 and 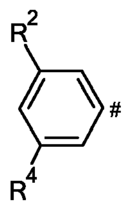 ,

wherein # indicates the point of attachment to X,

X represents a group selected from $CH_2$ and $(CH_2)_2$;

$R^1$ represents a hydrogen atom or a $-CH_2OH$ group;

$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;

$R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

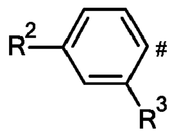 and 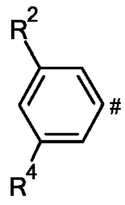 ,

wherein # indicates the point of attachment to X,

- X represents a group selected from $CH_2$ and $(CH_2)_2$;
- $R^1$ represents a hydrogen atom;
- $R^2$ represents a group selected from$C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and($C_1$-$C_3$-alkoxy)-$(CH_2)_2$-O-$(CH_2)_2$-O-$(CH_2)_2$-O-,wherein said $C_1$-$C_3$-alkoxy groups and $C_2$-$C_5$-alkoxy groups are optionally substituted,one, two, three or four times, with a fluorine atom;
- $R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

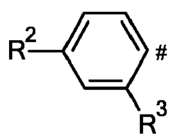 and 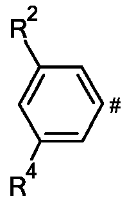 ,

wherein # indicates the point of attachment to X,

- X represents a $CH_2$ group;
- $R^1$ represents a hydrogen atom;
- $R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
- $R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

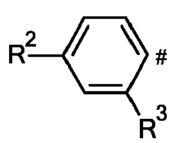 and 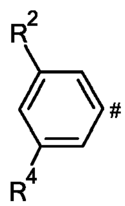 ,

wherein # indicates the point of attachment to X,

X represents a group selected from $CH_2$, $(CH_2)_2$, $(CH_2)_3$, $(CH_2)_4$ and *-$(CH_2)_2$-O-$CH_2$-#, wherein * indicates the point of attachment to Ar and # indicates the point of attachment to the acetic acid moiety;
$R^1$ represents a hydrogen atom or a group selected from $C_1$-$C_3$-alkyl, -$CH_2OH$, -$(CH_2)_2OH$ and $CH_2OCH_3$;
$R^2$ represents a group selected from C2-C4-alkoxy, ($H_3C$-$CH_2O$)-$(CH_2)_2$-O-, ($H_3C$-$CH_2O$)-$(CH_2)_2$-O-$(CH_2)_2$-O- and ($H_3C$-$CH_2O$)-$(CH_2)_2$-O-$(CH_2)_2$-O-$(CH_2)_2$-O-, wherein said $C_2$-$C_4$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

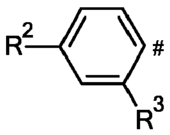
and
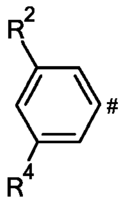
,

wherein # indicates the point of attachment to X,

X represents a group selected from $CH_2$, $(CH_2)_2$, $(CH_2)_3$, $(CH_2)_4$ and *-$(CH_2)_2$-O-$CH_2$-#, wherein * indicates the point of attachment to Ar and # indicates the point of attachment to the acetic acid moiety;
$R^1$ represents a hydrogen atom or a group selected from $C_1$-$C_3$-alkyl, -$CH_2OH$, -$(CH_2)_2OH$ and $CH_2OCH_3$;
$R^2$ represents a group selected from ($H_3C$-$CH_2O$)-$(CH_2)_2$-O-, ($H_3C$-$CH_2O$)-$(CH_2)_2$-O-$(CH_2)_2$-O- and ($H_3C$-$CH_2O$)-$(CH_2)_2$-O-$(CH_2)_2$-O-$(CH_2)_2$-O-;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

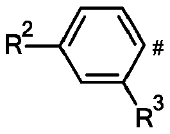
and
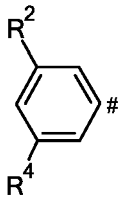
,

wherein # indicates the point of attachment to X,

X represents a group selected from $CH_2$ and $(CH_2)_2$;
$R^1$ represents a hydrogen atom or a group selected from $C_1$-$C_3$-alkyl, -$CH_2OH$, -$(CH_2)_2OH$ and $CH_2OCH_3$;
$R^2$ represents a group selected from ($H_3C$-$CH_2O$)-$(CH_2)_2$-O-, ($H_3C$-$CH_2O$)-$(CH_2)_2$-O-$(CH_2)_2$-O- and ($H_3C$-$CH_2O$)-$(CH_2)_2$-O-$(CH_2)_2$-O-$(CH_2)_2$-O-;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

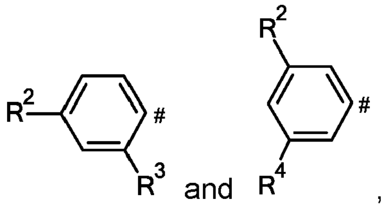

wherein # indicates the point of attachment to X,

X represents a group selected from $CH_2$ and $(CH_2)_2$;
$R^1$ represents a hydrogen atom or a $-CH_2OH$ group;
$R^2$ represents a group selected from $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

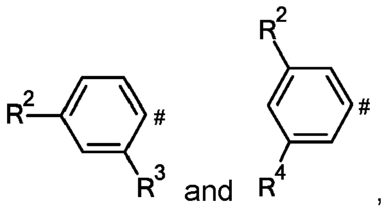

wherein # indicates the point of attachment to X,

X represents a group selected from $CH_2$ and $(CH_2)_2$;
$R^1$ represents a hydrogen atom;
$R^2$ represents a group selected from $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;
$R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a metal complex with a metal ion suitable for computed tomography, wherein:

Ar represents a group selected from

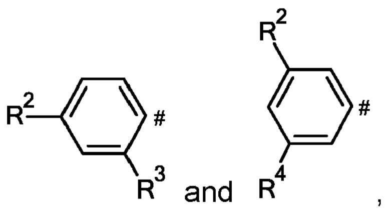

wherein # indicates the point of attachment to X,

X represents a $CH_2$ group;
$R^1$ represents a hydrogen atom;
$R^2$ represents a group selected from $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;

$R^3$ and $R^4$ represent, independently from each other, a hydrogen atom;

and stereoisomers, tautomers, N-oxides, hydrates, solvates, or salts thereof, or mixtures of same.

In accordance with a further embodiment of the third aspect, the present invention covers compounds of general formula (I), *supra,* in the form of a sodium ($Na^+$) salt of a metal complex with a metal ion suitable for computed tomography, wherein Ar, X, $R^1$, $R^2$, $R^3$, and $R^4$ are defined as described in any of the embodiments above, and stereoisomers, tautomers, hydrates, or solvates thereof, or mixtures of same

In a further embodiment of the first aspect, the invention relates to compounds of formula (I), wherein:

Ar represents a group selected from

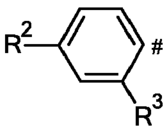 , 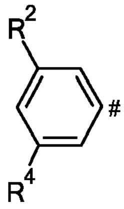 , 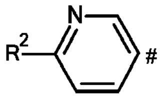 and 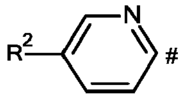 ,

wherein # indicates the point of attachment to X,
and stereoisomers, tautomers, N-oxides, hydrates, solvates, or salts thereof, or mixtures of same.

In a further embodiment of the first aspect, the invention relates to compounds of formula (I), wherein:

Ar represents a group selected from

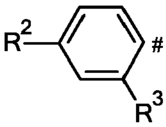 and 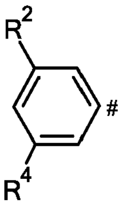

wherein # indicates the point of attachment to X,
and stereoisomers, tautomers, N-oxides, hydrates, solvates, or salts thereof, or mixtures of same.

In a further embodiment of the first aspect, the invention relates to compounds of formula (I), wherein:

Ar represents a group

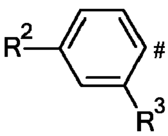 ,

wherein # indicates the point of attachment to X,
and stereoisomers, tautomers, N-oxides, hydrates, solvates, or salts thereof, or mixtures of same.

In a further embodiment of the first aspect, the invention relates to compounds of formula (I), wherein:

Ar represents a group

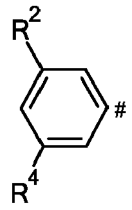

,

wherein # indicates the point of attachment to X,
and stereoisomers, tautomers, N-oxides, hydrates, solvates, or salts thereof, or mixtures of same.

In a further embodiment of the first aspect, the invention relates to compounds of formula (I), wherein:

X represents a group selected from

$CH_2$, $(CH_2)_2$, $(CH_2)_3$, $(CH_2)_4$ and $*-(CH_2)_2-O-CH_2-^{\#}$,

wherein * indicates the point of attachment to Ar and # indicates the point of attachment to the acetic acid moiety, and stereoisomers, tautomers, N-oxides, hydrates, solvates, or salts thereof, or mixtures of same.

In a further embodiment of the first aspect, the invention relates to compounds of formula (I), wherein:

X represents a group selected from $CH_2$ and $(CH_2)_2$,
and stereoisomers, tautomers, N-oxides, hydrates, solvates, or salts thereof, or mixtures of same.

In a further embodiment of the first aspect, the invention relates to compounds of formula (I), wherein:

X represents $(CH_2)$,
and stereoisomers, tautomers, N-oxides, hydrates, solvates, or salts thereof, or mixtures of same.

In a further embodiment of the first aspect, the invention relates to compounds of formula (I), wherein:

$R^1$ represents a hydrogen atom or a group selected from $C_1$-$C_3$-alkyl, $-CH_2OH$, $-(CH_2)_2OH$ and $-CH_2OCH_3$,
and stereoisomers, tautomers, N-oxides, hydrates, solvates, or salts thereof, or mixtures of same.

In a further embodiment of the first aspect, the invention relates to compounds of formula (I), wherein:

$R^1$ represents a hydrogen atom or a group selected from $-CH_2OH$ and $-(CH_2)_2OH$,
and stereoisomers, tautomers, N-oxides, hydrates, solvates, or salts thereof, or mixtures of same.

In a further embodiment of the first aspect, the invention relates to compounds of formula (I), wherein:

$R^1$ represents a hydrogen atom or a $-CH_2OH$ group,
and stereoisomers, tautomers, N-oxides, hydrates, solvates, or salts thereof, or mixtures of same.

In a further embodiment of the first aspect, the invention relates to compounds of formula (I), wherein:

$R^1$ represents a hydrogen atom,
and stereoisomers, tautomers, N-oxides, hydrates, solvates, or salts thereof, or mixtures of same.

In a further embodiment of the first aspect, the invention relates to compounds of formula (I), wherein:

$R^2$ represents a group selected from $C_2$-$C_6$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy goups and $C_2$-$C_6$-alkoxy groups are optionally substituted, one, two, three or four times, with a fluorine atom,
and stereoisomers, tautomers, N-oxides, hydrates, solvates, or salts thereof, or mixtures of same.

In a further embodiment of the first aspect, the invention relates to compounds of formula (I), wherein:

$R^2$ represents a group selected from $C_2$-$C_4$-alkoxy, $(H_3C\text{-}CH_2O)\text{-}(CH_2)_2\text{-}O\text{-}$, $(H_3C\text{-}CH_2O)\text{-}(CH_2)_2\text{-}O\text{-}(CH_2)_2\text{-}O\text{-}$ and $(H_3C\text{-}CH_2O)\text{-}(CH_2)_2\text{-}O\text{-}(CH_2)_2\text{-}O\text{-}(CH_2)_2\text{-}O\text{-}$,
wherein said $C_2$-$C_4$-alkoxy group is optionally substituted, one, two, three or four times, with a fluorine atom,
and stereoisomers, tautomers, N-oxides, hydrates, solvates, or salts thereof, or mixtures of same.

In a further embodiment of the first aspect, the invention relates to compounds of formula (I), wherein:

$R^2$ represents a group selected from $(H_3C\text{-}CH_2O)\text{-}(CH_2)_2\text{-}O\text{-}$, $(H_3C\text{-}CH_2O)\text{-}(CH_2)_2\text{-}O\text{-}(CH_2)_2\text{-}O\text{-}$and $(H_3C\text{-}CH_2O)\text{-}(CH_2)_2\text{-}O\text{-}(CH_2)_2\text{-}O\text{-}(CH_2)_2\text{-}O\text{-}$,
and stereoisomers, tautomers, N-oxides, hydrates, solvates, or salts thereof, or mixtures of same.

In a further embodiment of the first aspect, the invention relates to compounds of formula (I), wherein:

$R^3$ and $R^4$ represent, independently from each other, a hydrogen atom or a group selected from $C_2$-$C_5$-alkoxy, $(C_1\text{-}C_3\text{-alkoxy})\text{-}(CH_2)_2\text{-}O\text{-}$, $(C_1\text{-}C_3\text{-alkoxy})\text{-}(CH_2)_2\text{-}O\text{-}(CH_2)_2\text{-}O\text{-}$ and $(C_1\text{-}C_3\text{-alkoxy})\text{-}(CH_2)_2\text{-}O\text{-}(CH_2)_2\text{-}O\text{-}(CH_2)_2\text{-}O\text{-}$,
and stereoisomers, tautomers, hydrates, solvates, or salts thereof, or mixtures of same.

In a further embodiment of the first aspect, the invention relates to compounds of formula (I), wherein:

$R^3$ represents a hydrogen atom and $R^4$ represents a hydrogen atom or a group selected from $C_2$-$C_5$-alkoxy, $(C_1\text{-}C_3\text{-alkoxy})\text{-}(CH_2)_2\text{-}O\text{-}$, $(C_1\text{-}C_3\text{-alkoxy})\text{-}(CH_2)_2\text{-}O\text{-}(CH_2)_2\text{-}O\text{-}$ and $(C_1\text{-}C_3\text{-alkoxy})\text{-}(CH_2)_2\text{-}O\text{-}(CH_2)_2\text{-}O\text{-}(CH_2)_2\text{-}O\text{-}$,
and stereoisomers, tautomers, hydrates, solvates, or salts thereof, or mixtures of same.

In a further embodiment of the first aspect, the invention relates to compounds of formula (I), wherein:

$R^3$ and $R^4$ represent a hydrogen atom,
and stereoisomers, tautomers, hydrates, solvates, or salts thereof, or mixtures of same.

It is to be understood that the present invention relates also to any combination of the embodiments described above.

In the context of the present invention, the compounds of formula (I) may contain one or more chiral centers. When all three $R^1$ groups are a hydrogen atom, the carbon atom attached to X can be in the (R) or (S) configuration. Thus, the present invention includes the (R) and (S) enantiomers of the compounds of formula (I) when all three $R^1$ groups are a hydrogen atom, or mixtures thereof.

Further, when one or more of $R^1$ are different from a hydrogen atom, the carbon atoms attached to $R^1$ can be in the (R) or (S) configuration. Thus, the present invention includes all possible stereoisomers of the compounds of formula (I) when one or more of $R^1$ are different from a hydrogen atom, or mixtures thereof. When one of $R^1$ is different from a hydrogen atom, this includes the RR, SS, RS, SR stereoisomers of the compounds of formula (I), or mixtures thereof. When two of $R^1$ are different from a hydrogen atom, this includes the RRR, SSS, RRS, SSR, SRR, RSS, RSR and SRS stereoisomers of the compounds of formula (I), or mixtures thereof. When all three of $R^1$ are different from a hydrogen atom, this includes the RRRR, SRRR, RSRR, RRSR, RRRS, SSRR, SRSR, SRRS, RSSR, RSRS, RRSS, RSSS, SRSS, SSRS, SSSR, SSSS stereoisomers of the compounds of formula (I), or mixtures thereof.

Another embodiment of the first aspect are compounds of formula (I) selected from the group consisting of:

2,2',2'',2'''-[(2*S*)-2-(4-{2-[2-(2-Methoxyethoxy)ethoxy]ethoxy}benzyl)-1,4,7,10-tetraazacyclododecane-1,4,7,10-tetrayl]tetraacetic acid,
2,2',2'',2'''-[(2*S*)-2-{4-[2-(2-Ethoxyethoxy)ethoxy]benzyl}-1,4,7,10-tetraazacyclododecane-1,4,7,10-tetrayl]tetraacetic acid,
2,2',2'',2'''-[(2*R*)-2-(4-{2-[2-(2-Methoxyethoxy)ethoxy]ethoxy}benzyl)-1,4,7,10-tetraazacyclododecane-1,4,7,10-tetrayl]tetraacetic acid,
2,2',2'',2'''-{(2*S*)-2-[4-(2-Ethoxyethoxy)benzyl]-1,4,7,10-tetraazacyclododecane-1,4,7,10-tetrayl}tetraacetic acid,
2,2',2'',2'''-[(2S)-2-(4-{2-[2-(2-Ethoxyethoxy)ethoxy]ethoxy}benzyl)-1,4,7,10-tetraazacyclododecane-1,4,7,10-tetrayl]tetraacetic acid,
2,2',2'',2'''-[(2S)-2-({5-[2-(2-ethoxyethoxy)ethoxy]pyridin-2-yl}methyl)-1,4,7,10-tetraazacyclododecane-1,4,7,10-tetrayl]tetraacetic acid,
2,2'2'',2'''-[2-(4-Ethoxybenzyl)-1,4,7,10-tetraazacyclododecane-1,4,7,10-tetrayl]tetraacetic acid,

2,2',2",2'''-[(2S)-2-(4-Ethoxybenzyl)-1,4,7,10-tetraazacyclododecane-1,4,7,10-tetrayl]tetraacetic acid and
2,2',2",2'''-[(2S)-2-(4-Butoxybenzyl)-1,4,7,10-tetraazacyclododecane-1,4,7,10-tetrayl]tetraacetic acid

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

Ar represents a group selected from

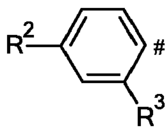 , 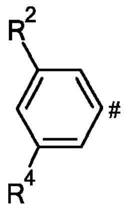 , 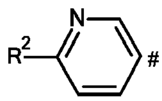 and 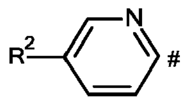 ,

wherein # indicates the point of attachment to X,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

Ar represents a group selected from

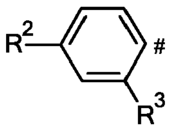 and 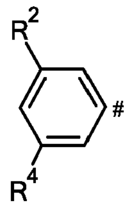

wherein # indicates the point of attachment to X,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

Ar represents a group

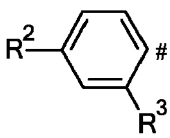 ,

wherein # indicates the point of attachment to X,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

Ar represents a group

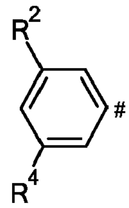

,

wherein # indicates the point of attachment to X,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

X represents a group selected from

$CH_2$, $(CH_2)_2$, $(CH_2)_3$, $(CH_2)_4$ and $*-(CH_2)_2-O-CH_2-^{\#}$,

wherein * indicates the point of attachment to Ar and # indicates the point of attachment to the acetic acid moiety, and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

X represents a group selected from $CH_2$ and $(CH_2)_2$,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

X represents $(CH_2)$,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^1$ represents a hydrogen atom or a group selected from $C_1$-$C_3$-alkyl, $-CH_2OH$, $-(CH_2)_2OH$ and $-CH_2OCH_3$,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^1$ represents a hydrogen atom or a group selected from $-CH_2OH$ and $-(CH_2)_2OH$,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^1$ represents a hydrogen atom or a $-CH_2OH$ group,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^1$ represents a hydrogen atom,
and stereoisomers, tautomers, N-oxides, hydrates, solvates, or salts thereof, or mixtures of same.

In a further embodiment of the second aspect, the invention relates to compounds of formula (I) in the form of

a complex with $Gd^{3+}$, wherein:

$R^2$ represents a group selected from $C_2$-$C_6$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy goups and $C_2$-$C_6$-alkoxy groups are optionally substituted, one, two, three or four times, with a fluorine atom,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^2$ represents a group selected from $C_2$-$C_4$-alkoxy, ($H_3C$-$CH_2O$)-$(CH_2)_2$-O-, ($H_3C$-$CH_2O$)-$(CH_2)_2$-O-$(CH_2)_2$-O- and ($H_3C$-$CH_2O$)-$(CH_2)_2$-O-$(CH_2)_2$-O-$(CH_2)_2$-O-,
wherein said $C_2$-$C_4$-alkoxy group is optionally substituted, one, two, three or four times, with a fluorine atom,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^2$ represents a group selected from ($H_3C$-$CH_2O$)-$(CH_2)_2$-O-, ($H_3C$-$CH_2O$)-$(CH_2)_2$-O-$(CH_2)_2$-O-and ($H_3C$-$CH_2O$)-$(CH_2)_2$-O-$(CH_2)_2$-O-$(CH_2)_2$-O-,
and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^3$ and $R^4$ represent, independently from each other, a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
and stereoisomers, tautomers, hydrates, solvates, or salts thereof, or mixtures of same.

In a further embodiment of the second aspect, the invention relates to compounds of formula (I) in the form of a complex with $Gd^{3+}$, wherein:

$R^3$ represents a hydrogen atom and $R^4$ represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
and stereoisomers, tautomers, hydrates, solvates, or salts thereof, or mixtures of same.

In a further embodiment of the second aspect, the invention relates to compounds of formula (I) in the form of a complex with $Gd^{3+}$, wherein:

$R^3$ and $R^4$ represent a hydrogen atom,
and stereoisomers, tautomers, hydrates, solvates, or salts thereof, or mixtures of same.

It is to be understood that the present invention relates also to any combination of the embodiments described above.

In the context of the present invention, the compounds of formula (I) in the form of a complex with $Gd^{3+}$ may contain one or more chiral centers. When all three of $R^1$ = H, the carbon atom attached to X can be in the (R) or (S) configuration. Thus, the present invention includes the (R) and (S) enantiomers of the compounds of formula (I) in the form of a complex with $Gd^{3+}$ when all three of $R^1$ = H, or mixtures thereof.

Further, when one or more of $R^1$ is/are different from a hydrogen atom, the carbon atoms attached to $R^1$ can be in the (R) or (S) configuration. Thus, the present invention includes all possible stereoisomers of the compounds of formula (I) in the form of a complex with $Gd^{3+}$ when one or more of $R^1$ is/are different from a hydrogen atom, or mixtures thereof. When one of $R^1$ is different from a hydrogen atom, this includes the RR, SS, RS, SR stereoisomers of the compounds of formula (I) in the form of a complex with $Gd^{3+}$, or mixtures thereof. When two of $R^1$ are different from a hydrogen atom, this includes the RRR, SSS, RRS, SSR, SRR, RSS, RSR and SRS stereoisomers of the compounds of formula (I) in the form of a complex with $Gd^{3+}$, or mixtures thereof. When all three of $R^1$ are different from a hydrogen atom, this includes the RRRR, RRRS, RSRR, RSSR, RSRS, RRSS, RRSR, RSSS, SSSS, SSSR, SRSS, SRRS, SRSR, SSRR, SSRS, SRRR stereoisomers of the compounds of formula (I) in the form of a complex with $Gd^{3+}$, or mixtures

thereof.

In accordance with a further embodiment of the second aspect, the present invention covers compounds of general formula (I), *supra,* in the form of a sodium ($Na^+$) salt of a complex with $Gd^{3+}$, wherein Ar, X, $R^1$, $R^2$, $R^3$ and $R^4$ are defined as described in any of the embodiments above, and stereoisomers, tautomers, hydrates, or solvates thereof, or mixtures of same.

Another embodiment of the second aspect are compounds of formula (I) in the form of a complex with $Gd^{3+}$, selected from the group consisting of:

Gadolinium-2,2',2''-[(2*S*)-10-(carboxymethyl)-2-(4-{2-[2-(2-methoxyethoxy)ethoxy] ethoxy}benzyl)-1,4,7,10-tetraazacyclododecane-1,4,7-triyl]triacetate,
Gadolinium-2,2',2''-[(2*S*)-10-(carboxymethyl)-2-{4-[2-(2-ethoxyethoxy)ethoxy]benzyl}-1,4,7,10-tetraazacyclododecane-1,4,7-triyl]triacetate,
Gadolinium-2,2',2''-[(2*R*)-10-(carboxymethyl)-2-(4-{2-[2-(2-methoxyethoxy)ethoxy] ethoxy}benzyl)-1,4,7,10-tetraazacyclododecane-1,4,7-triyl]triacetate,
Gadolinium-2,2',2''-{(2*S*)-10-(carboxymethyl)-2-[4-(2-ethoxyethoxy)benzyl]-1,4,7,10-tetraazacyclododecane-1,4,7-triyl}triacetate,
Gadolinium-2,2',2''-[(2*S*)-10-(carboxymethyl)-2-(4-{2-[2-(2-ethoxyethoxy)ethoxy]ethoxy} benzyl)-1,4,7,10-tetraazacyclododecane-1,4,7-triyl]triacetate,
Gadolinium 2,2',2''-[(2*S*)-10-(carboxymethyl)-2-({5-[2-(2-ethoxyethoxy)ethoxy]pyridin-2-yl}methyl)-1,4,7,10-tetraazacyclododecane-1,4,7-triyl]triacetate
Gadolinium-2,2',2''-[10-(carboxymethyl)-2-(4-ethoxybenzyl)-1,4,7,10-tetraazacyclododecane-1,4,7-triyl]triacetate,
Gadolinium-2,2',2''-[10-(carboxymethyl)-2-(4-ethoxybenzyl)-1,4,7,10-tetraazacyclododecane-1,4,7-triyl]triacetate racemic diasereomer 1,
Gadolinium-2,2',2''-[10-(carboxymethyl)-2-(4-ethoxybenzyl)-1,4,7,10-tetraazacyclododecane-1,4,7-triyl]triacetate racemic diasereomer 2,
Gadolinium-2,2',2''-[(2*S*)-10-(carboxymethyl)-2-(4-ethoxybenzyl)-1,4,7,10-tetraazacyclododecane-1,4,7-triyl]triacetate stereoisomer 1,
Gadolinium-2,2',2''-[(2*S*)-10-(carboxymethyl)-2-(4-ethoxybenzyl)-1,4,7,10-tetraazacyclododecane-1,4,7-triyl]triacetate stereoisomer 2,
Gadolinium-2,2',2''-[(2*S*)-2-(4-butoxybenzyl)-10-(carboxymethyl)-1,4,7,10-tetraaza cyclododecane-1,4,7-triyl]triacetate stereoisomer 1 and
Gadolinium-2,2',2''-[(2*S*)-2-(4-butoxybenzyl)-10-(carboxymethyl)-1,4,7,10-tetraaza cyclododecane-1,4,7-triyl]triacetate stereoisomer 2

and stereoisomers, tautomers, N-oxides, hydrates, solvates, or salts thereof, or mixtures of same.

Another embodiment of the second aspect are compounds of formula (I) in the form of a sodium ($Na^+$) salt of a complex with $Gd^{3+}$, said complex with $Gd^{3+}$ selected from the group consisting of:

Gadolinium-2,2',2''-[(2*S*)-10-(carboxymethyl)-2-(4-{2-[2-(2-methoxyethoxy)ethoxy] ethoxy}benzyl)-1,4,7,10-tetraazacyclododecane-1,4,7-triyl]triacetate,
Gadolinium-2,2',2''-[(2*S*)-10-(carboxymethyl)-2-{4-[2-(2-ethoxyethoxy)ethoxy]benzyl}-1,4,7,10-tetraazacyclododecane-1,4,7-triyl]triacetate,
Gadolinium-2,2',2''-[(2*R*)-10-(carboxymethyl)-2-(4-{2-[2-(2-methoxyethoxy)ethoxy] ethoxy}benzyl)-1,4,7,10-tetraazacyclododecane-1,4,7-triyl]triacetate,
Gadolinium-2,2',2''-{(2*S*)-10-(carboxymethyl)-2-[4-(2-ethoxyethoxy)benzyl]-1,4,7,10-tetraazacyclododecane-1,4,7-triyl}triacetate,
Gadolinium-2,2',2''-[(2*S*)-10-(carboxymethyl)-2-(4-{2-[2-(2-ethoxyethoxy)ethoxy]ethoxy} benzyl)-1,4,7,10-tetraazacyclododecane-1,4,7-triyl]triacetate,
Gadolinium 2,2',2''-[(2*S*)-10-(carboxymethyl)-2-({5-[2-(2-ethoxyethoxy)ethoxy]pyridin-2-yl}methyl)-1,4,7,10-tetraazacyclododecane-1,4,7-triyl]triacetate
Gadolinium-2,2',2''-[10-(carboxymethyl)-2-(4-ethoxybenzyl)-1,4,7,10-tetraazacyclododecane-1,4,7-triyl]triacetate,
Gadolinium-2,2',2''-[10-(carboxymethyl)-2-(4-ethoxybenzyl)-1,4,7,10-tetraazacyclododecane-1,4,7-triyl]triacetate racemic diasereomer 1,
Gadolinium-2,2',2''-[10-(carboxymethyl)-2-(4-ethoxybenzyl)-1,4,7,10-tetraazacyclododecane-1,4,7-triyl]triacetate racemic diasereomer 2,
Gadolinium-2,2',2''-[(2*S*)-10-(carboxymethyl)-2-(4-ethoxybenzyl)-1,4,7,10-tetraazacyclododecane-1,4,7-triyl]triacetate stereoisomer 1,
Gadolinium-2,2',2''-[(2*S*)-10-(carboxymethyl)-2-(4-ethoxybenzyl)-1,4,7,10-tetraazacyclododecane-1,4,7-triyl]triacetate stereoisomer 2,
Gadolinium-2,2',2"-[(2*S*)-2-(4-butoxybenzyl)-10-(carboxymethyl)-1,4,7,10-tetraaza cyclododecane-1,4,7-triyl]triacetate stereoisomer 1 and
Gadolinium-2,2',2"-[(2*S*)-2-(4-butoxybenzyl)-10-(carboxymethyl)-1,4,7,10-tetraaza cyclododecane-1,4,7-triyl]triacetate stereoisomer 2

and stereoisomers, tautomers, N-oxides, hydrates or solvates thereof, or mixtures of same.

In a further embodiment of the third aspect, the invention relates to compounds of formula (I) in the form of a metal complex with a metal ion suitable for computed tomography, wherein: Ar represents a group selected from

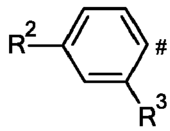 , 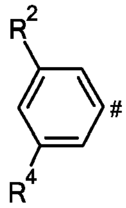 , 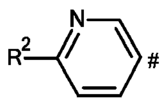 and 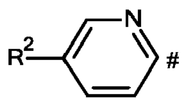 ,

wherein # indicates the point of attachment to X,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: Ar represents a group selected from

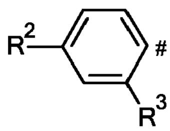 and 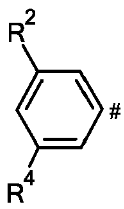

wherein # indicates the point of attachment to X,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: Ar represents a group

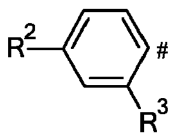 ,

wherein # indicates the point of attachment to X,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: Ar represents a group

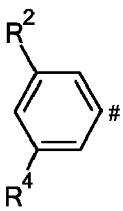 ,

wherein # indicates the point of attachment to X,
and stereoisomers, tautomers, N-oxides, hydrates, solvates, or salts thereof, or mixtures of same.

In a further embodiment of the third aspect, the invention relates to compounds of formula (I) in the form of a

metal complex with a metal ion suitable for computed tomography, wherein: X represents a group selected from

$CH_2$, $(CH_2)_2$, $(CH_2)_3$, $(CH_2)_4$ and $*-(CH_2)_2-O-CH_2-^{\#}$,

wherein * indicates the point of attachment to Ar and # indicates the point of attachment to the acetic acid moiety, and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: X represents a group selected from $CH_2$ and $(CH_2)_2$,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: X represents $(CH_2)$,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: $R^1$ represents a hydrogen atom or a group selected from $C_1$-$C_3$-alkyl, $-CH_2OH$, $-(CH_2)_2OH$ and $-CH_2OCH_3$,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: $R^1$ represents a hydrogen atom or a group selected from $-CH_2OH$ and $-(CH_2)_2OH$,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: $R^1$ represents a hydrogen atom or a $-CH_2OH$ group,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: $R^1$ represents a hydrogen atom,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: $R^2$ represents a group selected from $C_2$-$C_6$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,

wherein said $C_1$-$C_3$-alkoxy goups and $C_2$-$C_6$-alkoxy groups are optionally substituted, one, two, three or four times, with a fluorine atom,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: $R^2$ represents a group selected from $C_2$-$C_4$-alkoxy, $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$,

wherein said $C_2$-$C_4$-alkoxy group is optionally substituted, one, two, three or four times, with a fluorine atom,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: $R^2$ represents a group selected from $(H_3C-CH_2O)-(CH_2)_2-O-$, $(H_3C-CH_2O)-(CH_2)_2-O-(CH_2)_2-O-$and $(H_3C-CH_2O)-(CH_2)_2-O-(CH_2)_2-O-(CH_2)_2-O-$,
and stereoisomers, tautomers, N-oxides, hydrates, solvates, or salts thereof, or mixtures of same.

In a further embodiment of the third aspect, the invention relates to compounds of formula (I) in the form of a metal complex with a metal ion suitable for computed tomography, wherein: $R^3$ and $R^4$ represent, independently from each other, a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
and stereoisomers, tautomers, hydrates, solvates, or salts thereof, or mixtures of same.

In a further embodiment of the third aspect, the invention relates to compounds of formula (I) in the form of a metal complex with a metal ion suitable for computed tomography, wherein: $R^3$ represents a hydrogen atom and $R^4$ represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,

and stereoisomers, tautomers, hydrates, solvates, or salts thereof, or mixtures of same.

In a further embodiment of the third aspect, the invention relates to compounds of formula (I) in the form of a metal complex with a metal ion suitable for computed tomography, wherein: $R^3$ and $R^4$ represent a hydrogen atom, and stereoisomers, tautomers, hydrates, solvates, or salts thereof, or mixtures of same.

In accordance with another aspect, the present invention covers methods of preparing compounds of the present invention, said methods comprising the steps as described in the Experimental Section herein.

In accordance with a further aspect, the present invention covers intermediate compounds which are useful for the preparation of the compounds of general formula (I), and particularly for the preparation of the compounds of general formula (I) in the form of a complex with $Gd^{3+}$, *supra* or of the compounds of formula (I) in the form of a metal complex with a metal ion suitable for computed tomography.

Particularly, the invention covers intermediate compounds of general formula (II):

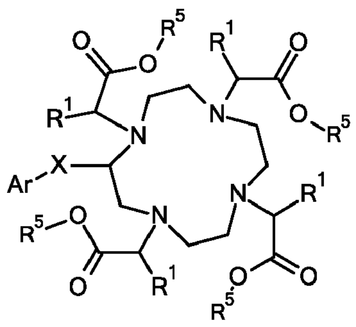

(II),

and stereoisomers, tautomers, N-oxides, hydrates, solvates, or salts thereof, or mixtures of same in which Ar, X, $R^1$, $R^2$ and $R^3$ are as defined for the compounds of general formula (I), *supra,* and $R^5$ represents a group selected from methyl, ethyl and tert.-butyl.

Particularly, the invention covers intermediate compounds of general formula (III):

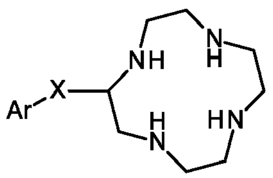

(III) ,

and stereoisomers, tautomers, N-oxides, hydrates, solvates, or salts thereof, or mixtures of same in which Ar and X are as defined for the compounds of general formula (I), *supra,*

In accordance with a further aspect, the present invention covers the use of the intermediate compounds of general formula (II):

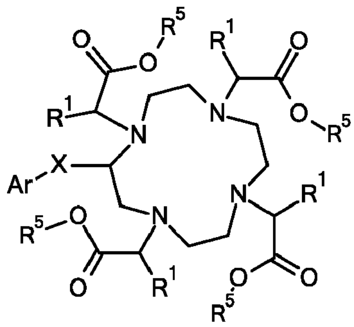

(II) ,

and stereoisomers, tautomers, N-oxides, hydrates, solvates, or salts thereof, or mixtures of same in which Ar, X and $R^1$ are as defined for the compounds of general formula (I), *supra,* and $R^5$ represents a group selected from methyl, ethyl and *tert.*-butyl, for the preparation of a compound of general formula (I), as defined *supra.*

In accordance with a further aspect, the present invention covers the use of the intermediate compounds of general formula (II):

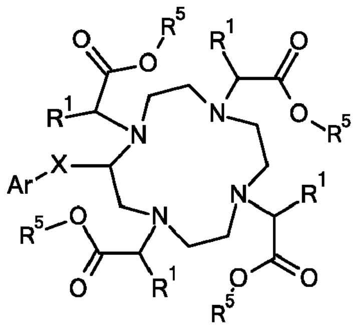

(II) ,

and stereoisomers, tautomers, N-oxides, hydrates, solvates, or salts thereof, or mixtures of same in which Ar, X and $R^1$ are as defined for the compounds of general formula (I), *supra,* and $R^5$ represents a group selected from methyl, ethyl and *tert.*-butyl, for the preparation of a compound of general formula (I) in the form of a complex with $Gd^{3+}$, as defined *supra* or of a compound of formula (I) in the form of a metal complex with a metal ion suitable for computed tomography, as defined *supra.*

In accordance with a further aspect, the present invention covers the use of the intermediate compounds of general formula (III):

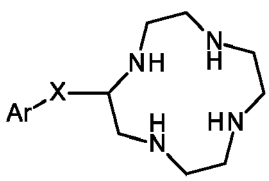

(III) ,

and stereoisomers, tautomers, N-oxides, hydrates, solvates, or salts thereof, or mixtures of same in which Ar and X are as defined for the compounds of general formula (I), *supra,* for the preparation of a compound of general formula (I), as defined *supra.*

In accordance with a further aspect, the present invention covers the use of the intermediate compounds of general formula (III):

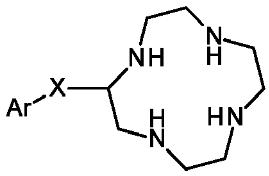

(III) ,

and stereoisomers, tautomers, N-oxides, hydrates, solvates, or salts thereof, or mixtures of same in which Ar and X are as defined for the compounds of general formula (I), *supra,* for the preparation of a compound of general formula (I) in the form of a complex with $Gd^{3+}$, as defined *supra* or of a compound of formula (I) in the form of a metal complex with a metal ion suitable for computed tomography, as defined *supra.*

More particularly still, the present invention covers the intermediate compounds which are disclosed in the Experimental Section of this text, *infra.*

Compounds of general formula (I) in the form of a $Gd^{3+}$ complex, *i.e.* $Gd^{3+}$ complexes of the compounds of general formula (I) of the present invention demonstrate a valuable complex stability, solubility, uptake into hepatocytes, relaxivity and a valuable tolerability and pharmacokinetic profile, which could not have been predicted. $Gd^{3+}$-containing compounds of general formula (I) of the present invention have surprisingly been found to effectively taken up into hepatocytes and providing a high relaxivity while maintaining a pharmacokinetic, good safety and tolerability profile and it is possible therefore that said compounds be used for diagnostic imaging. In particular, the compounds of formula (I) of the present invention can be used as contrast agents in contrast-enhanced MRI (CE-MRI), preferably multi-purpose MRI and liver MRI and more preferably for the detection of liver diseases like liver fibrosis, cirrhosis, metastases, hepatobiliary diseases and differential diagnosis of focal liver lesions and functional imaging in humans and animals. Additionally, the compounds of the present invention may be used as contrast agents in contrast-enhanced computed tomography imaging (CT imaging, CT), particularly to enhance vessel contrast in CT angiography of different body regions (cerebral, thoracic, cardiac, abdominal, extremities) and to enhance the parenchymal organ contrast preferably for the detection of liver diseases like liver fibrosis, cirrhosis, metastases, primary liver cancer, hepatobiliary diseases and differential diagnosis of focal liver lesions and functional liver imaging in humans and animals.

A further aspect of the invention is the use of a compound of general formula (I), *supra,* for diagnostic imaging.

A person skilled in the art would recognize that the compounds of general formula (I) of this invention can be used in combination with other MR-active metal ions instead of $Gd^{3+}$, such compounds also being encompassed by the present invention.

A further aspect of the invention is the use of a compound of general formula (I), *supra,* in the form of a complex with $Gd^{3+}$ for diagnostic imaging.

A further aspect of the invention is the use of a $Gd^{3+}$ complex of a compound of general formula *(I), supra,* for diagnostic imaging.

A further aspect of the invention is the use of a complex of a compound of general formula (I), in the form of a metal complex with a metal ion suitable for computed tomography, *supra,* for diagnostic imaging.

Preferably, the use of a compound of the invention, *i.e.* of a compound of general formula (I), *supra* and/or of a compound of general formula (I), *supra,* in the form of a complex with $Gd^{3+}$ and/or of a $Gd^{3+}$ complex of a compound of general formula (I), *supra,* in the diagnosis is performed using magnetic resonance imaging (MRI).

Preferably, the use of a compound of the invention, *i.e.* of a compound of general formula (I), *supra* and/or of a compound of general formula (I), *supra,* in the form of a complex with $Gd^{3+}$ and/or of a $Gd^{3+}$ complex of a compound of general formula (I), *supra,* in the diagnosis is performed using magnetic resonance imaging (MRI) or computed tomography (CT).A further aspect of the invention is the use of a compound of general formula (I), *supra,* for magnetic resonance imaging, preferably of the vascular, renal or of the hepatobiliary system or of the gastrointestinal tract, more preferably for magnetic resonance imaging of the liver.

A further aspect of the invention is the use of a compound of general formula (I), *supra,* in the form of a complex with $Gd^{3+}$ for magnetic resonance imaging, preferably of the vascular, renal or of the hepatobiliary system or of the gastrointestinal tract, more preferably for magnetic resonance imaging of the liver.

A further aspect of the invention is the use of a $Gd^{3+}$ complex of a compound of general formula *(I), supra,* for magnetic resonance imaging, preferably of the vascular, renal or of the hepatobiliary system or of the gastrointestinal tract, more preferably for magnetic resonance imaging of the liver.

Preferably, the use of a compound of the invention, *i.e.* of a compound of general formula (I), *supra* and/or of a compound of general formula (I), *supra,* in the form of a complex with $Gd^{3+}$ and/or of a $Gd^{3+}$ complex of a compound of general formula (I), *supra,* in the diagnosis is performed using computed tomography (CT).

A further aspect of the invention is the use of a compound of general formula (I), *supra,* for computed tomography

imaging, preferably of the vascular, renal or of the hepatobiliary system or of the gastrointestinal tract, more preferably for computed tomography imaging of the liver.

A further aspect of the invention is the use of a compound of general formula (I), *supra,* in the form of a complex with $Gd^{3+}$ for computed tomography imaging, preferably of the vascular, renal or of the hepatobiliary system or of the gastrointestinal tract, more preferably for computed tomography imaging of the liver.

A further aspect of the invention is the use of a $Gd^{3+}$ complex of a compound of general formula *(I), supra,* for computed tomography imaging, preferably of the vascular, renal or of the hepatobiliary system or of the gastrointestinal tract, more preferably for computed tomography imaging of the liver.

Preferably, the use of a compound of the invention, *i.e.* of a complex of a compound of general formula (I), in the form of a metal complex with a metal ion suitable for computed tomography, *supra,* in the diagnosis is performed using computed tomography (CT).

A further aspect of the invention is the use of a compound of general formula (I), *supra,* for computed tomography imaging, preferably of the vascular, renal or of the hepatobiliary system or of the gastrointestinal tract, more preferably for computed tomography imaging of the liver.

A further aspect of the invention is the use of a compound of general formula (I), *supra,* in the form of a metal complex with a metal ion suitable for computed tomography, for computed tomography imaging, preferably of the vascular, renal or of the hepatobiliary system or of the gastrointestinal tract, more preferably for computed tomography imaging of the liver.

A further aspect of the invention is the use of a complex of a compound of general formula (I), in the form of a metal complex with a metal ion suitable for computed tomography, *supra,* for computed tomography imaging, preferably of the vascular, renal or of the hepatobiliary system or of the gastrointestinal tract, more preferably for computed tomography imaging of the liver.

A further aspect of the invention are compounds of general formula (I) for use in diagnostic imaging.

A further aspect of the invention are compounds of general formula (I) in the form of a complex with $Gd^{3+}$ for use in diagnostic imaging.

A further aspect of the invention are $Gd^{3+}$ complexes of compounds of general formula (I), *supra,* for use in diagnostic imaging.

A further aspect of the invention are compounds of general formula (I) in the form of a metal complex with a metal ion suitable for computed tomography, for use in diagnostic imaging.A further aspect of the invention are compounds of general formula (I) for use in magnetic resonance imaging (MRI), preferably of the vascular, renal or of the hepatobiliary system or of the gastrointestinal tract, more preferably for magnetic resonance imaging of the liver.

A further aspect of the invention are compounds of general formula (I) in the form of a complex with $Gd^{3+}$ for use in magnetic resonance imaging (MRI), preferably of the vascular, renal or of the hepatobiliary system or of the gastrointestinal tract, more preferably for magnetic resonance imaging of the liver.

A further aspect of the invention are $Gd^{3+}$ complexes of compounds of general formula (I), *supra,* for use in magnetic resonance imaging (MRI), preferably of the vascular, renal or of the hepatobiliary system or of the gastrointestinal tract, more preferably for magnetic resonance imaging of the liver.

A person skilled in the art would recognize that the compounds of general formula (I) of this invention can be used in combination with other MR-active metal ions instead of $Gd^{3+}$, such compounds also being encompassed by the present invention.

A further aspect of the invention are compounds of general formula (I) for use in computed tomography imaging, preferably of the vascular, renal or of the hepatobiliary system or of the gastrointestinal tract, more preferably for computed tomography imaging of the liver.

A further aspect of the invention are compounds of general formula (I) in the form of a complex with $Gd^{3+}$ for use in computed tomography imaging, preferably of the vascular, renal or of the hepatobiliary system or of the gastrointestinal tract, more preferably for computed tomography imaging of the liver.

A further aspect of the invention are $Gd^{3+}$ complexes of compounds of general formula (I), *supra,* for use in computed tomography imaging, preferably of the vascular, renal or of the hepatobiliary system or of the gastrointestinal tract, more preferably for computed tomography imaging of the liver.

A further aspect of the invention are compounds of general formula (I) in the form of a metal complex with a metal ion suitable for computed tomography, for use in computed tomography imaging, preferably of the vascular, renal or of the hepatobiliary system or of the gastrointestinal tract, more preferably for computed tomography imaging of the liver.

A further aspect of the invention are compounds of general formula (I) in the form of a metal complex with a metal ion suitable for computed tomography, for use in computed tomography imaging, preferably of the vascular, renal or of the hepatobiliary system or of the gastrointestinal tract, more preferably for computed tomography imaging of the liver.

The invention also contains compounds of general formula (I) for the manufacture of diagnostic agents.

The invention also contains compounds of general formula (I) in the form of a complex with $Gd^{3+}$ for the manufacture of diagnostic agents.

A further aspect of the invention is the use of the compounds of general formula (I) or mixtures thereof for the manufacture of diagnostic agents.

A further aspect of the invention is the use of the compounds of general formula (I) in the form of a complex with $Gd^{3+}$ or mixtures thereof for the manufacture of diagnostic agents.

The invention also contains compounds of general formula (I) in the form of a metal complex with a metal ion suitable for computed tomography, for the manufacture of diagnostic agents.

A further aspect of the invention is the use of the compounds of general formula (I) or mixtures thereof for the manufacture of diagnostic agents for magnetic resonance imaging (MRI).

A further aspect of the invention is the use of the compounds of general formula (I) in the form of a complex with $Gd^{3+}$ or mixtures thereof for the manufacture of diagnostic agents for magnetic resonance imaging (MRI).

A further aspect of the invention is the use of the compounds of general formula (I) or mixtures thereof for the manufacture of diagnostic agents for magnetic resonance imaging (MRI) of the vascular, vascular, renal or of the hepatobiliary system or of the gastrointestinal tract, preferably for magnetic resonance imaging of the liver.

A further aspect of the invention is the use of the compounds of general formula (I) in the form of a complex with $Gd^{3+}$ or mixtures thereof for the manufacture of diagnostic agents for magnetic resonance imaging (MRI) of the vascular, renal or of the hepatobiliary system or of the gastrointestinal tract, preferably for magnetic resonance imaging of the liver.

A further aspect of the invention is the use of $Gd^{3+}$ complexes of compounds of general formula *(I), supra,* or mixtures thereof for the manufacture of diagnostic agents for magnetic resonance imaging (MRI) of the vascular, renal or of the hepatobiliary system or of the gastrointestinal tract, preferably for magnetic resonance imaging of the liver.

A further aspect of the invention is the use of the compounds of general formula (I) in the form of a metal complex with a metal ion suitable for computed tomography, or mixtures thereof for the manufacture of diagnostic agents.

A further aspect of the invention is the use of the compounds of general formula (I) or mixtures thereof for the manufacture of diagnostic agents for computed tomography (CT).

A further aspect of the invention is the use of the compounds of general formula (I) in the form of a complex with $Gd^{3+}$ or mixtures thereof for the manufacture of diagnostic agents for computed tomography (CT).

A further aspect of the invention is the use of the compounds of general formula (I) in the form of a metal complex with a metal ion suitable for computed tomography, or mixtures thereof for the manufacture of diagnostic agents for computed tomography (CT).

A further aspect of the invention is the use of the compounds of general formula (I) or mixtures thereof for the manufacture of diagnostic agents for computed tomography imaging of the vascular, vascular, renal or of the hepatobiliary system or of the gastrointestinal tract, preferably for computed tomography imaging of the liver.

A further aspect of the invention is the use of the compounds of general formula (I) in the form of a complex with $Gd^{3+}$ or mixtures thereof for the manufacture of diagnostic agents for computed tomography imaging of the vascular, renal or of the hepatobiliary system or of the gastrointestinal tract, preferably for computed tomography imaging of the liver.

A further aspect of the invention is the use of $Gd^{3+}$ complexes of compounds of general formula *(I), supra,* or mixtures thereof for the manufacture of diagnostic agents for computed tomography imaging of the vascular, renal or of the hepatobiliary system or of the gastrointestinal tract, preferably for computed tomography imaging of the liver.

A further aspect of the invention is the use of the compounds of general formula (I) in the form of a metal complex with a metal ion suitable for computed tomography, or mixtures thereof for the manufacture of diagnostic agents for computed tomography imaging of the vascular, renal or of the hepatobiliary system or of the gastrointestinal tract, preferably for computed tomography imaging of the liver.

A further aspect of the invention is a method of imaging body tissue in a patient, comprising the steps of administering to the patient an effective amount of one or more compounds of general formula (I) in the form of a complex with $Gd^{3+}$ in a pharmaceutically acceptable carrier, and subjecting the patient to NMR tomography.

A further aspect of the invention is a method of imaging body tissue in a patient, comprising the steps of administering to the patient an effective amount of one or more $Gd^{3+}$ complexes of the compounds of general formula (I) in a pharmaceutically acceptable carrier, and subjecting the patient to NMR tomography.

A further aspect of the invention is a method of imaging body tissue in a patient, comprising the steps of administering to the patient an effective amount of one or more compounds of general formula (I) in the form of a complex with $Gd^{3+}$ in a pharmaceutically acceptable carrier, and subjecting the patient to computed tomography (CT).

A further aspect of the invention is a method of imaging body tissue in a patient, comprising the steps of administering to the patient an effective amount of one or more $Gd^{3+}$ complexes of the compounds of general formula (I) in a pharmaceutically acceptable carrier, and subjecting the patient to computed tomography (CT).

A further aspect of the invention is a method of imaging body tissue in a patient, comprising the steps of administering to the patient an effective amount of one or more compounds of general formula (I) in the form of a metal complex with a metal ion suitable for computed tomography, in a pharmaceutically acceptable carrier, and subjecting the patient to computed tomography (CT).

For the manufacture of diagnostic agents, for example the administration to human or animal subjects, the compounds of general formula (I) in the form of a metal complex with a metal ion suitable for computed tomography, or in the form of complexes with $Gd^{3+}$, *i.e.* $Gd^{3+}$ complexes of the compounds of general formula (I) or mixtures will conveniently be formulated together with pharmaceutical carriers or excipients. The contrast media of the invention may conveniently contain pharmaceutical formulation aids, for example stabilizers, antioxidants, pH-adjusting agents, metal scavengers, electrolytes (e.g. sodium chloride), flavors and the like. The diagnostic agents of the invention may be formulated for parenteral or enteral administration or for direct administration into body cavities. For example, in the case of $Gd^{3+}$ complexes of the compounds of general formula (I), parenteral formulations contain a sterile solution or suspension in a dose of 0.0001-5 mmol gadolinium/kg body weight, preferably 0.001-0.5 mmol gadolinium/kg body weight, more preferably 0.005-0.1 mmol gadolinium/kg body weight of the compound of formula (I) according to this invention. Thus, the media of the invention may be in conventional pharmaceutical formulations such as solutions, suspensions, dispersions, syrups, etc. in physiologically acceptable carrier media, preferably in water for injections. When the contrast medium is formulated for parenteral administration, it will be preferably isotonic or hypertonic and close to pH 7.4.

In a further aspect, the invention is directed to a method of diagnosing and health monitoring of patients. This method comprises a) administering to a human in need of such diagnosis a compound of the invention for detecting the compound in the human as described above and herein, and b) measuring the signal arising from the administration of the compound to the human, preferably by magnetic resonance imaging (MRI).

In a further aspect, the invention is directed to a method of diagnosing and health monitoring of patients. This method comprises a) administering to a human in need of such diagnosis a compound of the invention, *i.e.* a compound of general formula (I) in the form of a complex with $Gd^{3+}$ for detecting the compound in the human as described above and herein, and b) measuring the signal arising from the administration of the compound to the human, preferably by magnetic resonance imaging (MRI).

In a further aspect, the invention is directed to a method of diagnosing and health monitoring of patients. This method comprises a) administering to a human in need of such diagnosis a compound of the invention, *i.e.* a $Gd^{3+}$ complex of a compound of general formula (I) for detecting the compound in the human as described above and herein, and b) measuring the signal arising from the administration of the compound to the human, preferably by magnetic resonance imaging (MRI).

In a further aspect, the invention is directed to a method of diagnosing and health monitoring of patients. This method comprises a) administering to a human in need of such diagnosis a compound of the invention for detecting the compound in the human as described above and herein, and b) measuring the signal arising from the administration of the compound to the human, preferably by computed tomography imaging.

In a further aspect, the invention is directed to a method of diagnosing and health monitoring of patients. This method comprises a) administering to a human in need of such diagnosis a compound of the invention, i.e. a compound of general formula (I) in the form of a complex with $Gd^{3+}$ for detecting the compound in the human as described above and herein, and b) measuring the signal arising from the administration of the compound to the human, preferably by computed tomography imaging.

In a further aspect, the invention is directed to a method of diagnosing and health monitoring of patients. This method comprises a) administering to a human in need of such diagnosis a compound of the invention, i.e. a $Gd^{3+}$ complex of a compound of general formula (I) for detecting the compound in the human as described above and herein, and b) measuring the signal arising from the administration of the compound to the human, preferably by computed tomography imaging.

In a further aspect, the invention is directed to a method of diagnosing and health monitoring of patients. This method comprises a) administering to a human in need of such diagnosis a compound of the invention, i.e. a compound of general formula (I) in the form of a metal complex with a metal ion suitable for computed tomography, for detecting the compound in the human as described above and herein, and b) measuring the signal arising from the administration of the compound to the human, preferably by computed tomography imaging.

## GENERAL SYNTHESIS

The compounds of general formula (I) described in this invention, including the compounds of general formula (I) in the form of a complex with $Gd^{3+}$ as well as the compounds of general formula (I) in the form of a metal complex with a metal ion suitable for computed tomography can be produced using the following general procedures depicted in Schemes 1 and 2 below. Unless otherwise specified, the groups $R^1$ to $R^7$ displayed therein have the meaning given in the description above. In case protective group chemistry is required for the introduction of one or more of the groups $R^1$, the groups $R^6$ can be employed. Thus, should no protective group chemistry be needed, the group $R^6$ equals the group $R^1$, In general, but not exclusively, the following groups are selected from $R^5$ = methyl, ethyl or tert-Butyl, and $R^7$ = methoxy, trifluoromethoxy.

For example, the compounds of general formula (I) described in this invention, including the compounds of general formula (I) in the form of a complex with $Gd^{3+}$ as well as the compounds of general formula (I) in the form of a metal complex with a metal ion suitable for computed tomography can be prepared by the procedure depicted in Scheme 1, where Ar-X-substituted cyclen is formed by an acid catalyzed tetramerization of benzylaziridines followed by benzyl deprotection.

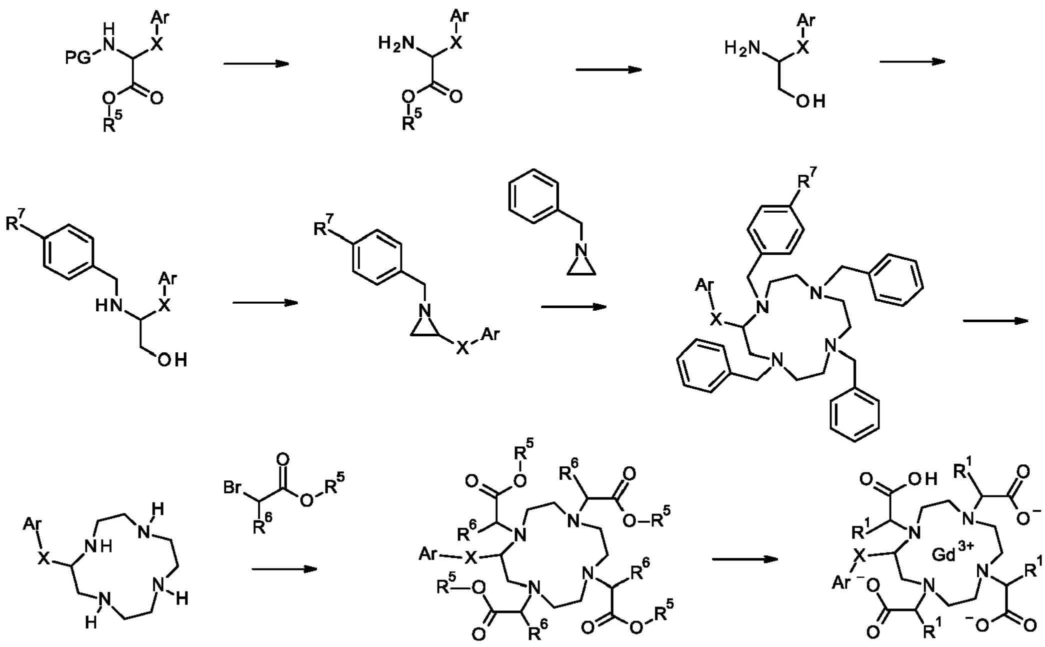

**Scheme 1.**

Alternatively, the compounds of general formula (I) described in this invention, including the compounds of general formula (I) in the form of a complex with $Gd^{3+}$ as well as the compounds of general formula (I) in the form of a metal complex with a metal ion suitable for computed tomography, particularly when $R^1$ = hydrogen, can be prepared according to the procedure depicted in Scheme 2, where Ar-X-substituted cyclen is formed by diamide formation followed by amide reduction.

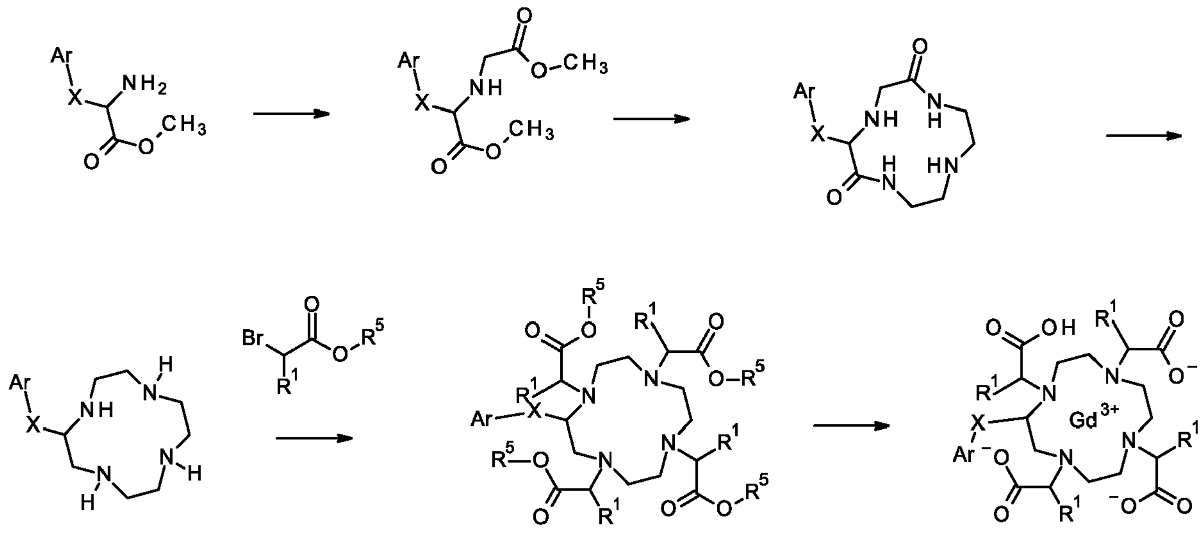

**Scheme 2.**

Literature for the synthetic procedures shown in scheme 1 and 2 can be found (see for example L. Dai, J. Zhang, Y. Chen,L.E. Mackenzie, R. Pal, and G.-L. Law Inorg. Chem. 2019, 58, 12506-12510, Q. Yuan , P. Xue , M. Fang , E. Fu and C. Wu Synthetic Communications 2003, 33:11, 1911-1916, WO 2019/051197 and WO 97/31905).

The schemes and procedures described above illustrate synthetic routes to the compounds of general formula (I) of the invention and are not intended to be limiting. It would be apparent to the person skilled in the art that the order of transformations as exemplified in the schemes can be modified in various ways. The order of transformations exemplified in the schemes is therefore not intended to be limiting. Appropriate protecting groups and their introduction and cleavage are well-known to the person skilled in the art (see for example T.W. Greene and P.G.M. Wuts in Protective Groups in Organic Synthesis, 3rd edition, Wiley 1999). Specific examples are described in the subsequent paragraphs.

The contents of the documents which are cited herein are hereby incorporated by reference.

In accordance with an embodiment, the present invention also relates to a method of preparing a compound of general formula (I) in the form of a complex with $Gd^{3+}$, *i.e.* a $Gd^{3+}$ complex of a compound of general formula (I), as defined *supra,* said method comprising the step of allowing a compound of general formula (I):

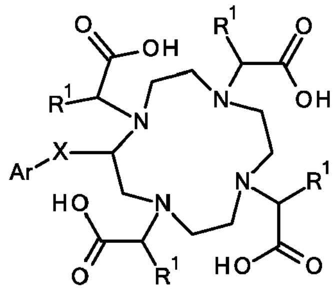

(**I**),

in which Ar, X and $R^1$ are as defined for the compounds of general formula (I), *supra,*
to react with a Gadolinium-(III)-salt,
thereby giving a compound of general formula (I), in which Ar, X and $R^1$ are as defined for the compounds of general formula (I), *supra,* in the form of a complex with $Gd^{3+}$, *i.e.* a $Gd^{3+}$ complex of a compound of general formula (I).

In accordance with a further embodiment, the present invention also relates to a method of preparing a compound of general formula (I) in the form of a complex with $Gd^{3+}$, *i.e.* a $Gd^{3+}$ complex of a compound of general formula (I) as defined *supra,* said method comprising the step of allowing a compound of general formula (I):

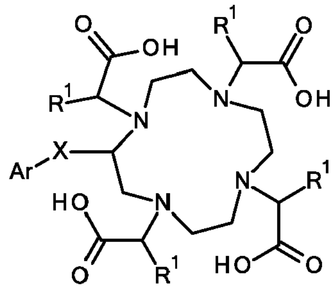

(**I**),

in which Ar, X and $R^1$ are as defined for the compounds of general formula (I), *supra,*
to react with a Gadolinium-(III)-salt, such as gadolinium oxide, gadolinium chloride, gadolinium acetate or gadolinium carbonate, thereby giving a compound of general formula (I), in which Ar, X and $R^1$ are as defined for the compounds of general formula (I), *supra,* in the form of a complex with $Gd^{3+}$, *i.e.* a $Gd^{3+}$ complex of a compound of general formula (I).

In accordance with a further embodiment, the present invention also relates to a method of preparing a compound of general formula (I) in the form of a complex with $Gd^{3+}$, *i.e.* a $Gd^{3+}$ complex of a compound of general formula (I) as defined *supra,* said method comprising the step of allowing a compound of general formula (I):

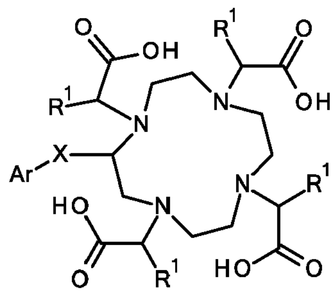

(**I**),

in which Ar, X and $R^1$ are as defined for the compounds of general formula (I), *supra,* to react with gadolinium (III) oxide,
thereby giving a compound of general formula (I), in which Ar, X and $R^1$ are as defined for the compounds of general formula (I), *supra,* in the form of a complex with $Gd^{3+}$, *i.e.* a $Gd^{3+}$ complex of a compound of general formula (I).

**DESCRIPTION OF THE FIGURES**

**Figure 1** Chemical stability of selected Examples during heat sterilization. Normalized HPLC-ICP-MS (Gd157) signal over time before and after one-, two- and three-times autoclaving (1 bar, 121°C for 20 min). All compounds were investigated at 1 mmol/L concentration in 10 mM Tris-HCl buffer at pH 7.4.

**Figure 2** Contrast-enhanced liver MRI in healthy mice before and after contrast agent application (~10 min) compared to reference compound 1 (RC1, dose: 0.025 mmol/kg bw, Gd-EOB-DTPA) and reference compound 2 (RC2, dose: 0.050 mmol/kg bw Gd-BOPTA). The studies were performed at a 4.7T preclinical MRI scanner equipped with a dedicated transmitreceive mouse body volume coil using a T1-weighted FLASH (Fast Low Angle Shot) sequence with retrospective respiratory gating.

**Figure 3** CT images of test samples in a water phantom presented in coronal reformation. The test samples in capital letters represents: A = example 4, B = example 5, C = example 6, D= example 2, E = example 12.

**EXPERIMENTAL SECTION**

Chemical names were generated using the ACD/Name software from ACD/Labs. In some cases generally accepted names of commercially available reagents were used in place of ACD/Name generated names.
The following table 1 lists the abbreviations used in this paragraph and in the Examples section as far as they are not explained within the text body. Other abbreviations have their meanings customary *per se* to the skilled person.

**Table 1:** Abbreviations

| Abbreviation | Meaning |
|---|---|
| AcOH | acetic acid |
| ACN | acetonitrile |
| aq. | aqueous |
| AUC | Area under the curve |
| br | broad signal (NMR) |
| bw | Body weight |
| $C_{Gd}$ | Gadolinium concentration |
| CPMG | Carr-Purcell-Meiboom-Gill (MRI sequence) |
| d | doublet (NMR) |

(continued)

| Abbreviation | Meaning |
|---|---|
| DAD | Diode Array Detector |
| DAST | Diethylaminosulfur trifluoride |
| DBU | 1,8-diazabicyclo(5.4.0)undec-7-ene |
| DCM | Dichloromethane |
| dd | doublet of doublet (NMR) |
| DIPEA | diisopropylethylamine |
| DMA | *N,N*-dimethylacetamide |
| DMAP | 4-dimethylaminopyridine |
| DMF | *N,N*-dimethylformamide |
| DMSO | Dimethylsulfoxide |
| EDC.HCl | N-(3-Dimethylaminopropyl)-N'-ethylcarbodiimide hydrochloride salt |
| ESI | electrospray (ES) ionization |
| EtOAc | ethyl acetate |
| EtOH | ethanol |
| FLASH | Fast Low Angle Shot |
| Gd | Gadolinium |
| h | hour(s) |
| HATU | Hexafluorophosphate azabenzotriazole tetramethyl uronium |
| HCl | hydrogen chloride, hydrochloric acid |
| HPLC | high performance liquid chromatography |
| ICP-MS | inductively coupled plasma mass spectrometry |
| IR | Inversion Recovery |
| LC-MS | liquid chromatography-mass spectrometry |
| m | multiplet (NMR) |
| mCPBA | meta-Chloroperoxybenzoic acid |
| MeCN | Acetonitrile |
| MeOH | Methanol |
| min | minute(s) |
| MS | mass spectrometry |
| MTBE | Methyl-*tert*-butylether |
| MRI | Magnetic Resonance Imaging |
| MWD | multiple wavelength detector |
| NaCL | sodium chloride |
| NMR | Nuclear Magnetic Resonance spectroscopy : chemical shifts ($\delta$) are given in ppm. The chemical shifts were corrected by setting the DMSO signal to 2.50 ppm using unless otherwise stated. |
| q | quartet (NMR) |
| $r_i$ | relaxivities (where i=1, 2) |

(continued)

| Abbreviation | Meaning |
|---|---|
| $r_1$ or r1 | reflect how T1 relaxation rates change as a function of contrast agent concentration (slope T1 versus Gd concentration) |
| $r_2$ or r2 | reflect how T2 relaxation rates change as a function of contrast agent concentration (slope T2 versus Gd concentration) |
| $R_1$ | longitudinal relaxation rate ($1/r_1$) |
| $R_2$ | transversal relaxation rate (1/ $r_2$) |
| Rt or RT | room temperature |
| $R_t$, Rt | retention time |
| s | singulet (NMR) |
| sat. | saturated |
| SFC | supercritical fluid chromatography |
| $T_1$ or T1 | longitudinal relaxation time (T1 relaxation time) |
| $T_2$ or T2 | transversal relaxation time (T2 relaxation time) |
| t | triplet (NMR) |
| td | triplet of doublet (NMR) |
| TEA | triethylamine |
| TFA | trifluoroacetic acid |
| THF | tetrahyd rofu ran |
| δ | chemical shift |

Other abbreviations have their meanings customary *per se* to the skilled person.

The various aspects of the invention described in this application are illustrated by the following examples which are not meant to limit the invention in any way.

The example testing experiments described herein serve to illustrate the present invention and the invention is not limited to the examples given.

**EXPERIMENTAL SECTION - GENERAL PART**

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be removed by trituration using a suitable solvent. In some cases, the compounds may be purified by chromatography, particularly flash column chromatography, using for example prepacked silica gel cartridges, e.g. Biotage SNAP cartridges KP-Sil® or KP-NH® in combination with a Biotage autopurifier system (SP4® or Isolera Four®) and eluents such as gradients of hexane/ethyl acetate or DCM/methanol. In flash column chromatography, unmodified ("regular") silica gel may be used as well as aminophase functionalized silica gel. If reference is made to flash column chromatography or to flash chromatography in the experimental section without specification of a stationary phase, regular silica gel was used.

In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the

present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

**Analytical LC-MS Methods:**

Method 1:

Instrument: Waters Acquity UPLCMS SingleQuad; Column: Acquity UPLC BEH C18 1.7 μm, 50×2.1mm; eluent A: water + 0.1 vol. % formic acid (99 %), eluent B: acetonitrile; gradient: 0-1.6 min. 1-99 % B, 1.6-2.0 min. 99 % B; flow 0.8 ml/min; temperature: 60 °C; DAD scan: 210-400 nm.

Method 2:

Instrument: Waters Acquity UPLCMS SingleQuad; Column: Acquity UPLC BEH C18 1.7 μm, 50×2.1mm; eluent A: water + 0.2 vol. % aqueous ammonia (32 %), eluent B: acetonitrile; gradient: 0-1.6 min. 1-99 % B, 1.6-2.0 min. 99 % B; flow 0.8 ml/min; temperature: 60 °C; DAD scan: 210-400 nm.

Method 3:

Instrument: SHIMADZU LCMS-2020 SingleQuad; Column: Chromolith@Flash RP-18E 25x2 mm; eluent A: water + 0.0375 vol % trifluoroacetic acid, eluent B: acetonitrile + 0.01875 vol% trifluoroacetic acid; gradient: 0-0.8 min, 5-95% B, 0.8-1.2 min 95% B; flow 1.5 ml/min; temperature: 50 °C; PDA: 220 nm & 254 nm.

Method 4:

Instrument: SHIMADZU LCMS-2020 SingleQuad; Column: Chromolith@Flash RP-18E, 25x2 mm; eluent A: water + 0.0375 vol % trifluoroacetic acid, eluent B: acetonitrile + 0.01875 vol% trifluoroacetic acid; gradient: 0-0.8 min 0-60% B, 0.8-1.2 min 60% B; flow 1.5 ml/min; temperature: 50 °C; PDA: 220 nm & 254 nm.

**Preparative HPLC Methods:**

Method 5:

Instrument: Labomatic HD-5000, pump head HDK-280, gradient module NDB-1000, fraction collector Labomatic Labocol Vario 2000, Knauer UV detector Azura UVD 2.1S, Prepcon 5 software. Column: Chromatorex C18 10μM 125x30 mm; Eluent A: water + 0.1% formic acid; Eluent B: acetonitrile; gradient: given for intermediates and examples, rate 150 mL/min, temperature 25°C.; UV 220 nm

Method 6:

Instrument: Labomatic HD-5000, pump head HDK-280, gradient module NDB-1000, fraction collector Labomatic Labocol Vario 2000, Knauer UV detector Azura UVD 2.1S, Prepcon 5 software. Column: Chromatorex C18 10μM 125x30 mm; Eluent A: water; Eluent B: acetonitrile; gradient: given for intermediates and examples, rate 150 mL/min, temperature 25°C.; UV 220 nm.

**NMR Spectra:**

The multiplicities of proton signals in $^1$H NMR spectra given in the following paragraphs reflect the observed signal form and do not take into account any higher-order signal phenomena. As a rule, the chemical shift data refers to the center of the signal in question. In the case of wide multiplets, a range is specified. Signals hidden by solvent or water were either assigned tentatively or are not listed. Strongly broadened signals - *e.g.* caused by rapid rotation of molecular moieties or by interchanging protons - have also been assigned tentatively (often referred to as a broad multiplet or broad singlet) or are not shown.

The $^1$H-NMR data of selected compounds are listed in the form of $^1$H-NMR peaklists. Therein, for each signal

peak the δ value in ppm is given, followed by the signal intensity, reported in round brackets. The δ value-signal intensity pairs from different peaks are separated by commas. Therefore, a peaklist is described by the general form: $\delta_1$ (intensity$_1$), $\delta_2$ (intensity$_2$), ... , $\delta_i$ (intensity$_i$), ... , $\delta_n$ (intensity$_n$).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A $^1$H-NMR peaklist is similar to a classical $^1$H-NMR readout, and thus usually contains all the peaks listed in a classical NMR interpretation. Moreover, similar to classical $^1$H-NMR printouts, peaklists can show solvent signals, signals derived from stereoisomers of the particular target compound, peaks of impurities, $^{13}$C satellite peaks, and/or spinning sidebands. The peaks of stereoisomers, and/or peaks of impurities are typically displayed with a lower intensity compared to the peaks of the target compound (e.g., with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical $^1$H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

**Synthsis of Intermediates**

**Intermediate 1-1A**

**ethyl *N*-(*tert*-butoxycarbonyl)-O-{2-[2-(2-methoxyethoxy)ethoxy]ethyl}-L-tyrosinate**

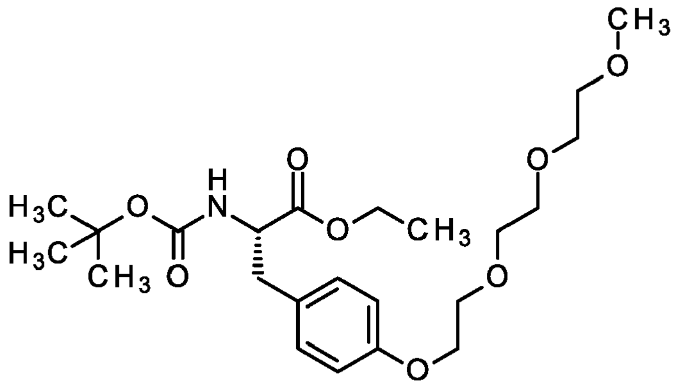

To ethyl *N*-(*tert*-butoxycarbonyl)-L-tyrosinate (5.00 g, 16.2 mmol, CAS:72594-77-5.) in THF (60 ml) was added 2-[2-(2-methoxyethoxy)ethoxy]ethan-1-ol (3.98 g, 24.2 mmol; CAS:112-35-6) followed by triphenylphosphine (8.05 g, 30.7 mmol) and di-*tert*-butyl (E)-diazene-1,2-dicarboxylate (7.07 g, 30.7 mmol) at 0°C. The mixture was stirred for 17 h while it warmed to RT. One half of the solvent was removed under reduced pressure and hexane (60 ml) was added and the mixture was stirred for 1 h while a precipitate formed. The precipitate was removed by filtration. The filtrate was concentrated under reduced pressure and purified by column chromatography (silica, hexane / EtOAc 0-50%) to yield 7.08 g (75% purity, 72% yield) of the title compound.

LC-MS (method 2): $R_t$ = 1.26 min; MS (ESIpos): m/z = 356 [M+H]$^+$.

$^1$H-NMR (400MHz, $CDCl_3$): δ [ppm]= 1.23 (t, 3H), 1.42 (s, 9H), 3.02 (br t, 2H), 3.38 (s, 3H), 3.53 - 3.59 (m, 2H), 3.64 - 3.70 (m, 6H), 3.71 - 3.75 (m, 2H), 3.79 - 3.89 (m, 2H), 4.07 - 4.12 (t, 2H), 4.12 - 4.20 (q, 2H), 4.44 - 4.55 (m, 1H), 4.95 (br d, 1H), 6.83 (d, 2H), 7.03 (d, 2H).

**Intermediate 1-1B**

**methyl-*N*-(*tert*-butoxycarbonyl)-O-{2-[2-(2-methoxyethoxy)ethoxy]ethyl}-L-tyrosinate**

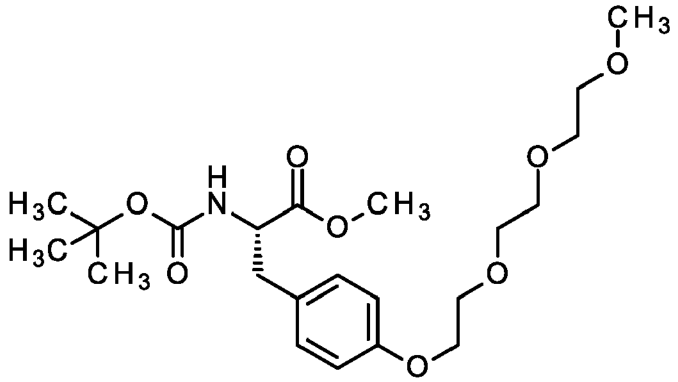

To a solution of methyl-*N*-(*tert*-butoxycarbonyl)-L-tyrosinate (7.13 g, 24.2 mmol) and 2-[2-(2-Methoxyethoxy)ethoxy]ethyl-4-methylbenzensulfonat (16.2 g, 50.7 mmol) in DMF (71 ml) was added potassium carbonate (6,68 g, 48,3 mmol) and 401mg potassium iodid anhydrous (401 mg, 2.42 mmol) and stirred at 80°C for 16h under nitrogen. The mixture was diluted with water and EtOAc and the layers were separated. The water phase was extracted with EtOAc. The combined organic layers were washed with brine, dried over repellent filter and concentrated in vacuum to to yield 16.3 g (78 % purity, quantitative) of the title compound.

LC-MS (method 2): $R_t$ = 1.15 min; MS (ESIpos): m/z = 342 [M+H]-BOC$^+$.

**Intermediate 1-2A**

***tert*-butyl-[(2*S*)-1-hydroxy-3-(4-{2-[2-(2-methoxyethoxy)ethoxy]ethoxy}phenyl)propan-2-yl]carbamate**

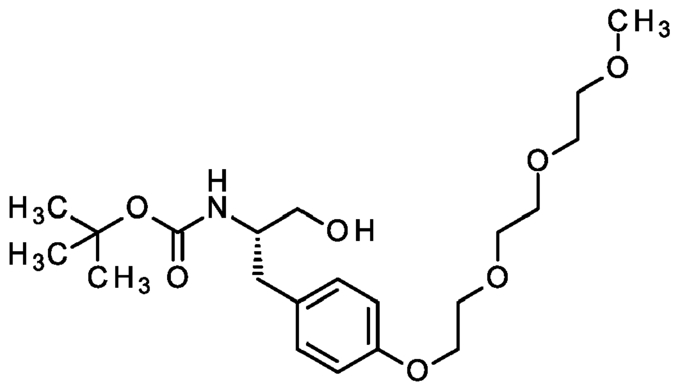

To ethyl-*N*-(*tert*-butoxycarbonyl)-O-{2-[2-(2-methoxyethoxy)ethoxy]ethyl}-L-tyrosinate (Intermediate 1-1A, 7.94 g, 17.4 mmol) dissolved in THF (190 ml) was added lithium aluminum hydride in THF (35 ml, 1.0 M, 35 mmol; CAS:16853-85-3) dropwise at 0°C. The mixture was stirred for 1 h while it warmed to RT. The reaction mixture was diluted with 2N HCL and EtOAc was added, phases were separated and the aqueous phase was extracted with EA. Brine was added to the aqueous phase and was extracted with DCM/EtOH (9:1) two times. The combined organic extracts were dried and concentrated under reduced pressure to yield 3,95g (89% purity, 49% yield) of the title compound.

LC-MS (method 2): $R_t$ = 1.03 min; MS (ESIpos): m/z = 314 [M+H] - BOC$^+$

$^1$H-NMR (400MHz, $CDCl_3$): $\delta$ [ppm]= 1.41 (s, 8H), 2.76 (d, 2H), 3.38 (s, 3H), 3.50 - 3.59 (m, 3H), 3.62 - 3.71 (m, 5H), 3.71 - 3.75 (m, 2H), 3.80 (s, 1H), 3.83 - 3.88 (m, 2H), 4.07 - 4.15 (m, 2H), 6.83 - 6.88 (d, 2H), 7.10 (d, 2H).

**Intermediate 1-2B**

**methy)-O-{2-[2-(2-methoxyethoxy)ethoxy]ethy)}-L-tyrosinate**

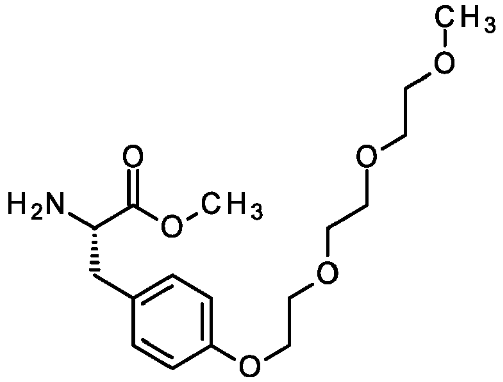

To a solution of methyl-*N*-(*tert*-butoxycarbonyl)-O-{2-[2-(2-methoxyethoxy)ethoxy]ethyl}-L-tyrosinate (Intermediate 1-1B, 22.8 g, 51.7 mmol) in DCM (170 ml) was added HCL (4N in dioxane) (65 ml, 4.0 M, 260 mmol) the mixture was stirred for 1,5 h at room temperature. The mixture was concentrated in vacuum. The residue was dissolved in DCM/EtOH (9+1) and washed with sodium hydrogen carbonate. The water phase was extracted once with DCM/EtOH (20%). The combined organic layer was washed with brine, dried by filtration through a water repellent filter and concentrated in vacuum to yield 14.1 g (79% purity, 63% yield) of the title compound.

LC-MS (method 2): $R_t$ = 0.84 min; MS (ESIpos): m/z = 342 $[M+H]^+$ .

$^1$H NMR (400 MHz, $CDCl_3$) $\delta$ [ppm]= 2.81 (dd, 1H), 3.01 (dd, Hz, 1H), 3.38 (s, 3H), 3.52 - 3.57 (m, 2H), 3.63 - 3.75 (m, 12H), 3.85 (dd, 2H), 4.07 - 4.14 (m, 2H), 6.83 - 6.87 (m, 2H), 7.04 - 7.12 (m, 2 H).

**Intermediate 1-3A**

**(2*S*)-2-amino-3-(4-{2-[2-(2-methoxyethoxy)ethoxy]ethoxy}phenyl)propan-1-ol**

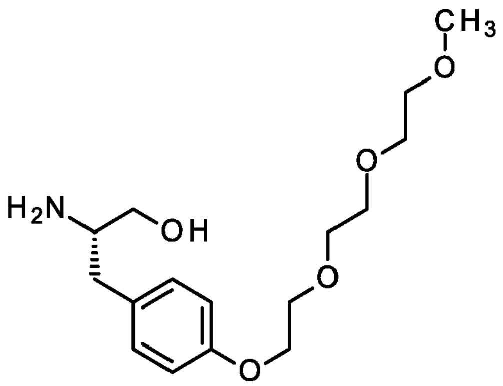

To *tert*-butyl-[(2*S*)-1-hydroxy-3-(4-{2-[2-(2-methoxyethoxy)ethoxy]ethoxy}phenyl)propan-2-yl]carbamate (Intermediate 1-2A, 3.95 g, 9.55 mmol) dissolved in DCM (32 ml) was added HCl in dioxane (12 ml, 4.0 M, 48 mmol; CAS:7647-01-0) and the mixture was stirred for 1 h at RT. The solvent was removed under reduced pressure. The residue was dissolved in DCM/EtOH (10%) and extracted with saturated $NaHCO_3$ solution. The aqueous phase and was extracted once with DCM/EtOH (10%) and twice with DCM/EtOH (20%). The aqueous phase was concentrated under reduced pressure, stirred with EtOH and solids were removed by filtration. The filtrate and the combined organic extracts were dried and concentrated under reduced pressure to yield 2,76 g (93% purity, 86% yield) of the title compound.

LC-MS (method 2): $R_t$ = 0.75 min; MS (ESIpos): m/z = 314 $[M+H]^+$.

$^{1}$H-NMR (400MHz, $CDCl_3$): δ [ppm]= 2.62 (br dd, 1H), 2.69 - 2.87 (m, 1H), 3.12 - 3.26 (m, 1H), 3.36 - 3.38 (m, 3H), 3.40 - 3.48 (dd, 1H), 3.53 - 3.59 (m, 2H), 3.59 - 3.71 (m, 6H), 3.71 - 3.75 (m, 2H), 3.82 - 3.89 (m, 2H), 4.04 - 4.15 (m, 2H), 6.82 - 6.89 (m, 2H), 7.05 - 7.15 (m, 2H).

## Intermediate 1-3B

**methy)-O-{2-[2-(2-methoxyethoxy)ethoxy]ethy)}-N-(2-methoxy-2-oxoethy))-L-tyrosinate**

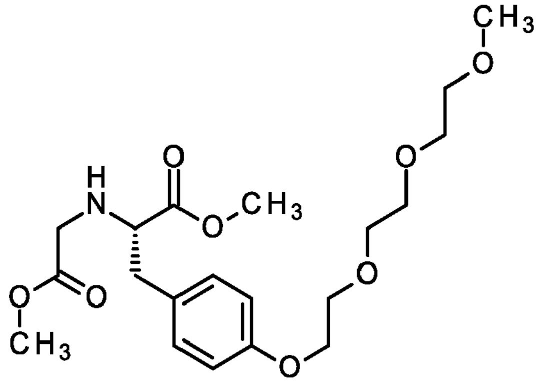

To a solution of methyl-O-{2-[2-(2-methoxyethoxy)ethoxy]ethyl}-L-tyrosinate (Intermediate 1-2B, 14.1 g, 41.4 mmol) acetonitrile (65 ml) was added potassium carbonate (8.58 g, 62.1 mmol) and methyl chloroacetate (3.6 ml, 41 mmol) and the mixture was stirred for 16 h at 50°C.The precipitate was filtered off and the filtrate was concentrated under reduced pressure. The residue was purified by column chromatography (silica, hexane / EtOAc 80-100% EtOAc) to yield 3,71 g (84% purity, 18% yield) of the title compound.

LC-MS (method 2): $R_t$ = 0.97 min; MS (ESIpos): m/z = 414 $[M+H]^+$.

$^{1}$H NMR (400 MHz, $CDCl_3$) δ [ppm]= 2.87 - 2.99 (m, 2 H) 3.31 - 3.45 (m, 5H), 3.52 - 3.59 (m, 3H), 3.64 - 3.75 (m, 12H), 3.81 - 3.87 (m, 2H), 4.07 - 4.12 (m, 2H) 6.82 - 6.86 (m, 2H) 7.06 - 7.12 (m, 2H).

Optical rotation:$[\alpha]_D$ = 7.7° +/- 0.8° (c = 4.7 mg/ml, 20°C, 589 nm, $CHCl_3$).

## Intermediate 1-4A

**(2*S*)-2-[(*E*)-(4-methoxybenzyliden)amino]-3-(4-(2-[2-(2-methoxyethoxy)ethoxy]ethoxy}phenyl)propan-1-ol**

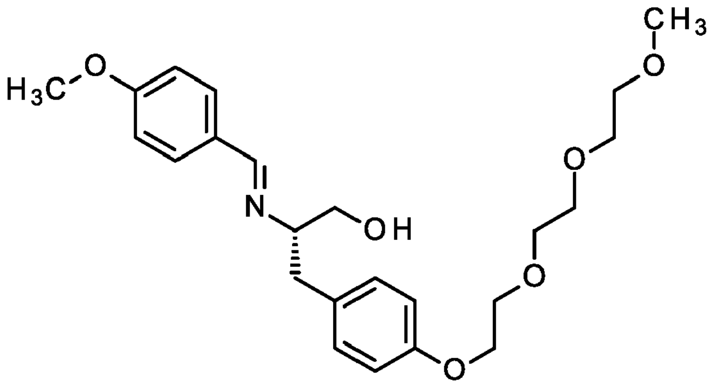

To (2*S*)-2-amino-3-(4-{2-[2-(2-methoxyethoxy)ethoxy]ethoxy}phenyl)propan-1-ol (Intermediate 1-3A, 2.28 g, 7.28 mmol) dissolved in toluene (30 ml) was added molecular sieve (5.1 g) and 4-methoxybenzaldehyd (890 μl, 7.3 mmol; CAS:123-11-5) and the mixture was stirred for 2 h at 90°C. Molecular sieve was removed by filtration, the filtrate was concentrated under reduced pressure to yield 3,35g (86% purity, 92% yield) of the title compound.

LC-MS (method 2): $R_t$ = 1.06 min; MS (ESIpos): m/z = 432 $[M+H]^+$

$^1$H-NMR (400MHz, $CDCl_3$): δ [ppm]= 2.35 (s, 1H), 2.65 - 2.66 (m, 1H), 2.75 - 2.94 (m, 2H), 3.36 - 3.39 (m, 3H), 3.52 - 3.57 (m, 4H), 3.63 - 3.70 (m, 4H), 3.70 - 3.74 (m, 2H), 3.79 - 3.83 (m, 2H), 3.84 - 3.85 (m, 3H), 4.05 - 4.09 (m, 2H), 6.77 - 6.80 (m, 2H), 6.98 - 7.06 (m, 4H), 7.63 (d, 2H), 7.91 - 7.94 (m, 1H).

## Intermediate 1-5A

**(2*S*)-2-[(4-methoxybenzyl)amino]-3-(4-(2-[2-(2-methoxyethoxy)ethoxy]ethoxy}phenyl)propan-1-ol**

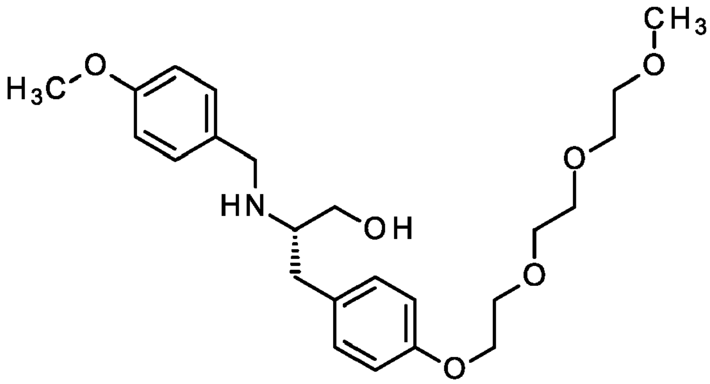

To (2*S*)-2-[(*E*)-(4-methoxybenzyliden)amino]-3-(4-{2-[2-(2-methoxyethoxy)ethoxy]ethoxy} phenyl)propan-1-ol (Intermediate 1-4A, 3.35 g, 7.76 mmol) dissolved in MeOH (23ml) was added sodium borohydride (441 mg, 11.6 mmol; CAS:16940-66-2) at 0°C and the mixture was stirred for 1h. Acetone (2,3 ml) was added and the mixture was warmed to RT. The mixture was filtered over Celite, washed with MeOH and concentrated under reduced pressure. The residue was dissolved in DCM/EtOH (9+1) and extracted with water. The aqueous phase and was extracted with DCM/EtOH (9+1). The combined organic extract was washed with brine, dried and concentrated under reduced pressure to yield 2,68 g (93% purity, 74% yield) of the title compound.

LC-MS (method 2): $R_t$ = 1.06 min; MS (ESIpos): m/z = 434 $[M+H]^+$

$^1$H NMR (400MHz, $CDCl_3$): δ [ppm]= 2.72 - 2.90 (m, 2H), 2.93 - 3.03 (m, 1H), 3.35 - 3.38 (s, 3H), 3.39 - 3.45 (m, 1H), 3.54 - 3.58 (m, 2H), 3.64 - 3.70 (m, 6H), 3.72 - 3.75 (m, 3H), 3.74 - 3.76 (m, 1H), 3.77 - 3.79 (m, 4H), 3.84 - 3.88 (m, 2H), 4.08 - 4.13 (m, 2H), 6.82 - 6.86 (m, 4H), 7.03 - 7.08 (m, 2H), 7.18 - 7.23 (m, 2H).

## Intermediate 1-6A

**(2*S*)-1-(4-methoxybenzyl)-2-(4-{2-[2-(2-methoxyethoxy)ethoxy]ethoxy}benzyl)aziridine**

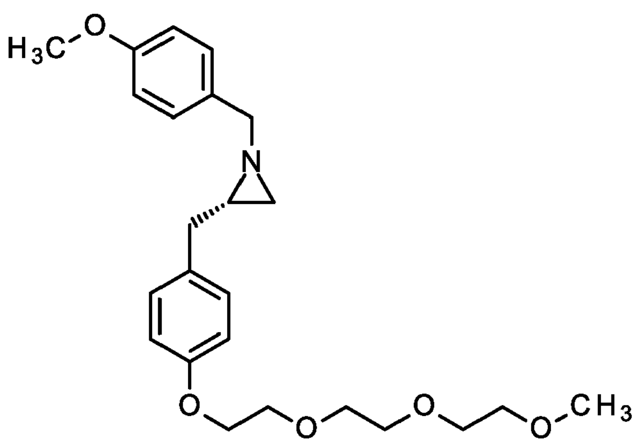

To (2S)-2-[(4-Methoxybenzyl)amino]-3-(4-{2-[2-(2-methoxyethoxy)ethoxy]ethoxy}phenyl) propan-1-ol (Intermediate 1-5A, 500 mg, 1.15 mmol) dissolved in THF (4,5 ml) was added triphenylphosphine (423 mg, 1.61 mmol; CAS:603-35-0) and di-*tert*-butyl-(*E*)-diazen-1,2-dicarboxylate (372 mg, 1.61 mmol; CAS:870-50-8) at 0°C and the mixture was stirred for 16 h while it warmed to RT. One half of the solvent was removed under reduced pressure, hexane (10 ml) was added and the mixture was stirred for 1,5 h while a precipitate formed. The precipitate was removed by filtration. The filtrate was concentrated under reduced pressure and purified by column chromatography (silica gel, hexane / EtOAc, 40-100%) to yield 182 mg (85% purity, 32% yield) of the title compound.

LC-MS (method 2): $R_t$ = 1.20 min; MS (ESIpos): m/z = 416 $[M+H]^+$

$^1H$ NMR (400MHz, $CDCl_3$): δ [ppm]= 1.41 - 1.57 (m, 2H), 1.68 - 1.73 (m, 1H), 2.50 - 2.60 (m, 1H), 2.67 - 2.79 (m, 1H), 3.26 - 3.46 (m, 2H), 3.38 (s, 3H), 3.53 - 3.58 (m, 2H), 3.63 - 3.71 (m, 4H), 3.71 - 3.78 (m, 2H), 3.78 - 3.87 (m, 5H), 4.08 - 4.12 (m, 2H), 6.75 - 6.86 (m, 4H), 7.03 - 7.08 (m, 2H), 7.15 - 7.22 (m, 2H).

**Cyclen intermediate 1-1A**

**(2*S*)-1,7,10-tribenzyl-4-(4-methoxybenzyl)-2-(4-{2-[2-(2-methoxyethoxy)ethoxy]ethoxy}benzyl)-1,4,7,10-tetraazacyclododecane**

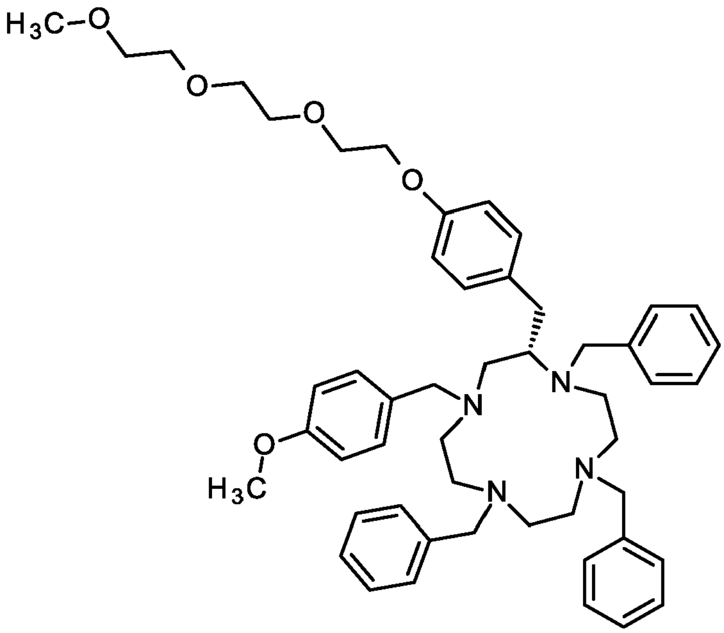

To (2S)-1-(4-methoxybenzyl)-2-(4-{2-[2-(2-methoxyethoxy)ethoxy]ethoxy}benzyl)aziridine (Intermediate 1-6A, 1.00 g, 2.41 mmol) and 1-benzylaziridine (20 ml, 0.49 M, 9.6 mmol) (emulsion in water 0,065 g/ml) dissolved in EtOH (13ml) was slowly added p-toluenesulfonic acid monohydrate (572 mg, 3.0 mmol; CAS: 6192-52-5,) in water (0,3 ml) over 8 h at 60°C followed by additional stirring at 60°C for 7 h and reflux for 2 h. After cooling to RT sodium hydroxide (179 mg, 4.47 mmol) dissolved in water (360 μl) was added and the reaction mixture was extracted twice with DCM. The combined organic extracts were washed with brine, dried, concentrated under reduced pressure and purified by column chromatography (amino phase functionalized silica gel, hexane / EtOAc, 40-100%) to yield 1,02 g (56% purity, 29% yield) of the title compound.

LC-MS (method 1): $R_t$ = 1.26 min; MS (ESIpos): m/z = 815 $[M+H]^+$

**Cyclen intermediate 1-1B**

**(3*S*)-3-(4-{2-[2-(2-methoxyethoxy)ethoxy]ethoxy}benzyl)-1,4,7,10-tetraazacyclododecane-2,6-dione**

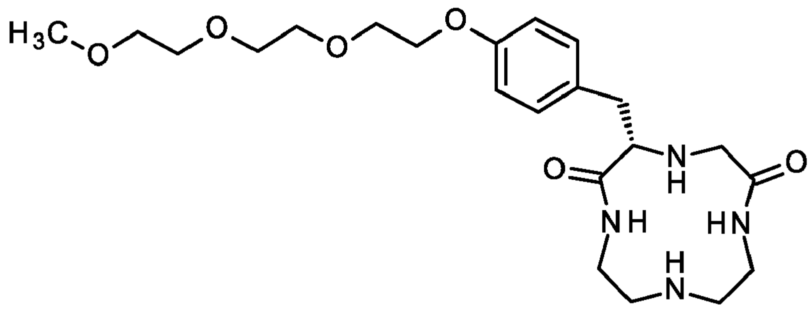

To a solution of Methyl-O-{2-[2-(2-methoxyethoxy)ethoxy]ethyl}-N-(2-methoxy-2-oxoethyl)-L-tyrosinate (Intermediate 1-3B, 10.5 g, 25.3 mmol) in Methanol (250ml) was added N-(2-aminoethyl)ethane-1,2-diamine (2.7 ml, 25 mmol) and the mixture was stirred for 30 days at 40°C. The mixture was concentrated under reduced pressure and purified by column chromatography (amino phase functionalized silica gel, 380 g, DCM / EtOH, 0-10%) to yield 4.32 g (41% purity, 15% yield) of the title compound.

LC-MS (method 1): $R_t$ = 0.55 min; MS (ESIpos): m/z = 453 $[M+H]^+$

Cyclen intermediate 1-2

**(2*S*)-2-(4-{2-[2-(2-methoxyethoxy)ethoxy]ethoxy}benzyl)-1,4,7,10-tetraazacyclododecanee**

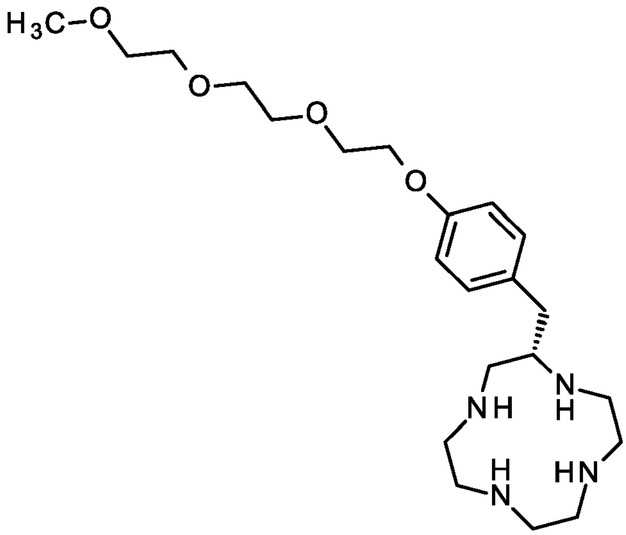

To (2S)-1,7,10-Tribenzyl-4-(4-methoxybenzyl)-2-(4-{2-[2-(2-methoxyethoxy)ethoxy]ethoxy} benzyl)-1,4,7,10-tetraazacyclododecane (Cyclen intermediate 1-1A,1.02 g, 1.25 mmol) dissolved in EtOH (13ml) and acetic acid (13ml) was added palladium on activated carbon (200 mg, 10%, 188 μmol; CAS-RN:7440-05-3) and stirred under hydrogen pressure (40 bar) at 80°C for 42 h. The catalyst was removed by filtration and washed with EtOH and THF. The filtrate was concentrated under reduced pressure to yield 1,87 g (30% purity, 77% yield) of the title compound.

LC-MS (method 1): $R_t$ = 0,57 min; MS (ESIpos): m/z = 425 $[M+H]^+$

Cyclen intermediate 1-2 alternative procedure

**(2*S*)-2-(4-{2-[2-(2-methoxyethoxy)ethoxy]ethoxy}benzyl)-1,4,7,10-tetraazacyclododecane**

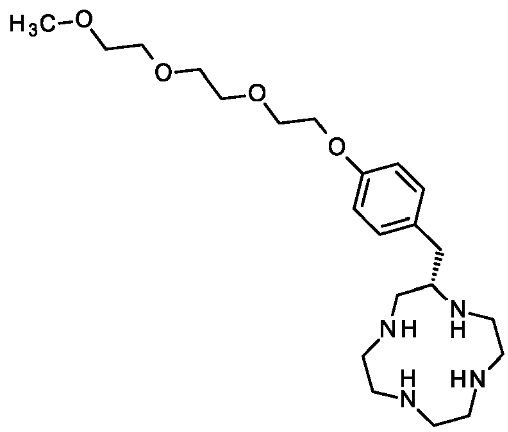

To a suspension of (3*S*)-3-(4-{2-[2-(2-methoxyethoxy)ethoxy]ethoxy}benzyl)-1,4,7,10-tetraaza cyclododecane-2,6-dione (Cyclen intermediate 1-1B, 4.32 g, 9.55 mmol) in THF (158 ml) was added DIBAL in toluene (190 ml, 1.0 M, 190 mmol) dropwise and the resulting mixture was stirred 1h at RT. The mixture was cooled to 0°C, toluene / THF (1:1, 320 ml), water (14 ml) and sodium fluoride (32.1 g, 764 mmol) were added and stirring was continued over night at RT. The solid was filtered off and washed with toluene / THF (1:1) und THF. The filtrate was concentrated under reduced pressure to yield 1,82 g (70% purity, 31% yield) of the title compound.

LC-MS (method 1): $R_t$ = 0.50 min; MS (ESIpos): m/z = 425 $[M+H]^+$

## Cyclen intermediate 1-3

**tetra-*tert*-butyl-2,2',2",2'''-[(2*S*)-2-(4-{2-[2-(2-methoxyethoxy)ethoxy]ethoxy}benzyl)-1,4,7,10-tetraazacyclododecane-1,4,7,10-tetrayl]tetraacetate**

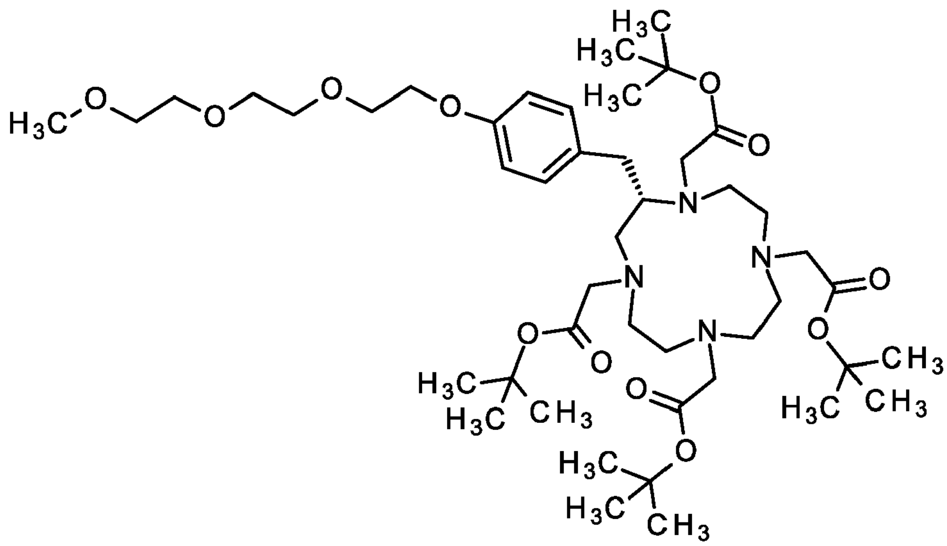

To (2*S*)-2-(4-{2-[2-(2-methoxyethoxy)ethoxy]ethoxy}benzyl)-1,4,7,10-tetraazacyclododecane (1.37 g, 3.23 mmol) dissolved in acetonitrile (42 ml) was added potassium carbonate (4,46 g, 32,2 mmol; CAS-RN:584-08-7) and the mixture was stirred for 1h at 50°C. *Tert*-butyl bromoacetate (3.0 ml, 21 mmol) was added and stirring at RT was continued for 3 days. *Tert*-butyl methyl ether and water were added and phases were separated. The aqueous phase was extracted with *tert*-butyl methyl ether. The combined organic extracts were washed with aqueous sodium hydroxide solution (1M) and brine, dried and concentrated under reduced pressure to yield 2,84 g (43% purity, 43% yield) of the title compound.

LC-MS (method 1): $R_t$ = 1.31 min; MS (ESIpos): m/z = 882 $[M+H]^+$

**Free ligand 1**

**2,2',2",2'''-[(2*S*)-2-(4-{2-[2-(2-methoxyethoxy)ethoxy]ethoxy}benzyl)-1,4,7,10-tetraazacyclododecane-1,4,7,10-tetrayl]tetraacetic acid**

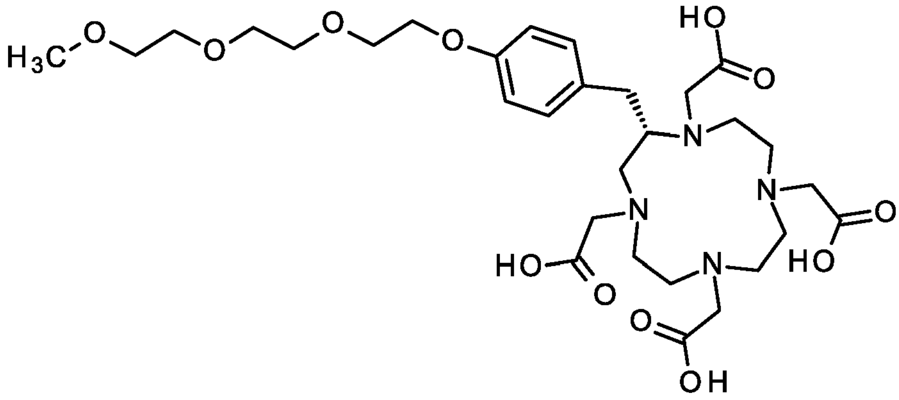

Tetra-tert-butyl-2,2',2",2'''-[(2S)-2-(4-{2-[2-(2-methoxyethoxy)ethoxy]ethoxy}benzyl)-1,4,7,10-tetraazacyclododecane-1,4,7,10-tetrayl]tetraacetate (2.84 g, 3.22 mmol) was dissolved in formic acid (41 ml, 1.1 mol; CAS-RN:64-18-6) and stirred for 18h at 70°C. The reaction mixture was concentrated under reduced pressure and co-distilled twice with toluene / EtOH to yield 2,19 g (60% purity, 62% yield) of the title compound,

LC-MS (method 1): $R_t$ = 0,63 min; MS (ESIpos): m/z = 657 $[M+H]^+$.

**Intermediate 2-1**

**ethyl-*N*-(*tert*-butoxycarbonyl)-O-[2-(2-ethoxyethoxy)ethyl]-L-tyrosinate**

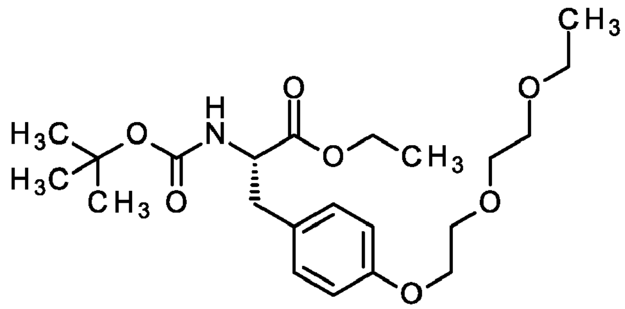

To ethyl *N*-(*tert*-butoxycarbonyl)-L-tyrosinate (1.0 g, 16.2 mmol, CAS:72594-77-5.) in THF (12 ml) was added 2-(2-ethoxyethoxy)ethanol (650 µl, 4.8 mmol), followed by triphenylphosphine (1.61 g, 6.14 mmol) and di-*tert*-butyl (*E*)-diazene-1,2-dicarboxylate (1.41 g, 6.14 mmol) at 0°C. The mixture was stirred for 20 h while it warmed to RT. One half of the solvent was removed under reduced pressure and hexane (14 ml) was added and the mixture was stirred for 1 h while a precipitate formed. The precipitate was removed by filtration. The filtrate was concentrated under reduced pressure and purified by column chromatography (silica gel, hexane / EtOAc, 0-50%) to yield 1,15 g (85% purity, 71% yield) of the title compound.

LC-MS (method 2): $R_t$ = 1,33 min; MS (ESIpos): m/z = 326 $[M-BOC+H]^+$.

$^1$H-NMR (400MHz, $CDCl_3$): δ [ppm]= 1.19 - 1.26 (m, 6H), 1.42 (s, 9H), 2.93 - 3.09 (m, 2H), 3.54 (q, 2H), 3.59 - 3.63 (m, 2H), 3.69 - 3.75 (m, 2H), 3.83 - 3.89 (dd, 2H), 4.09 - 4.12 (m, 2H), 4.15 (q, 2H), 4.44 - 4.55 (m, 1H), 4.87 - 5.03 (m, 1H), 6.81 - 6.86 (m, 2H), 7.03 (d, 2H).

**Intermediate 2-2**

**tert-buty)-[(2*S*)-1-{4-[2-(2-ethoxyethoxy)ethoxy]phenyl}-3-hydroxypropan-2-yl]carbamate**

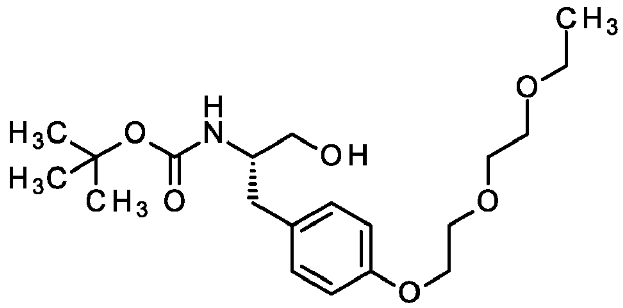

To ethyl-*N*-(*tert*-butoxycarbonyl)-O-[2-(2-ethoxyethoxy)ethyl]-L-tyrosinate (16.5 g, 65% purity, 25.2 mmol) in THF (276 ml) was added dropwise lithium aluminum hydride in THF (50 ml, 1.0 M, 50 mmol; CAS-RN:16853-85-3) at 0°C. The mixture was stirred for 2 h while it warmed to RT. The reaction mixture was diluted with 2N HCL and EtOAc was added, phases were separated and the aqueous phase was extracted with EtOAc. Brine was added to the aqueous phase and extraction with DCM/EtOH (9 :1) was repeated two times. The combined organic extract was dried and concentrated under reduced pressure to yield 11,43 g (72% purity, 85% yield) of the title compound.

LC-MS (method 2): $R_t$ = 1.11 min; MS (ESIpos): m/z = 284 $[M-BOC+H]^+$.

$^1H$ NMR (400 MHz, $CDCl_3$) δ [ppm]= 1.19 - 1.24 (m, 3H), 1.41 (s, 9H), 2.76 (d, 2H), 3.48 - 3.68 (m, 9H), 3.71 - 3.74 (m, 2H), 3.83 - 3.87 (m, 2H), 4.11 (dd, , 2H), 6.79 - 6.88 (m, 2 H), 7.10 (d, 2H).

**Intermediate 2-3**

**(2S)-2-Amino-3-{4-[2-(2-ethoxyethoxy)ethoxy]phenyl}propan-1-ol**

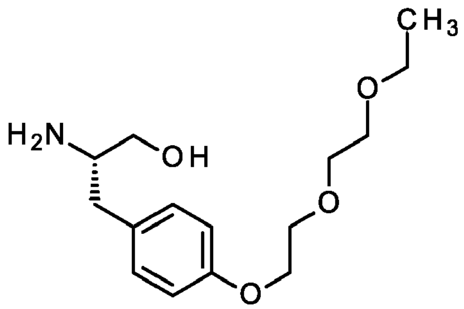

To tert-Butyl-[(2S)-1-{4-[2-(2-ethoxyethoxy)ethoxy]phenyl}-3-hydroxypropan-2-yl]carbamate (3.56 g, 9.28 mmol) dissolved in DCM (31ml) was added HCl in dioxane (12 ml, 4.0 M, 48 mmol; CAS:7647-01-0) and stirred for 1 h at RT. The solvent was removed under reduced pressure. The residue was dissolved in DCM/EtOH (10%) and extracted with saturated $NaHCO_3$ solution and brine. The aqueous phase and was extracted twice with DCM/EtOH (10%). The combined organic extracts were dried and concentrated under reduced pressure to yield 2.15 g (90 % purity, 74 % yield) of the title compound.

LC-MS (method 2): $R_t$ = 0.64 min; MS (ESIpos): m/z = 284 $[M+H]^+$

$^1H$-NMR (400MHz, $CDCl_3$): δ [ppm]= 1.14 - 1.26 (m, 3H), 2.37 (br s, 3H), 2.51 - 2.87 (dd, 2H), 3.10 - 3.20 (m, 1H), 3.33 - 3.51 (m, 2H), 3.51 - 3.74 (m, 6H), 3.82 - 3.88 (t, 2H), 4.07 - 4.15 (t, 2H), 6.81 - 6.89 (d, 2H), 7.05 - 7.14 (d, 2H).

**Intermediate 2-4**

**(2S)-3-{4-[2-(2-Ethoxyethoxy)ethoxy]phenyl}-2-[(E)-(4-methoxybenzyliden)amino]propan-1-ol**

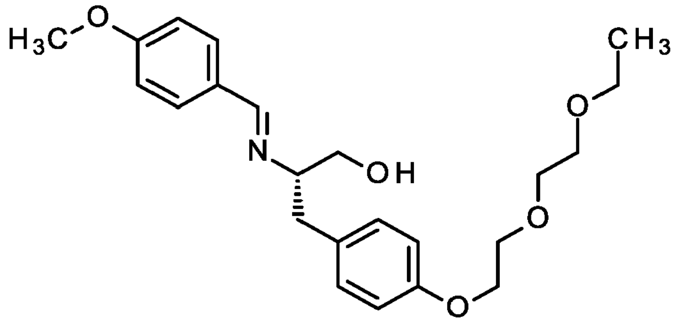

To (2*S*)-2-amino-3-{4-[2-(2-ethoxyethoxy)ethoxy]phenyl}propan-1-ol (2.15 g, 7.60 mmol) dissolved in toluene (29 ml) was added molecular sieve (953 mg) and 4-methoxybenzaldehyd (930 µl, 7.6 mmol; CAS:123-11-5) and the mixture was stirred for 2 h at 90°C. Molecular sieve was removed by filtration, the filtrate was concentrated under reduced pressure to yield 3,5 g (quantitative) of the title compound.

LC-MS (method 1): $R_t$ = 0.85 min; MS (ESIpos): m/z = 402 $[M+H]^+$

$^1$H-NMR (400 MHz, $CDCl_3$) δ [ppm]: 1.168 (1.75), 1.189 (1.17), 1.202 (4.96), 1.208 (1.26), 1.219 (10.60), 1.226 (2.43), 1.236 (5.54), 1.243 (1.27), 1.440 (2.84), 1.968 (0.70), 2.281 (0.41), 2.365 (7.84), 2.753 (0.69), 2.774 (0.68), 2.788 (1.04), 2.809 (1.06), 2.819 (0.78), 2.838 (0.79), 2.875 (0.88), 2.888 (0.89), 2.909 (0.54), 2.923 (0.53), 3.444 (0.50), 3.452 (0.42), 3.513 (1.55), 3.521 (0.75), 3.530 (4.63), 3.539 (1.40), 3.548 (4.86), 3.556 (1.22), 3.566 (1.78), 3.605 (2.00), 3.615 (3.49), 3.620 (2.50), 3.628 (3.84), 3.706 (3.32), 3.713 (2.25), 3.719 (3.59), 3.730 (2.70), 3.742 (1.02), 3.760 (1.32), 3.769 (1.23), 3.779 (1.25), 3.796 (1.11), 3.810 (0.84), 3.818 (0.89), 3.823 (0.79), 3.831 (2.07), 3.838 (2.41), 3.850 (14.58), 3.856 (3.24), 3.861 (2.08), 3.906 (16.00), 4.080 (2.09), 4.084 (1.36), 4.093 (2.64), 4.105 (1.76), 4.125 (0.70), 6.786 (2.41), 6.807 (2.76), 6.834 (1.16), 6.855 (1.53), 6.874 (0.59), 6.883 (0.56), 6.902 (2.76), 6.919 (1.04), 6.924 (2.80), 7.008 (2.89), 7.013 (0.96), 7.026 (1.10), 7.031 (3.37), 7.036 (2.93), 7.058 (2.21), 7.118 (1.31), 7.139 (1.20), 7.167 (1.03), 7.175 (1.36), 7.193 (2.14), 7.247 (1.66), 7.252 (0.56), 7.280 (0.51), 7.284 (0.75), 7.615 (2.69), 7.637 (2.48), 7.838 (0.41), 7.844 (3.41), 7.849 (1.08), 7.861 (1.07), 7.867 (3.27), 7.874 (0.54), 7.940 (2.75), 9.900 (3.92).

**Intermediate 2-5**

**(2*S*)-3-{4-[2-(2-ethoxyethoxy)ethoxy]phenyl}-2-[(4-methoxybenzyl)amino]propan-1-ol**

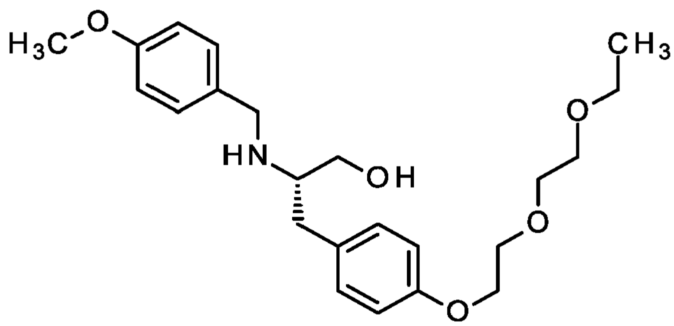

To (2*S*)-3-{4-[2-(2-ethoxyethoxy)ethoxy]phenyl}-2-[(*E*)-(4-methoxybenzyliden)amino]propan-1-ol (3.5 g, 8.72 mmol) dissolved in MeOH (26 ml) was added sodium borohydride (495 mg, 13.1 mmol; CAS:16940-66-2) in 3 portions at 0°C and the mixture was stirred for 1h. Acetone (2,6 ml) was added and the mixture was warmed to RT. The mixture was filtered over Celite, washed with MeOH and the filtrate was concentrated under reduced pressure. The residue was dissolved in EtOAc and extracted with water. The aqueous phase and was extracted with EtOAc twice. The combined

organic extracts were dried and concentrated under reduced pressure to yield 4.59 g (75% purity, 98% yield) of the title compound.

LC-MS method 1): $R_t$ = 0.82 min; MS (ESIpos): m/z = 403 $[M+H]^+$ .

$^1H$ NMR (400 MHz, $CDCl_3$) δ ppm 1.20 - 1.24 (t, 3H), 2.67 - 2.83 (m, 2H), 2.84 - 2.94 (m, 1 H) 3.26 - 3.35 (m, 1H), 3.38 - 3.67 (m, 7H), 3.67 - 3.74 (m, 4 H), 3.77 - 3.80 (m, 3H), 3.84 - 3.89 (m, 2H), 4.09 - 4.15 (m, 2H), 6.80 - 6.86 (m, 4H), 7.05 (d, 2H), 7.12 (d, 2H).

**Intermediate 2-6**

**(2*S*)-2-{4-[2-(2-ethoxyethoxy)ethoxy]benzyl}-1-(4-methoxybenzyl)aziridine**

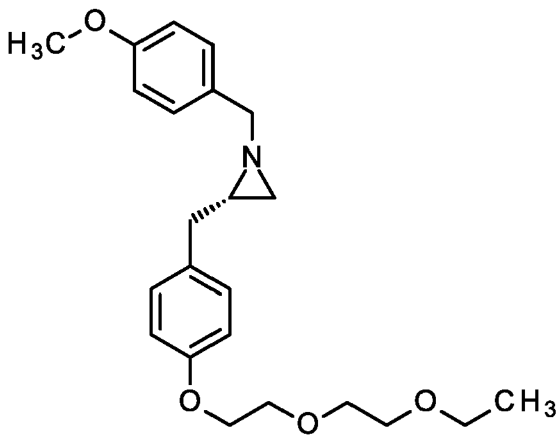

To (2S)-3-{4-[2-(2-ethoxyethoxy)ethoxy]phenyl}-2-[(4-methoxybenzyl)amino]propan-1-ol (1.00 g, 2.48 mmol) dissolved in THF (9.7 ml) was added triphenylphosphine (910 mg, 3.47 mmol; CAS:603-35-0) and di-*tert*-butyl-(*E*)-diazen-1,2-dicarboxylate (799 mg, 3.47 mmol; CAS:870-50-8) at 0°C and stirred for 16 h while it warmed to RT. One half of the solvent was removed under reduced pressure, hexane (30 ml) was added and the mixture was stirred for 1,5 h while a precipitate formed. The precipitate was removed by filtration. The filtrate was concentrated under reduced pressure and purified by column chromatography (silica gel, hexane / EtOAc, 40-100%) to yield 684 mg (40% purity, 29% yield) of the title compound.

LC-MS (method 2): $R_t$ = 1.28 min; MS (ESIpos): m/z = 386 $[M+H]^+$

1H-NMR (400MHz, $CDCl_3$): δ [ppm]= 1.18 - 1.24 (m, 3H), 1.40 - 1.45 (m, 1H), 1.64 - 1.73 (m, 2H), 2.49 - 2.60 (dd, 1H), 2.68 - 2.77 (dd, 1H), 3.27 - 3.43 (q, 2H), 3.49 - 3.59 (m, 3H), 3.60 - 3.65 (m, 2H), 3.69 - 3.74 (m, 2H), 3.70 - 3.70 (m, 1H), 3.77 - 3.82 (m, 1H), 3.80 (s, 3H), 3.83 - 3.90 (m, 2H), 4.08 - 4.13 (m, 2H), 6.77 - 6.83 (m, 4H), 7.04 - 7.10 (m, 2H), 7.16 - 7.24 (m, 2H).

**Cvclen intermediate 2-1**

**(2*S*)-1,7,10-tribenzyl-2-{4-[2-(2-ethoxyethoxy)ethoxy]benzyl}-4-(4-methoxybenzyl)-1,4,7,10-tetraazacyclododecane**

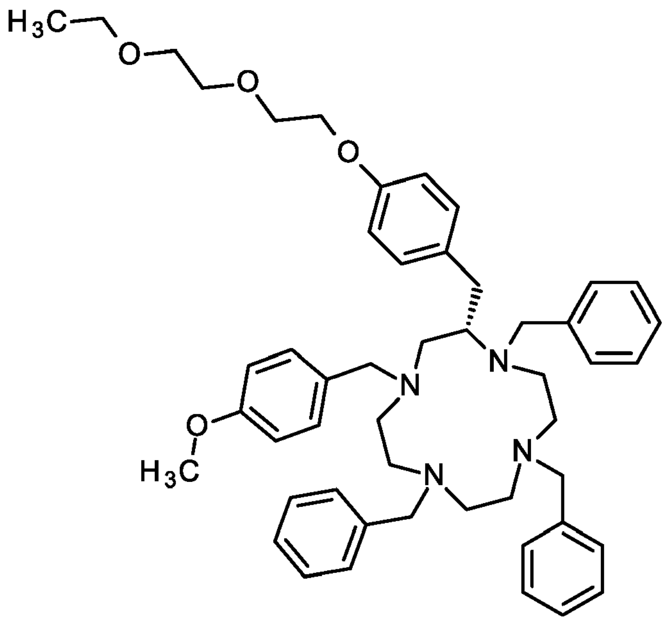

To (2S)-2-{4-[2-(2-ethoxyethoxy)ethoxy]benzyl}-1-(4-methoxybenzyl)aziridine (806 mg, 50 % purity, 1.05 mmol) and 1-benzylaziridine (8.6 ml, 0.49 M, 4.2 mmol) (emulsion in water 0,065 g/ml) dissolved in EtOH (5.6 ml) was added p-toluenesulfonic acid monohydrate (249 mg, 1.31 mmol; CAS-RN:6192-52-5) in water (120 μl) over 8 h slowly at 60°C followed by additional stirring at 60°C for 7h and reflux for 2h. After cooling to RT sodium hydroxide (77.7 mg, 1.94 mmol) dissolved in water (16 0μl) was added and the reaction mixture was extracted twice with DCM. The combined organic extracts were washed with brine, dried, concentrated under reduced pressure and purified by column chromatography (amino phase functionalized silica gel, hexane / EtOAc, 40-100%) to yield 616 mg (30% purity, 23% yield) of the title compound.

LC-MS (method 1): $R_t$ = 1.37 min; MS (ESIpos): m/z = 785 $[M+H]^+$

<u>**Cyclen intermediate 2-2**</u>

**(2*S*)-2-{4-[2-(2-Ethoxyethoxy)ethoxy]benzyl}-1,4,7,10-tetraazacyclododecane**

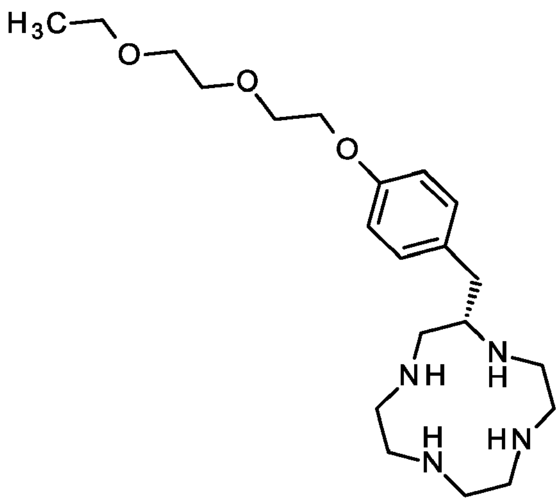

To (2S)-1,7,10-tribenzyl-2-{4-[2-(2-ethoxyethoxy)ethoxy]benzyl}-4-(4-methoxybenzyl)-1,4,7, 10-tetraazacyclododecane (620 mg, 790 μmol) dissolved in EtOH (15 ml) and acetic acid (15 ml) was added palladium on activated carbon (150 mg, 10%, 142 μmol; CAS: 7440-05-3) and stirred under hydrogen pressure (40 bar) at 80°C for 84 h. The catalyst was removed by filtration and washed with EtOH and THF. The filtrate was concentrated under reduced pressure

to yield 530 mg (26% purity, 44% yield) of the title compound.

LC-MS (method 1): ): $R_t$ = 0.59 min; MS (ESIpos): m/z = 395 $[M+H]^+$

**Cyclen intermediate 2-3**

**tetra-tert-buty)-2,2',2",2'''-[(2*S*)-2-{4-[2-(2-ethoxyethoxy)ethoxy]benzy)}-1,4,7,10-tetraazacyclododecane-1,4,7,10-tetrayl]tetraacetate**

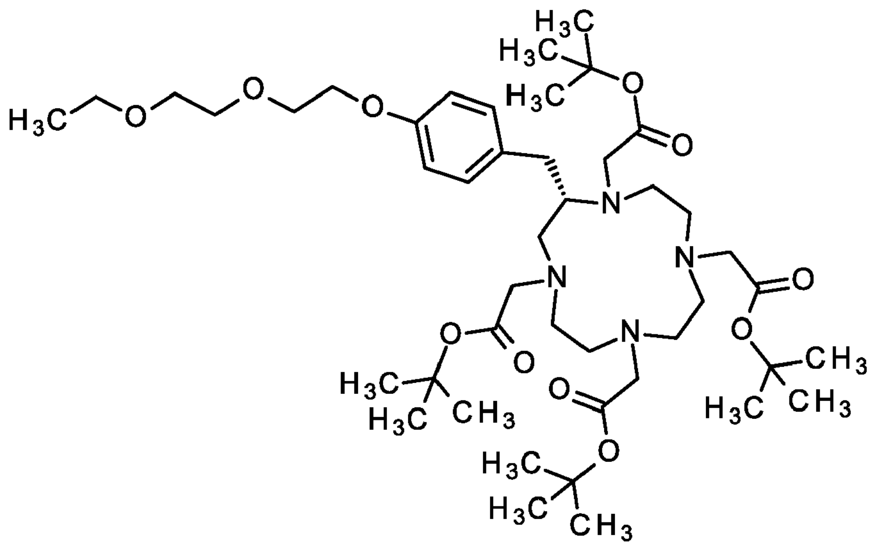

To (2S)-2-{4-[2-(2-ethoxyethoxy)ethoxy]benzyl}-1,4,7,10-tetraazacyclododecane (530 mg, 1.34 mmol) dissolved in acetonitrile (18 ml) was added potassium carbonate (1.86 g, 13.4 mmol; CAS: 584-08-7) and stirred for 1 h at 50°C. *Tert*-butyl bromoacetate (870 µl, 5.9 mmol) was added and stirred at RT for 16 h. *Tert*-butyl methyl ether and water were added and phases were separated. The aqueous phase was extracted with *tert*-butyl methyl ether. The combined organic extracts were washed with aqueous sodium hydroxide solution (1M) and brine, dried and concentrated under reduced pressure to yield 0,96 g of an intermediate. To the intermediate dissolved in acetonitrile (17 ml) was added potassium carbonate (360 mg, 2.61 mmol; CAS: 584-08-7) and *tert*-butyl bromoacetate (230 µl, 1.6 mmol) and the mixture was stirred at RT for 5 days. *Tert*-butyl methyl ether and water were added and phases were separated. The aqueous phase was extracted with *tert*-butyl methyl ether. The combined organic extract was washed with aqueous sodium hydroxide solution (1M) and brine, dried and concentrated under reduced pressure to yield 0,95 g of (31% purity, 27% yield) of the title compound.

LC-MS (method 1): $R_t$ = 1.33 min; MS (ESIpos): m/z = 851 $[M+H]^+$

**Free ligand 2**

**2,2',2",2'''-[(2*S*)-2-(4-[2-(2-ethoxyethoxy)ethoxy]benzyl)-1,4,7,10-tetraazacyclododecane-1,4,7,10-tetrayl]tetraacetic acid**

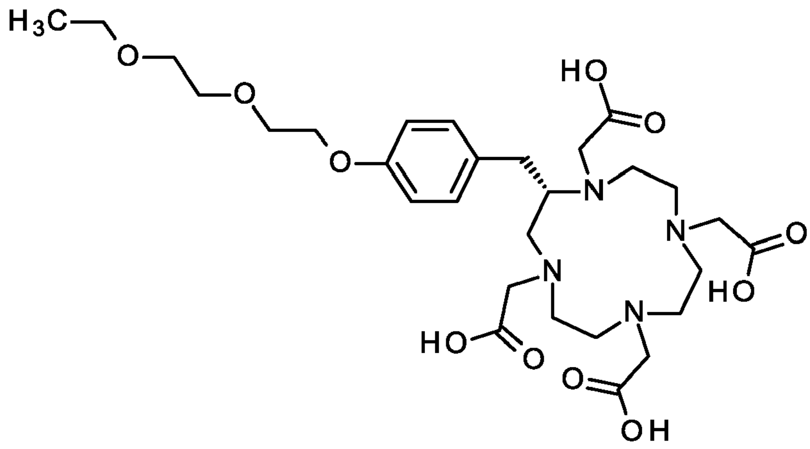

Tetra-*tert*-butyl-2,2',2",2'''-[(2S)-2-{4-[2-(2-ethoxyethoxy)ethoxy]benzyl}-1,4,7,10-tetraaza cyclododecane-1,4,7,10-tetrayl]tetraacetate (950 mg, 1.12 mmol) was dissolved in formic acid (14 ml, 0.38 mol; CAS: 64-18-6) and stirred for 22 h at 70°C. The reaction mixture was concentrated under reduced pressure and co-distilled twice with toluene/EtOH to yield 0.76 g (6% purity, 7% yield) of the title compound,

LC-MS (method 1): $R_t$ = 0.68 min; MS (ESIpos): m/z = 627 $[M+H]^+$

__Intermediate 3-1__

**methyl-*N*-(*tert*-butoxycarbonyl)-O-{2-[2-(2-methoxyethoxy)ethoxy]ethyl}-D-tyrosinate**

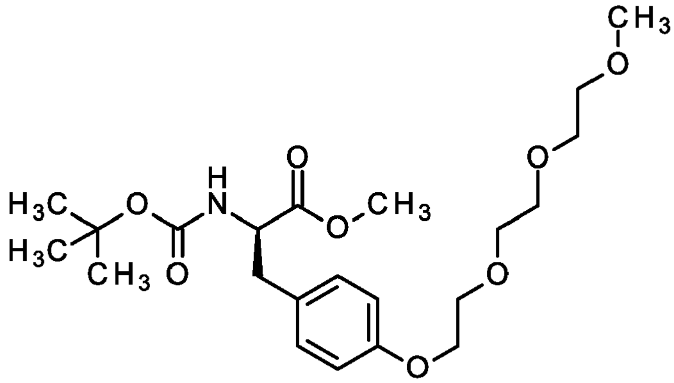

To methyl-*N*-(*tert*-butoxycarbonyl)-D-tyrosinate (5.00 g, 16.9 mmol) in THF (63 ml) was added 2-[2-(2-methoxyethoxy)ethoxy]ethanol (5.56 g, 33.9 mmol; CAS-RN:[112-35-6]), followed by triphenylphosphine (8.44 g, 32.2 mmol) and di-*tert*-butyl (*E*)-diazene-1,2-dicarboxylate (9.36 g, 40.6 mmol) at 0°C. The mixture was stirred for 15 h while it warmed to RT. One half of the solvent was removed under reduced pressure and hexane (30 ml) was added and the mixture was stirred for 2 h while a precipitate formed. The precipitate was removed by filtration. The filtrate was concentrated under reduced pressure and purified by column chromatography (silica gel, hexane / EtOAc, 0-50%)to yield 3.75 g (90% purity, 45% yield) of the title compound.

LC-MS (method 2): $R_t$ = 1.17 min; MS (ESIpos): m/z = 342 $[M+H-BOC]^+$.

$^1H$ NMR (400 MHz, $CDCl_3$) δ [ppm]= 1.42 (s, 9H), 3.01 (br t, 2H), 3.38 (s, 3H), 3.53 - 3.57 (m, 2H), 3.63 - 3.70 (m, 4H), 3.70 (s, 3H), 3.71 - 3.75 (m, 2H), 3.84 (m, 2H), 4.08 - 4.12 (m, 2H), 4.53 (br d, 1H), 4.94 (br d, 1H), 6.81 - 6.86 (m, 2H), 7.01 (d, 2H).

**Intermediate 3-2**

**tert-butyl-[(2*R*)-1-hydroxy-3-(4-{2-[2-(2-methoxyethoxy)ethoxy]ethoxy}phenyl)propan-2-yl]carbamate**

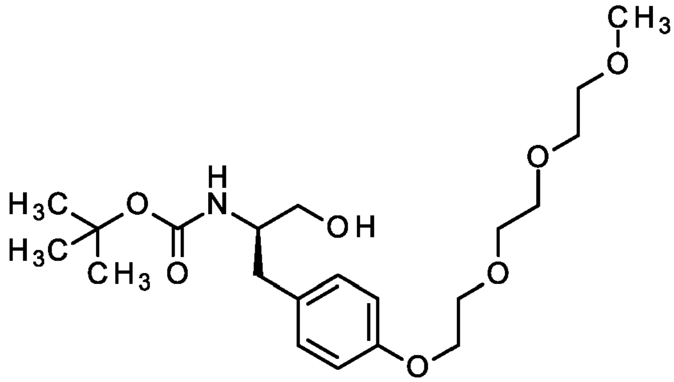

To methyl-*N*-(*tert*-butoxycarbonyl)-O-{2-[2-(2-methoxyethoxy)ethoxy]ethyl}-D-tyrosinate (3.77 g, 8.54 mmol) dissolved in THF (95 ml) was added lithium aluminum hydride in THF (17 ml, 1.0 M, 17 mmol; CAS-RN:16853-85-3) dropwise at 0°C. The mixture was stirred for 2 h while it warmed to RT. The reaction mixture was diluted with 2N HCL and EtOAc was added, phases were separated and the aqueous phase was extracted with EtOAc. Brine was added to the aqueous phase and was extracted with DCM/EtOH (9:1) two times. The combined organic extract was dried and concentrated under reduced pressure to yield 3.13 g (85% purity, 75% yield) of the title compound.

LC-MS (method 2): $R_t$ = 1.03 min; MS (ESIpos): m/z = 314 $[M+H-BOC]^+$.

$^1$H NMR (400 MHz, $CDCl_3$) $\delta$ ppm 1.41 (s, 9H), 2.76 (d, 2 H), 3.38 (s, 3H), 3.50 - 3.57 (m, 3H), 3.62 - 3.75 (m, 8H), 3.76 - 3.86 (m, 3H), 4.03 - 4.15 (m, 2H), 6.81 - 6.88 (m, 2H), 7.07 - 7.13 (m, 2H).

**Intermediate 3-3**

**(2*R*)-2-amino-3-(4-{2-[2-(2-methoxyethoxy)ethoxy]ethoxy}phenyl)propan-1-ol**

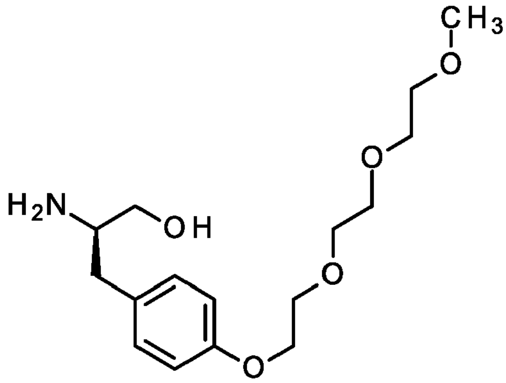

To *tert*-butyl-[(2*R*)-1-hydroxy-3-(4-{2-[2-(2-methoxyethoxy)ethoxy]ethoxy}phenyl)propan-2-yl]carbamate (3.13 g, 7.57 mmol) dissolved in DCM (25ml) was added HCl in dioxane (9.5 ml, 4.0 M, 38 mmol; CAS:7647-01-0) and stirred for 1 h at RT. The solvent was removed under reduced pressure. The residue was dissolved in DCM/EtOH (10%) and extracted with saturated $NaHCO_3$ solution and brine. The aqueous phase and was extracted twice with DCM/EtOH (10%). The combined organic extract was dried and concentrated under reduced pressure to yield 2.06 g (90% purity, 78% yield) of the title compound.

LC-MS (method 2): $R_t$ = 0.75 min; MS (ESIpos): m/z = 314 $[M+H]^+$.

$^1$H NMR (400 MHz, $CDCl_3$) $\delta$ [ppm]= 2.57 (br s, 3H), 2.64 - 2.85 (m, 3H), 3.20 - 3.30 (m, 1H), 3.36 - 3.39 (s,

3H), 3.43 - 3.50 (m, 1H), 3.53 - 3.57 (m, 2H), 3.62 - 3.70 (m, 5H), 3.71 - 3.75 (m, 2H), 3.81 - 3.88 (m, 2H), 4.08 - 4.12 (m, 2H), 6.81 - 6.87 (m, 2H), 7.08 - 7.14 (m, 2H).

## Intermediate 3-4

**(2*R*)-2-[(*E*)-(4-methoxybenzyliden)amino]-3-(4-(2-[2-(2-methoxyethoxy)ethoxy] ethoxy}phenyl)propan-1-ol**

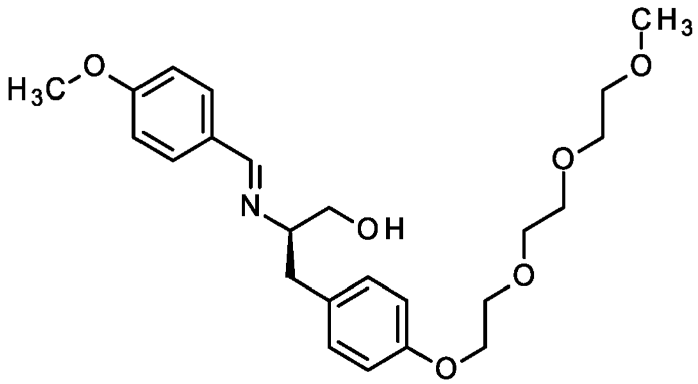

To (2*R*)-2-amino-3-(4-{2-[2-(2-methoxyethoxy)ethoxy]ethoxy}phenyl)propan-1-ol (2.06 g, 6.57 mmol) dissolved in toluene (27 ml) was added molecular sieve (4.63 g) and 4-methoxybenzaldehyd (800 µl, 6.6 mmol; CAS:123-11-5) and stirred for 2 h at 90°C. Molecular sieve was removed by filtration, the filtrate was concentrated under reduced pressure to yield 2.41 g (70% purity, 59% yield) of the title compound.

LC-MS (method 2): $R_t$ = 1.05 min; MS (ESIpos): m/z = 432 $[M+H]^+$.

$^1H$ NMR (400 MHz, $CDCl_3$) δ [ppm]: 1.167 (0.66), 1.192 (0.64), 1.250 (0.85), 1.268 (1.87), 1.286 (0.98), 1.440 (2.56), 1.971 (1.09), 2.055 (3.49), 2.364 (1.83), 2.792 (0.41), 2.877 (0.44), 3.380 (7.97), 3.382 (7.30), 3.389 (4.88), 3.542 (1.11), 3.551 (1.79), 3.558 (1.66), 3.565 (2.18), 3.573 (0.96), 3.641 (1.25), 3.646 (1.39), 3.648 (1.07), 3.653 (2.15), 3.658 (1.52), 3.661 (1.34), 3.665 (1.76), 3.668 (1.70), 3.679 (1.87), 3.686 (1.23), 3.691 (1.81), 3.702 (0.67), 3.722 (1.69), 3.731 (1.35), 3.736 (2.06), 3.746 (1.13), 3.749 (0.92), 3.761 (0.84), 3.768 (0.66), 3.779 (0.50), 3.795 (0.43), 3.810 (0.40), 3.818 (0.52), 3.825 (1.14), 3.830 (1.15), 3.840 (2.04), 3.852 (6.74), 3.873 (0.53), 3.906 (16.00), 4.071 (0.85), 4.085 (1.18), 4.095 (0.82), 4.103 (0.81), 4.121 (1.21), 4.138 (0.84), 6.784 (1.14), 6.806 (1.15), 6.833 (0.89), 6.855 (1.10), 6.879 (0.53), 6.905 (1.11), 6.927 (1.14), 7.009 (2.51), 7.013 (0.73), 7.026 (0.81), 7.031 (2.79), 7.038 (1.22), 7.060 (0.84), 7.141 (1.05), 7.162 (0.88), 7.193 (0.47), 7.247 (0.44), 7.625 (0.96), 7.648 (0.89), 7.844 (3.37), 7.849 (0.95), 7.861 (0.97), 7.867 (3.13), 7.946 (0.98), 9.901 (3.44).

## Intermediate 3-5

**(2*R*)-2-[(4-methoxybenzyl)amino]-3-(4-{2-[2-(2-methoxyethoxy)ethoxy]ethoxy} phenyl)propan-1-ol**

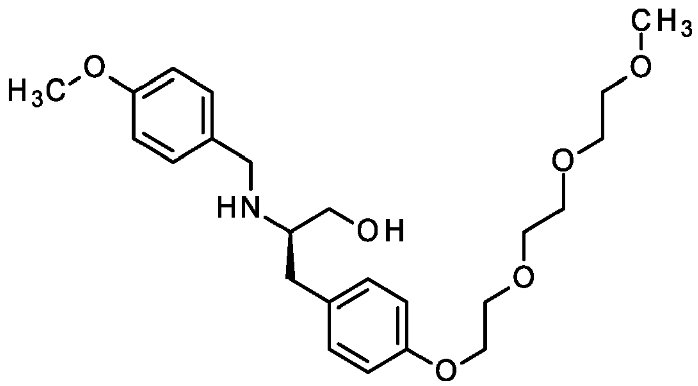

To (2*R*)-2-[(*E*)-(4-methoxybenzyliden)amino]-3-(4-{2-[2-(2-methoxyethoxy)ethoxy]ethoxy} phenyl)propan-1-ol (2.43 g, 5.63 mmol) dissolved in MeOH (17ml) was added sodium borohydride (320 mg, 8.45 mmol; CAS: 16940-66-2) in 3 portions at 0°C and stirred for 1h. Acetone (1.7ml) was added and warmed to RT. The mixture was filtered over Celite, washed with MeOH and concentrated under reduced pressure. The residue was dissolved in DCM/EtOH (9:1) and extracted with water. The aqueous phase and was extracted with DCM/EtOH (9:1) twice. The combined organic extracts were extracted with brine, dried and concentrated under reduced pressure to yield 2,31 g (60% purity, 57% yield) of the title compound.

LC-MS method 1): $R_t$ = 1.03 min; MS (ESIpos): m/z = 434 $[M+H]^+$

$^1$H NMR (400 MHz, $CDCl_3$) δ [ppm]: 1.177 (0.60), 1.439 (1.57), 1.969 (0.76), 2.770 (0.40), 2.789 (0.51), 2.845 (0.44), 3.384 (2.13), 3.387 (16.00), 3.549 (1.25), 3.557 (1.42), 3.560 (1.84), 3.565 (1.42), 3.568 (0.51), 3.572 (2.30), 3.648 (0.44), 3.653 (2.67), 3.660 (1.87), 3.665 (1.87), 3.668 (1.54), 3.677 (1.53), 3.679 (1.15), 3.682 (1.13), 3.691 (2.21), 3.697 (1.57), 3.704 (1.66), 3.706 (1.53), 3.737 (1.73), 3.739 (1.83), 3.746 (1.36), 3.753 (1.86), 3.761 (0.79), 3.763 (0.75), 3.767 (0.61), 3.773 (1.14), 3.786 (11.52), 3.793 (0.59), 3.814 (0.59), 3.821 (13.27), 3.827 (1.55), 3.850 (1.30), 3.861 (1.55), 3.864 (1.20), 3.873 (1.41), 4.104 (1.43), 4.117 (1.56), 4.129 (1.12), 4.633 (4.14), 6.840 (1.90), 6.844 (2.39), 6.849 (0.95), 6.857 (0.88), 6.861 (2.58), 6.865 (2.36), 6.894 (1.96), 6.899 (0.71), 6.911 (0.65), 6.916 (2.12), 7.057 (1.64), 7.078 (1.35), 7.199 (1.34), 7.220 (1.16), 7.296 (1.70), 7.302 (0.55), 7.313 (0.47), 7.318 (1.59).

**Intermediate 3-6**

**(2*R*)-1-(4-methoxybenzyl)-2-(4-{2-[2-(2-methoxyethoxy)ethoxy]ethoxy}benzyl)aziridine**

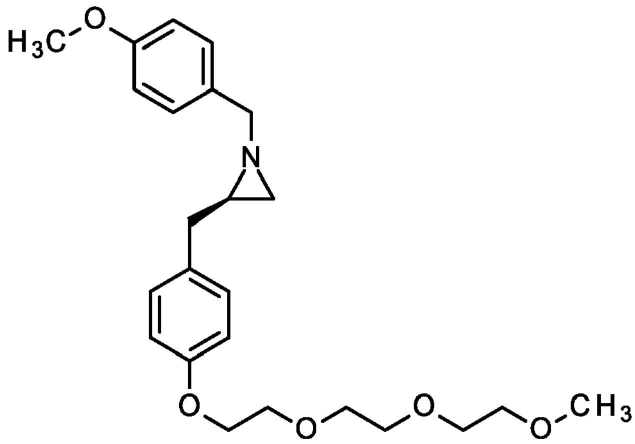

To (2R)-2-[(4-methoxybenzyl)amino]-3-(4-{2-[2-(2-methoxyethoxy)ethoxy]ethoxy}phenyl) propan-1-ol (2.31 g, 5.33 mmol) dissolved in THF (21ml) was added (1.96 g, 7.46 mmol; CAS:603-35-0) and di-*tert*-butyl-(*E*)-diazen-1,2-dicarboxylate (1.72 g, 7.46 mmol; CAS:870-50-8) at 0°C and the mixture was stirred for 16h while it warmed to RT. One half of the solvent was removed under reduced pressure, hexane (45 ml) was added and the mixture was stirred for 1,5 h while a precipitate formed. The precipitate was removed by filtration. The filtrate was concentrated under reduced pressure and purified by column chromatography (silica gel, hexane / EtOAc, 40-100%) to yield 1.70 g (85% purity, 65% yield) of the title compound.

LC-MS (method 2): $R_t$ = 1.17 min; MS (ESIpos): m/z = 416 $[M+H]^+$

$^1$H NMR (400 MHz, $CDCl_3$) δ [ppm]= 1.66 - 1.75 (m, 2H), 2.52 - 2.59 (m, 1H), 2.73 (dd, 1H), 3.27 - 3.43 (m, 5H), 3.35 - 3.40 (m, 4H), 3.52 - 3.58 (m, 2H), 3.64 - 3.71 (m, 4H), 3.71 - 3.82 (m, 1H), 3.78 - 3.82 (m, 2H), 3.85 (dd, 2H), 4.08 - 4.12 (m, 2H), 6.76 - 6.85 (m, 4H), 7.05 - 7.11 (m, 1H), 7.10 - 7.11 (m, 1H), 7.20 (d, 2H).

**Cyclen intermediate 3-1**

**(2*R*)-1,7,10-tribenzyl-4-(4-methoxybenzyl)-2-(4-{2-[2-(2-methoxyethoxy) ethoxy]ethoxy}benzyl)-1,4,7,10-tetraazacyclododecane**

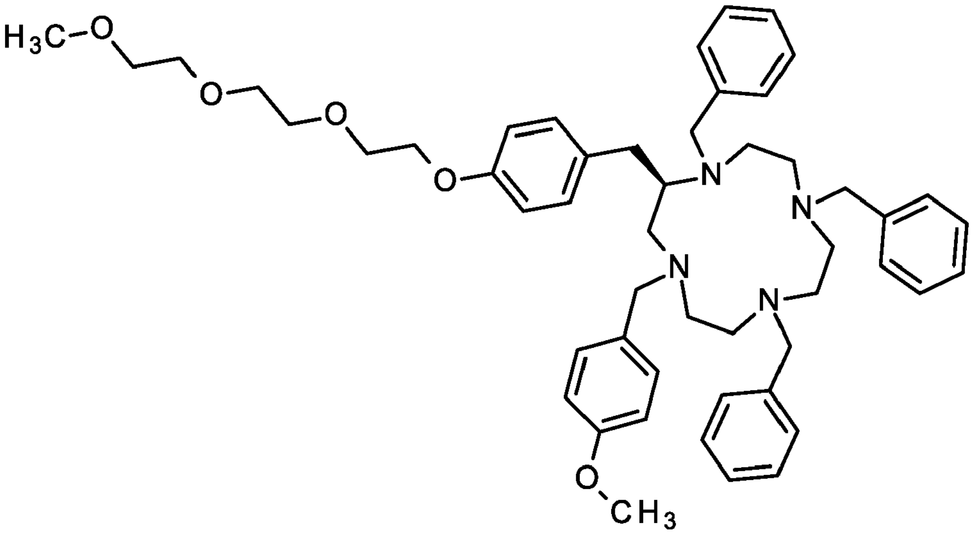

To (2R)-1-(4-methoxybenzyl)-2-(4-{2-[2-(2-methoxyethoxy)ethoxy]ethoxy}benzyl)aziridine (1.70 g, 85% purtity, 3.48 mmol) and 1-benzylaziridine (29 ml, 0.49 M, 14 mmol) (emulsion in water 0,065 g/ml) dissolved in EtOH (19 ml) was added p-toluenesulfonic acid monohydrate (827 mg, 4,35 mmol; CAS: 6192-52-5) in water (410 μl) over 8 h slowly at 60°C followed by additional stirring at 60°C for 7 h and reflux for 2 h. After cooling to RT sodium hydroxide (259 mg, 6,46 mmol) dissolved in water (520 μl) was added and the reaction mixture was extracted twice with DCM. The combined organic extracts were washed with brine, dried, concentrated under reduced pressure and purified by column chromatography (amino phase functionalized silica gel, hexane / EtOAc 40-100%) to yield 1.46 g (48% purity, 25% yield) of the title compound.

LC-MS (method 1): $R_t$ = 1.20 min; MS (ESIpos): m/z = 815 $[M+H]^+$.

## Cyclen intermediate 3-2

**(2*R*)-2-(4-{2-[2-(2-methoxyethoxy)ethoxy]ethoxy}benzyl)-1,4,7,10-tetraazacyclododecane**

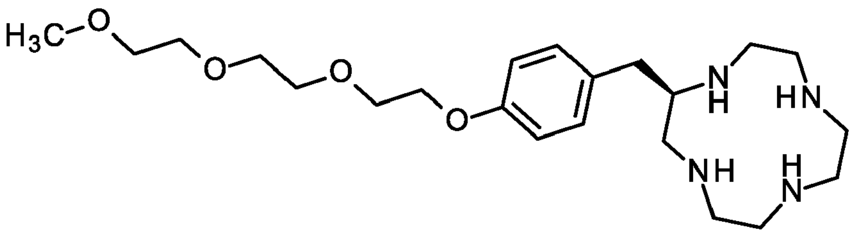

To (2*R*)-1,7,10-tribenzyl-4-(4-methoxybenzyl)-2-(4-{2-[2-(2-methoxyethoxy)ethoxy]ethoxy} benzyl)-1,4,7,10-tetraazacyclododecane (1.46 g, 1.79 mmol) dissolved in EtOH (19 ml) and acetic acid (19 ml) was added palladium on activated carbon (286 mg, 10% purity, 269 μmol; CAS: 7440-05-3) and stirred under hydrogen pressure (40 bar) at 80°C for 43 h. The catalyst was removed by filtration and washed with EtOH and THF. The filtrate was concentrated under reduced pressure to yield 1,63 g (23% purity, 49% yield) of the title compound.

LC-MS (method 1): ): $R_t$ = 0.54 min; MS (ESIpos): m/z = 425 $[M+H]^+$

## Cyclen intermediate 3-3

**Tetra-tert-buty)-2,2',2",2'''-[(2R)-2-(4-{2-[2-(2-methoxyethoxy)ethoxy]ethoxy}benzy))-1,4,7,10-tetraazacyclododecane-1,4,7,10-tetrayl]tetraacetate**

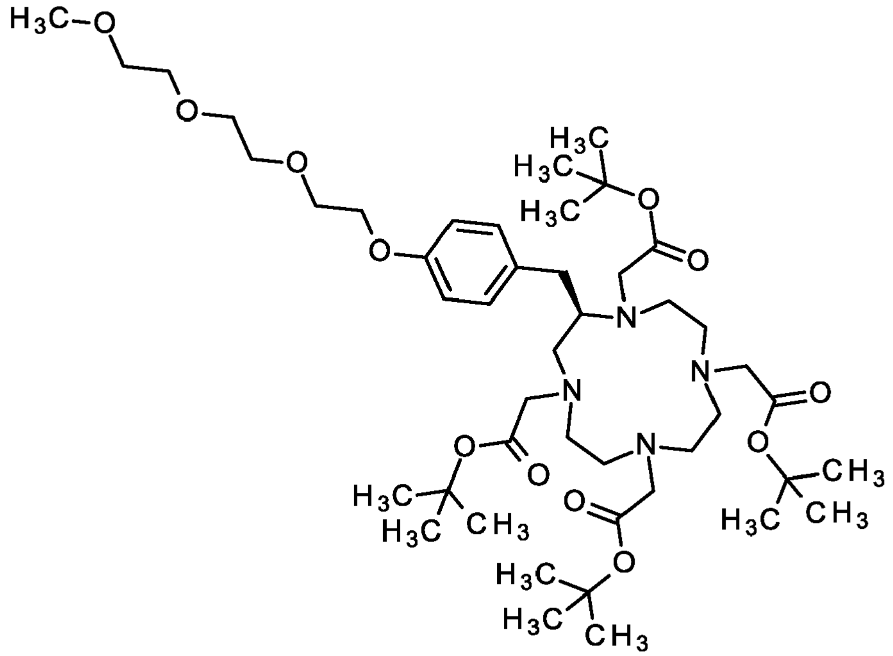

To (2*R*)-2-(4-{2-[2-(2-methoxyethoxy)ethoxy]ethoxy}benzyl)-1,4,7,10-tetraazacyclododecane (1.63 g, 3.84 mmol) dissolved in acetonitrile (51 ml) was added potassium carbonate (5.31 g, 38.4 mmol; CAS-RN:584-08-7) and the mixture was stirred for 1h at 50°C. *Tert*-butyl bromoacetate (2.5 ml, 17 mmol) was added and stirred at RT for 43h. *Tert*-butyl methyl ether and water were added and phases were separated. The aqueous phase was extracted with *tert*-butyl methyl ether. The combined organic extract was washed with aqueous sodium hydroxide solution (1M) and brine, dried and concentrated under reduced pressure to yield 3,51 g of (43% purity, 45% yield) of the title compound.
LC-MS (method 1): $R_t$ = 1.35 min; MS (ESIpos): m/z = 881 $[M+H]^+$.

## Free ligand 3

**2,2',2",2'''-[(2*R*)-2-(4-(2-[2-(2-methoxyethoxy)ethoxy]ethoxy)benzyl)-1,4,7,10-tetraazacyclododecane-1,4,7,10-tetrayl]tetraacetic acid**

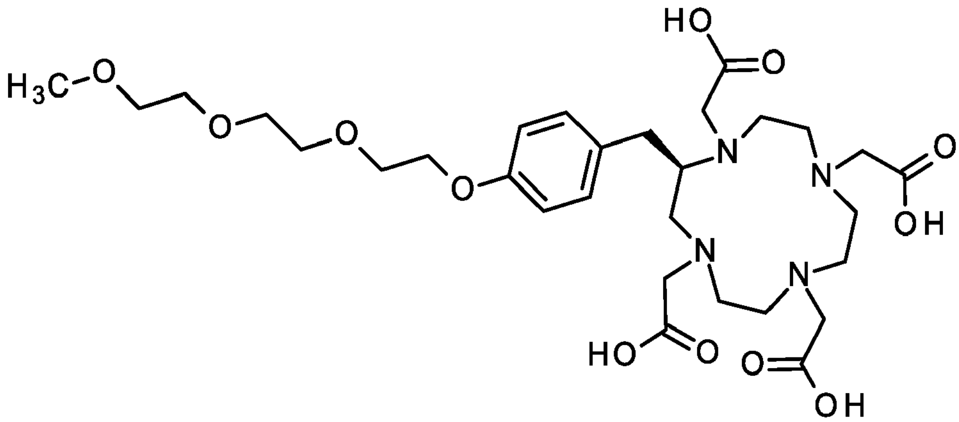

Tetra-*tert*-butyl-2,2',2",2'''-[(2*R*)-2-(4-{2-[2-(2-methoxyethoxy)ethoxy]ethoxylbenzyl)-1,4,7,10-tetraazacyclododecane-1,4,7,10-tetrayl]tetraacetate (3.51 g, 3.98 mmol) was dissolved in formic acid (51 ml, 1.4 mol; CAS-RN:64-18-6) and stirred for 24 h at 70°C. The reaction mixture was concentrated under reduced pressure and co-distilled twice with toluene/EtOH to yield 2,78g (56% purity, 60% yield) of the title compound.
LC-MS (method 1): $R_t$ = 0.60 min; MS (ESIpos): m/z = 655 $[M+H]^+$.

Intermediate 4-1

**methyl-*N*-(*tert*-butoxycarbonyl)-O-(2-ethoxyethyl)-L-tyrosinate**

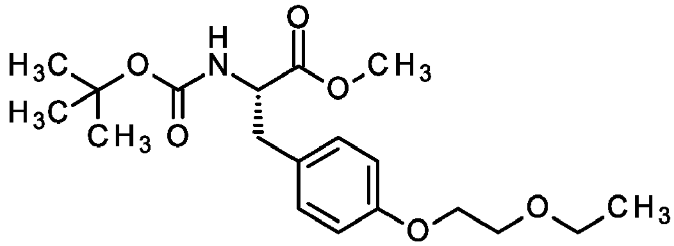

To a solution of methyl *N*-(*tert*-butoxycarbonyl)-L-tyrosinate (60 g, 203 mmol) in *N*,*N*-dimethyl formamide (150 ml) were added potassium carbonate (28.1 g, 203 mmol) and 1-bromo-2-ethoxyethane (34 ml, 300 mmol) at room temperature and the mixture was stirred 60°C for 16 hours. Ethyl acetate and water were added and layers were separated. The aqueous phase was extracted with ethyl acetate. The combined extract was washed with brine, dried over sodium sulfate, filtered, and concentrated under reduced pressure to give a residue. The residue was purified by column chromatography (silica gel, hexane / ethyl acetate, 0 - 5%) to yield 63,2 g (85% yield) of the title compound.

LC-MS (method 3): $R_t$ = 0.94 min; MS (ESIpos): m/z = 268 $[M\text{-}BOC+H]^+$.

Intermediate 4-2

***tert*-butyl-{(2*S*)-1-[4-(2-ethoxyethoxy)phenyl]-3-hydroxypropan-2-yl}carbamate**

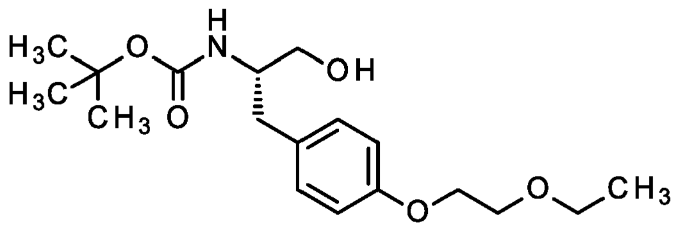

To a solution of methyl-*N*-(*tert*-butoxycarbonyl)-O-(2-ethoxyethyl)-L-tyrosinate (63.2 g, 172 mmol) in tetrahydrofuran (650 ml) was added lithium aluminum hydride (7.63 g, 206 mmol) at 0 °C. The mixture was stirred at room temperature for 2 hours under nitrogen. The mixture was quenched with saturated ammonium chloride solution and saturated potassium sodium tartrate tetrahydrate solution at 0°C. The mixture was stirred for 1 hour at room temperature and then extracted with ethyl acetate. The combined organic phase was washed with brine, dried over anhydrous sodium sulfate and concentrated under reduced pressure to yield 58 g (99% yield) of the title compound.

LC-MS (method 3): $R_t$ = 0.84 min; MS (ESIpos): m/z = 240 $[M\text{-}BOC+H]^+$.

Intermediate 4-3

**(2*S*)-2-amino-3-[4-(2-ethoxyethoxy)phenyl]propan-1-olhydrochloride (1:1)**

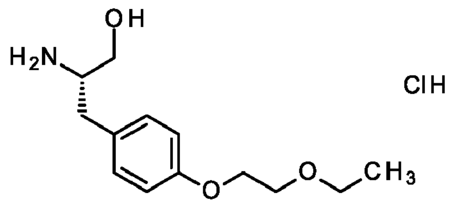

To a solution of *tert*-butyl-{(2*S*)-1-[4-(2-ethoxyethoxy)phenyl]-3-hydroxypropan-2-yl}carbamate (48.0 g, 141 mmol) in methyl acetate (100 ml) was added hydrochloric acid (220 ml, 4.0 M in ethyl acetate , 880 mmol) at room temperature. The mixture was stirred at room temperature for 1 hour. The mixture was concentrated under reduced pressure to yield 43.9 g (113% yield) of the title compound.

LC-MS (method 3): $R_t$ = 0.73 min; MS (ESIpos): m/z = 240 $[M+H]^+$.

## Intermediate 4-4

**(2S)-3-[4-(2-ethoxyethoxy)phenyl]-2-[(4-methoxybenzyl)amino]propan-1-ol**

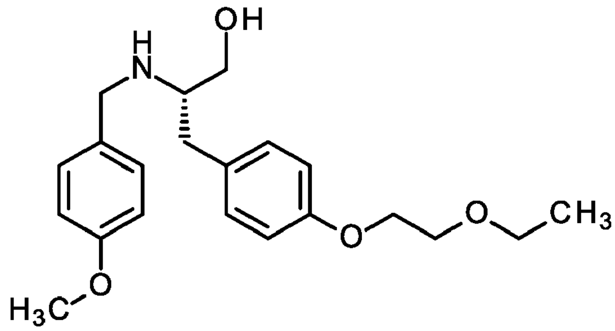

To a solution of (2*S*)-2-amino-3-[4-(2-ethoxyethoxy)phenyl]propan-1-ol hydrochloride (43.9 g, 159 mmol) in methanol was added sodium acetate (13.1 g, 159 mmol) at room temperature. After 0.5 h acetic acid (57 ml), 4-methoxybenzaldehyde (19 ml, 160 mmol) and sodium cyanoborohydride (15.0 g, 239 mmol) were added at room temperature. The mixture was stirred at 50 °C for 16 hours under nitrogen. The mixture was quenched with saturated sodium bicarbonate solution, and then concentrated to give a residue. The residue was diluted with water, and then extracted with dichloromethane. The combined organic phase was washed with brine, dried over anhydrous sodium sulfate, filtered, concentrated and purified by preparative HPLC (Instrument: Agela-H1000GC500; Column: Phenomenex luna C18 I.D.150mm*H400mm, 15μm; 100 Å; eluent A: water (0.1% formic acid), eluent B: acetonitrile; gradient: 15%-22%,5min;22%,20min; flow 400 ml/min; temperature: room temperature; Detector: UV 220/254 nm) to yield 18,1 g (32% yield) of the title compound.

LC-MS (method 3): $R_t$ = 0.78 min; MS (ESIpos): m/z = 360 $[M+H]^+$.

$^1H$ NMR (400 MHz, DMSO-$d_6$): δ [ppm] = 7.17 (d, 2H), 7.07 (d, 2H), 6.83 (d, 4H), 4.48 (t, 1H), 4.07-4.00 (m, 2H), 3.72 (s, 3H), 3.69-3.64 (m, 4H), 3.49 (q, 2H), 3.35-3.17 (m, 3H), 2.69-2.53 (m, 3H), 1.12 (t, 3H).

## Intermediate 4-5

**(2*S*)-2-[4-(2-ethoxyethoxy)benzyl]-1-(4-methoxybenzyl)aziridine**

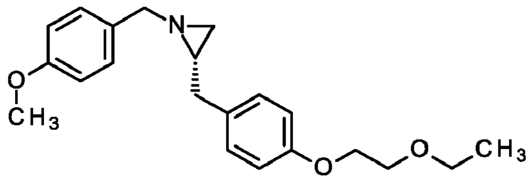

To a solution of (2*S*)-3-[4-(2-ethoxyethoxy)phenyl]-2-[(4-methoxybenzyl)amino]propan-1-ol (11.5 g, 32.0 mmol) in tetrahydrofuran (200 ml) were added tri-n-butyl phosphine (12 ml, 48 mmol) and (*E*)-*N*,*N*,*N*',*N*'-tetramethyldiazene-1,2-dicarboxamide (8.26 g, 48.0 mmol) at room temperature. The mixture was stirred at room temperature for 2 hours under nitrogen and filtered. The filtrate was concentrated and purified by flash column chromatography (100-200 mesh, hexane / ethyl acetate, 0 to 50%) to yield 11,5 g (97% purity, quantitative) of the title compound.

LC-MS (method 3): $R_t$ = 0.77 min; MS (ESIpos): m/z = 342 $[M+H]^+$

$^1H$ NMR (400 MHz, DMSO-$d_6$): δ [ppm]= 7.19 (d, 2H), 7.08 (d, 2H), 6.85-6.78 (m, 4H), 4.06-4.00 (m, 2H), 3.73 (s, 3H), 3.71-3.64 (m, 2H), 3.49 (q, 2H), 3.33-3.17 (m, 2H), 2.63-2.56 (m, 1H), 2.49-2.43 (m, 1H), 1.72-1.62 (m, 1H), 1.53 (d, 1H), 1.34 (d, 1H), 1.12 (t, 3H).

## Cyclen intermediate 4-1

**(2*S*)-1,7,10-tribenzyl-2-[4-(2-ethoxyethoxy)benzyl]-4-(4-methoxybenzyl)-1,4,7,10-tetraazacyclododecane**

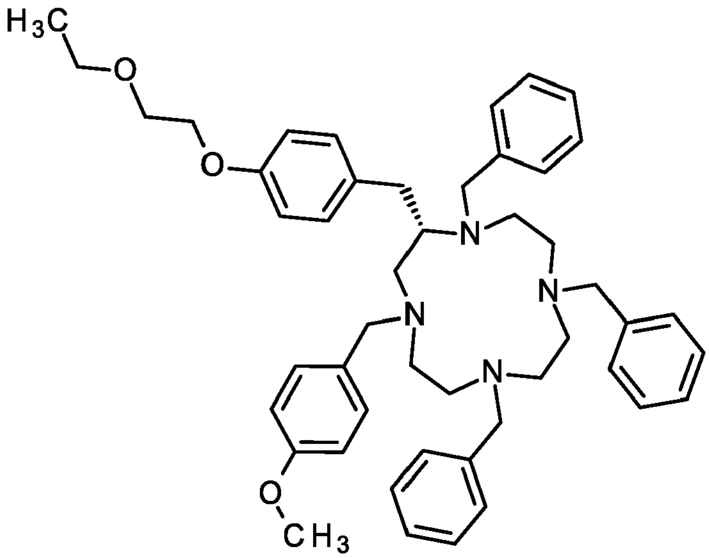

To (2S)-2-[4-(2-ethoxyethoxy)benzyl]-1-(4-methoxybenzyl)aziridine (3.00 g, 8.79 mmol) and 1-benzylaziridine (72 ml, 0.49M, 35 mmol) (emulsion in water 0,065 g/ml) dissolved in EtOH (47 ml) was added slowly p-toluenesulfonic acid monohydrate (2.09 g, 11.0 mmol) in water (1 ml) over 8 h at 60°C followed by additional stirring at 60°C for 7h and reflux for 2h. After cooling to RT sodium hydroxide (653 mg, 16.3 mmol) in water (1,3 ml) was added and the reaction mixture was extracted twice with DCM. The combined organic extract was washed with brine, dried, concentrated under reduced pressure and purified by column chromatography (amino phase functionalized silica gel, hexane / EtOAc, 40-100%) to yield 930 mg (5,2% yield) of the title compound.

LC-MS (method 1): $R_t$ = 1.37 min; MS (ESIpos): m/z = 741 $[M+H]^+$.

## Cyclen intermediate 4-2

**(2*S*)-2-[4-(2-ethoxyethoxy)benzyl]-1,4,7,10-tetraazacyclododecane**

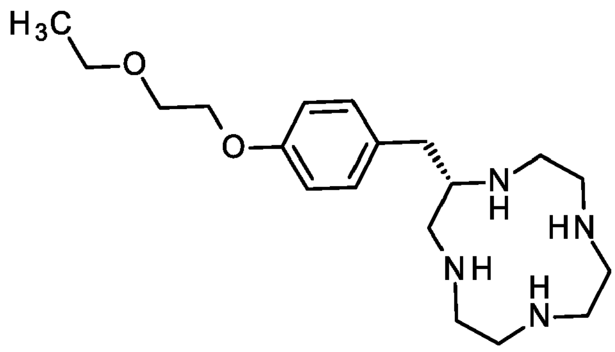

To (2S)-1,7,10-tribenzyl-2-[4-(2-ethoxyethoxy)benzyl]-4-(4-methoxybenzyl)-1,4,7,10-tetraaza cyclododecane (930 mg, 1.25 mmol) dissolved in EtOH (13 ml) and acetic acid (13 ml) was added palladium on activated carbon (200 mg, 10% purity, 188 μmol) and the mixture was stirred under hydrogen pressure (40 bar) at 80°C for 42 h. The catalyst was removed by filtration and washed with EtOH and THF. The filtrate was concentrated under reduced pressure to yield 897 mg (48% purity, 98% yield) of the title compound.

LC-MS (method 1): $R_t$ = 0.52 min; MS (ESIpos): m/z = 351 $[M+H]^+$

## Cyclen intermediate 4-3

**Tetra-tert-butyl-2,2',2",2'''-((2S)-2-[4-(2-ethoxyethoxy)benzyl]-1,4,7,10-tetraaza cyclododecane-1,4,7,10-tetrayl}tetraacetate**

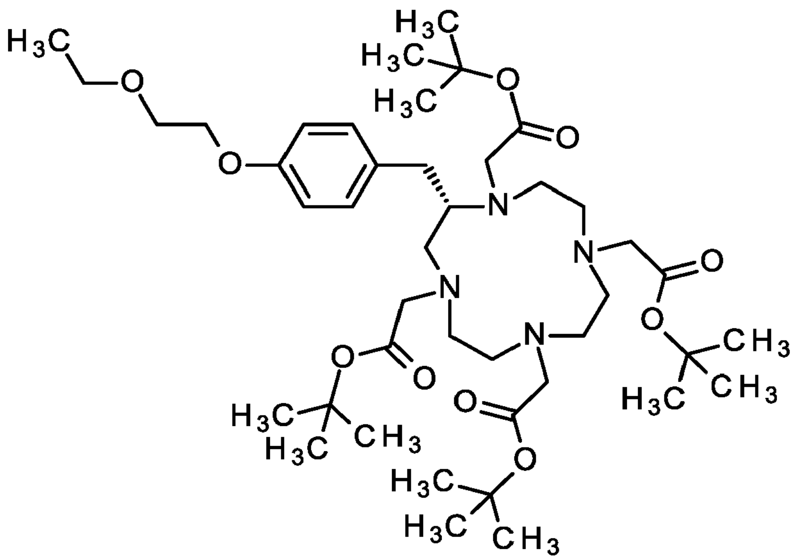

To (2S)-2-[4-(2-ethoxyethoxy)benzyl]-1,4,7,10-tetraazacyclododecane (897 mg, 2.56 mmol) dissolved in acetonitrile (34 ml) was added potassium carbonate (3.54 g, 25.6 mmol) and stirred for 1h at 50°C. *Tert*-butyl bromoacetate (2.1 ml, 14 mmol) was added and stirred at RT for 90h. *Tert*-butyl methyl ether and water were added and phases were separated. The aqueous phase was extracted with *tert*-butyl methyl ether. The combined organic extracts were washed with aqueous sodium hydroxide solution (1M) and brine, dried and concentrated under reduced pressure to yield 1,37g of (46% purity, 30% yield) of the title compound.

LC-MS (method 1): $R_t$ = 1.32 min; MS (ESIpos): m/z = 808 $[M+H]^+$

**Free ligand 4**

**2,2',2",2'"((2*S*)-2-[4-(2-ethoxyethoxy)benzyl]-1,4,7,10-tetraazacyclododecane-1,4,7,10-tetrayl}tetraacetic acid**

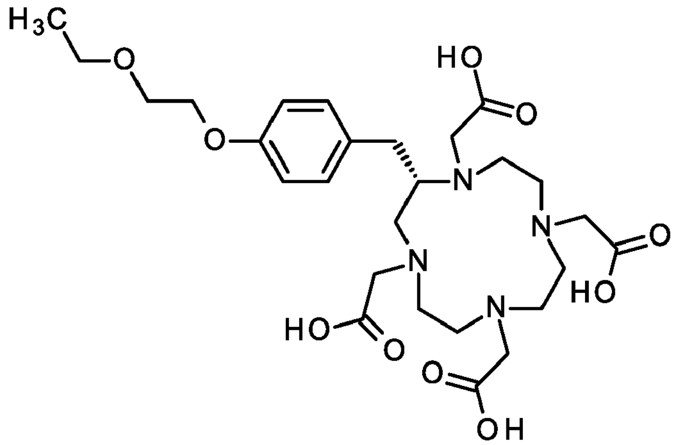

Tetra-*tert*-butyl-2,2',2",2'"-{(2*S*)-2-[4-(2-ethoxyethoxy)benzyl]-1,4,7,10-tetraazacyclo dodecane-1,4,7,10-tetrayl}tetraacetate (1.37 g, 1.70 mmol) was dissolved in formic acid (22 ml, 580 mmol) and stirred for 4 h at 70°C. The reaction mixture was concentrated under reduced pressure and co-distilled twice with toluene / EtOH to yield 1,09 g (50% purity, 55% yield) of the title compound.

LC-MS (method 1): $R_t$ = 0.62 min; MS (ESIpos): m/z = 583 $[M+H]^+$.

**Intermediate 5-1**

**2-[2-(2-ethoxyethoxy)ethoxy]ethyl-4-methylbenzensulfonate**

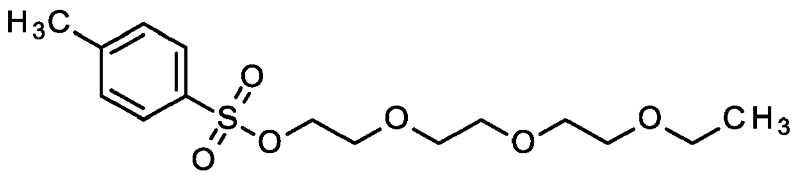

To a mixture of 2-[2-(2-ethoxyethoxy)ethoxy]ethanol (50 g, 281 mmol) and sodium hydroxide (4M in water) (140 ml, 560 mmol) in tetrahydrofuran (600 ml) was added a solution of 4-toluenesulfonyl chloride (64.2 g, 337 mmol) in tetrahydrofuran (150 ml) at 0°C. The mixture was stirred at room temperature for 16 hours under nitrogen. The mixture was diluted with ethyl acetate and washed with brine, The organic phase was dried over anhydrous sodium sulfate, filtered and concentrated to yield 92,8 g (quantitative) of the title compound.

LC-MS (method 3): $R_t$ = 0.88 min; MS (ESIpos): m/z = 333 $[M+H]^+$

**Intermediate 5-2**

**methyl-*N*-(*tert*-butoxycarbonyl)-O-(2-[2-(2-ethoxyethoxy)ethoxy]ethyl)-L-tyrosinate**

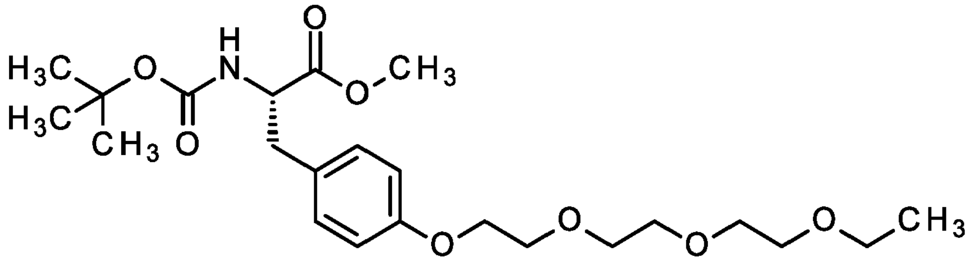

To a solution of methyl-*N*-(*tert*-butoxycarbonyl)-L-tyrosinate (40 g, 135 mmol) and 2-[2-(2-ethoxyethoxy)ethoxy]ethyl-4-methylbenzensulfonat (90 g, 271 mmol) in *N,N*-dimethylformamide (400 ml) was added potassium carbonate (37.4 g, 271 mmol) and sodium iodide (2.03 g, 13.5 mmol) at room temperature. The mixture was stirred at 80 °C for 16 hours under nitrogen. The mixture was diluted with water and ethyl acetate. After separated, the organic phase was washed with brine, dried over anhydrous sodium sulfate filtered and concentrated to yield 75,8 g (quantitative) of the title compound.

LC-MS (method 3): $R_t$ = 0.92 min; MS (ESIpos): m/z = 356.3 $[M-100+H]^+$.

## Intermediate 5-3

### *tert*-butyl-[(2*S*)-1-(4-{2-[2-(2-ethoxyethoxy)ethoxy]ethoxy}phenyl)-3-hydroxypropan-2-yl]carbamate

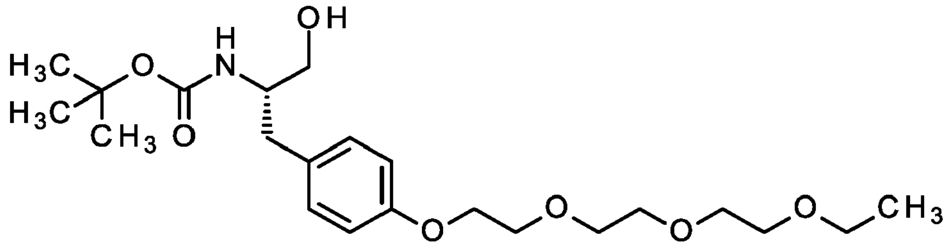

To a solution of methyl-*N*-(*tert*-butoxycarbonyl)-O-{2-[2-(2-ethoxyethoxy)ethoxy]ethyl}-L-tyrosinate (75.8 g, 166 mmol) in tetrahydrofuran (750 ml) was added lithium aluminum hydride (6.15 g, 166 mmol) at 0 °C. The mixture was stirred at room temperature for 2 hours under nitrogen. The mixture was quenched with saturated ammonium chloride solution and saturated potassium sodium tartrate tetrahydrate solution at 0 °C, and then extracted with ethyl acetate. The combined organic phase was washed with brine, dried over anhydrous sodium sulfate and concentrated in vacuum to yield 52,2 g (73% yield) of the title compound.

LC-MS (method 3): $R_t$ = 0.877 min; MS (ESIpos): m/z = 328.3 $[M-100+H]^+$.

## Intermediate 5-4

### (2*S*)-2-Amino-3-(4-{2-[2-(2-ethoxyethoxy)ethoxy]ethoxy}phenyl)propan-1-ol

### hydrochloride (1:1)

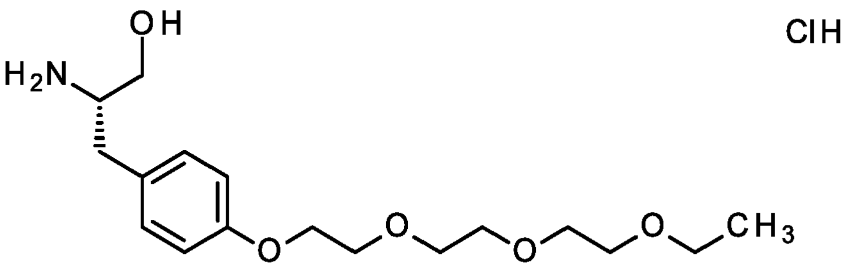

To a solution of *tert*-butyl-[(2*S*)-1-(4-{2-[2-(2-ethoxyethoxy)ethoxy]ethoxy}phenyl)-3-hydroxypropan-2-yl]carbamate (52.2 g, 122 mmol) in ethyl acetate (70 ml) was added hydrochloric acid (4N in ethyl acetate) (200 ml, 800 mmol) at room temperature. The mixture was stirred at room temperature for 1 hour. The mixture was concentrated to yield 51,4 g (quantitative) of the title compound.
LC-MS (method 3): $R_t$ = 0.630 min; MS (ESIpos): m/z = 328.3 $[M+H]^+$.

## Intermediate 5-5

**(2*S*)-3-(4-{2-[2-(2-ethoxyethoxy)ethoxy]ethoxy}phenyl)-2-[(4-methoxybenzyl)amino]propan-1-ol**

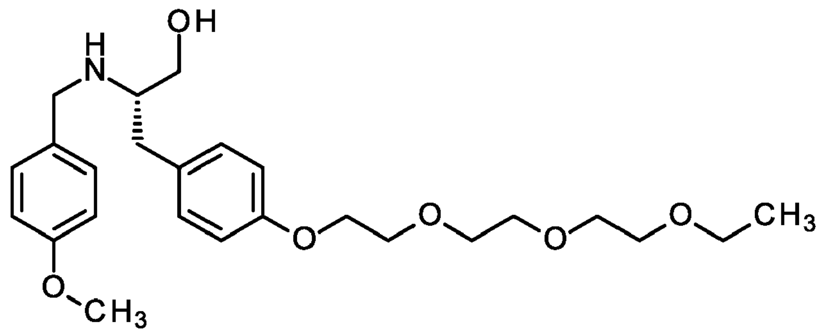

To a solution of (2S)-2-Amino-3-(4-{2-[2-(2-ethoxyethoxy)ethoxy]ethoxy}phenyl)propan-1-olhydrochlorid (51.4 g, 141 mmol) in methanol (500 ml) was added sodium acetate (11.6 g, 141 mmol) at room temperature. After 0.5 hour, acetic acid (60 ml), 4-methoxybenzaldehyde (17 ml, 140 mmol) and sodium cyanoborohydride (13.3 g, 212 mmol) were added at room temperature . The mixture was stirred at 50 °C for 16 hours under nitrogen. The mixture was quenched with saturated sodium bicarbonate solution, and then concentrated, diluted with water, and then extracted with ethyl acetate. The combined organic phase was washed with brine, dried over anhydrous sodium sulfate filtered and concentrated. The residue was purified by reversed phase (Instrument: Agela-H1000GC500; Column: Welch Ultimate XB_C18 I.D.100mm*H400mm,20/40μm; 100 Å; eluent A: water (0.1% formic acid), eluent B: acetonitrile; gradient: 10%-25%,35min;25%,23min; flow: 400 ml/min; temperature: room temperature; Detector: UV 220/254 nm) to yield 19,1 g (30 % yield) of the title compound.
LC-MS (method 3): $R_t$ = 0.79 min; MS (ESIpos): m/z = 448 $[M+H]^+$

## Intermediate 5-6

**(2*S*)-2-(4-{2-[2-(2-ethoxyethoxy)ethoxy]ethoxy}benzyl)-1-(4-methoxybenzyl)aziridine**

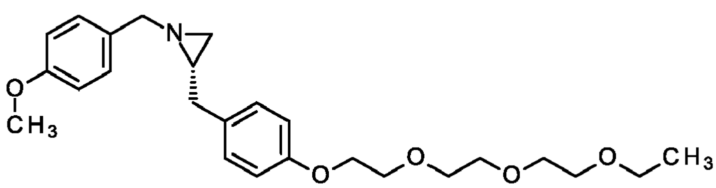

To a solution of (2S)-3-(4-{2-[2-(2-ethoxyethoxy)ethoxy]ethoxy}phenyl)-2-[(4-methoxybenzyl) amino]propan-1-ol (19.1 g, 42.7 mmol) in tetrahydrofuran (270 ml) were added tri-n-butyl phosphine (16 ml, 64 mmol) and (*E*)-*N*,*N*,*N*',*N*'-tetramethyldiazene-1,2-dicarboxamide (11.0 g, 64 mmol) at room temperature. The mixture was stirred at room temperature for 16 hours under nitrogen. The mixture was filtered, the filtrate was concentrated and purified by column chromatography (silica gel 100-200 mesh, hexane / ethyl acetate, 0 to 50%) to yield 10.2 g (91% purity, 51% yield) of the title compound.
LC-MS (method 3): $R_t$ = 0.78 min; MS (ESIpos): m/z = 452.4 $[M+Na]^+$.
$^1H$ NMR (400 MHz, DMSO-$d_6$): δ [ppm]= 7.19 (d, 2H), 7.09 (d, 2H), 6.86-6.76 (m, 4H), 4.07-3.99 (m, 2H), 3.76-3.68 (m, 5H), 3.60-3.56 (m, 2H), 3.55-3.49 (m, 4H), 3.48-3.38 (m, 4H), 3.33-3.19 (m, 2H), 2.63-2.55 (m, 1H),

2.48-2.43 (m, 1H), 1.73-1.64 (m, 1H), 1.54 (d, 1H), 1.34 (d, 1H), 1.09 (t, 3H).

**Cyclen intermediate 5-1**

**(2*S*)-1,7,10-tribenzyl-2-(4-{2-[2-(2-ethoxyethoxy)ethoxy]ethoxy}benzy))-4-(4-methoxybenzyl)-1,4,7,10-tetraaza-cyclododecan**

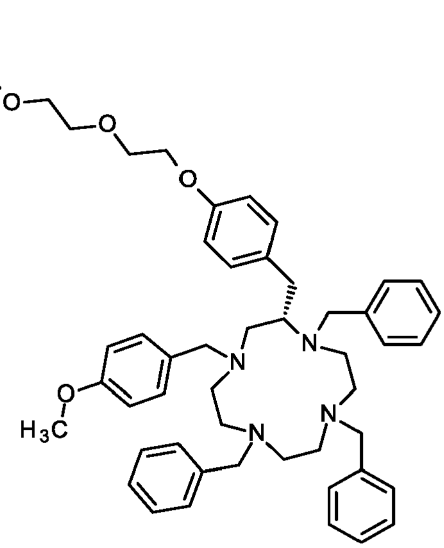

To (2S)-2-(4-{2-[2-(2-ethoxyethoxy)ethoxy]ethoxy}benzyl)-1-(4-methoxybenzyl)aziridine (3.00 g, 6.98 mmol) and 1-benzylaziridine (57 ml, 0.49 M, 28 mmol) (emulsion in water 0,065g/ml) dissolved in EtOH (37 ml) was added p-toluenesulfonic acid monohydrat (1.66 g, 8.73 mmol) dissolved in water (830 μl) over 8 h slowly at 60°C followed by stirring at 60°C for 7h and reflux for 2h. After cooling to RT sodium hydroxide (519 mg, 13.0 mmol) dissolved in water (1 ml) was added and the reaction mixture was extracted twice with DCM. The combined organic extracts were washed with brine, dried, concentrated under reduced pressure and purified by column chromatography (amino phase functionalized silica gel, hexane / EtOAc 40-100%) to yield 480 mg (92% purity, 8% yield) of the title compound.

LC-MS (method 1): $R_t$ = 1.24 min; MS (ESIpos): m/z = 830 $[M+H]^+$

**Cyclen intermediate 5-2**

**(2*S*)-2-(4-{2-[2-(2-ethoxyethoxy)ethoxy]ethoxy}benzyl)-1,4,7,10-tetraazacyclododecan**

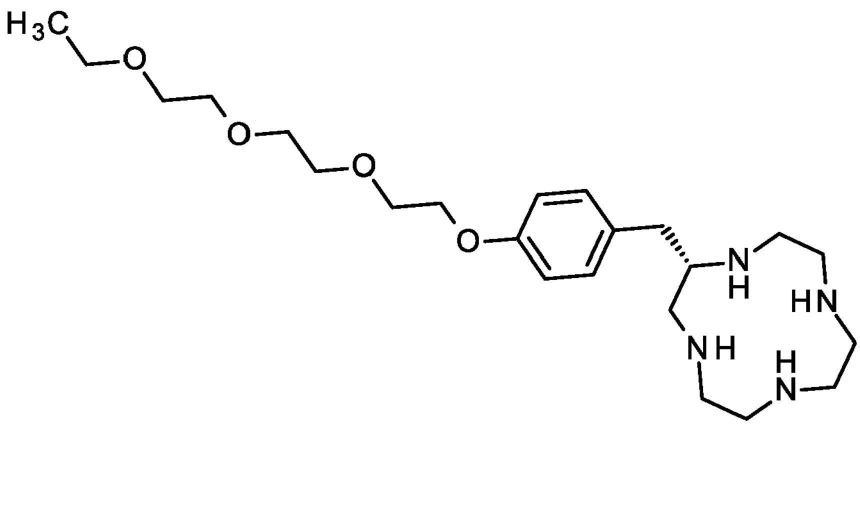

To (2*S*)-1,7,10-tribenzyl-2-(4-{2-[2-(2-ethoxyethoxy)ethoxy]ethoxy}benzyl)-4-(4-methoxy benzyl)-1,4,7,10-tetraazacyclododecane (480 mg, 579 μmol) dissolved in EtOH (6.1 ml) and acetic acid (6.0 ml) was added palladium on activated carbon (92,4 mg, 10% purity, 86,8 μmol) and the mixture was stirred under hydrogen pressure (40 bar) at 80°C for 42 h. The catalyst was removed by filtration and washed with EtOH and THF. The filtrate was concentrated under reduced pressure to yield 535 mg (82% purity, quantitative) of the title compound.

LC-MS (method 1): $R_t$ = 0.58 min; MS (ESIpos): m/z = 439 $[M+H]^+$.

**Cyclen intermediate 5-3**

**tetra-tert-buty)-2,2',2",2'''-[(2*S*)-2-(4-{2-[2-(2-ethoxyethoxy)ethoxy]ethoxy}benzy))-1,4,7,10-tetraazacyclododecane-1,4,7,10-tetrayl]tetraacetate**

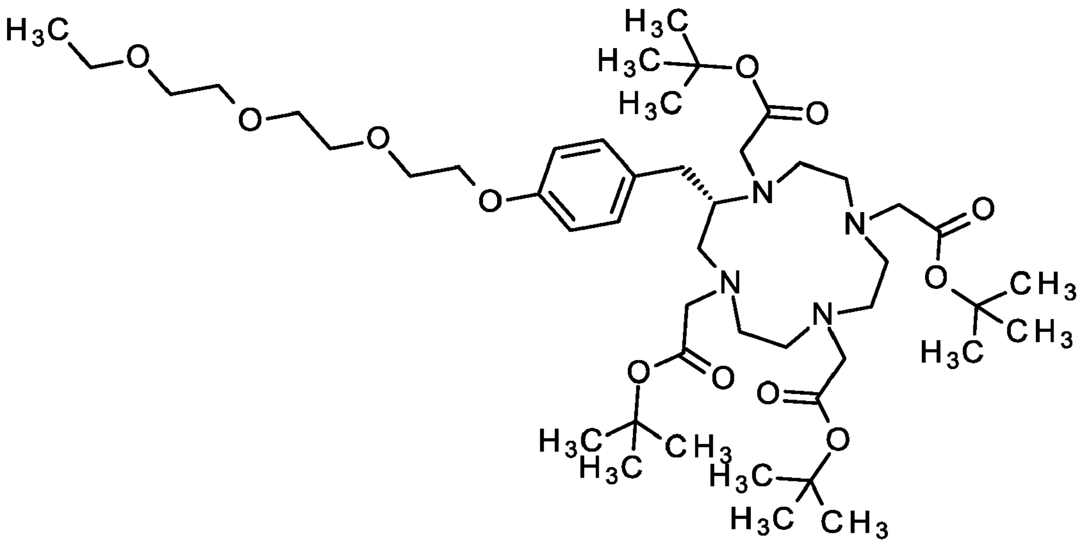

To (2S)-2-(4-{2-[2-(2-ethoxyethoxy)ethoxy]ethoxy}benzyl)-1,4,7,10-tetraazacyclododecane (535 mg, 1.22 mmol) dissolved in acetonitrile (16 ml) was added potassium carbonate (1.69 g, 12.2 mmol) and stirred for 1h at 50°C. *Tert-butyl* bromoacetate (990 μl, 6.7 mmol) was added and stirred at RT for 90h. *Tert-butyl* methyl ether and water were added and phases were separated. The aqueous phase was extracted with *tert*-butyl methyl ether. The combined organic extracts were washed with aqueous sodium hydroxide solution (1M) and brine, dried and concentrated under reduced pressure to yield 568 mg of (82% purity, 43% yield) of the title compound.

LC-MS (method 1): $R_t$ = 1.25 min; MS (ESIpos): m/z = 895 $[M+H]^+$

**Free ligand 5**

**2,2',2",2'''-[(2*S*)-2-(4-(2-[2-(2-ethoxyethoxy)ethoxy]ethoxy)benzyl)-1,4,7,10-tetraazacyclododecane-1,4,7,10-tetrayl]tetraacetic acid**

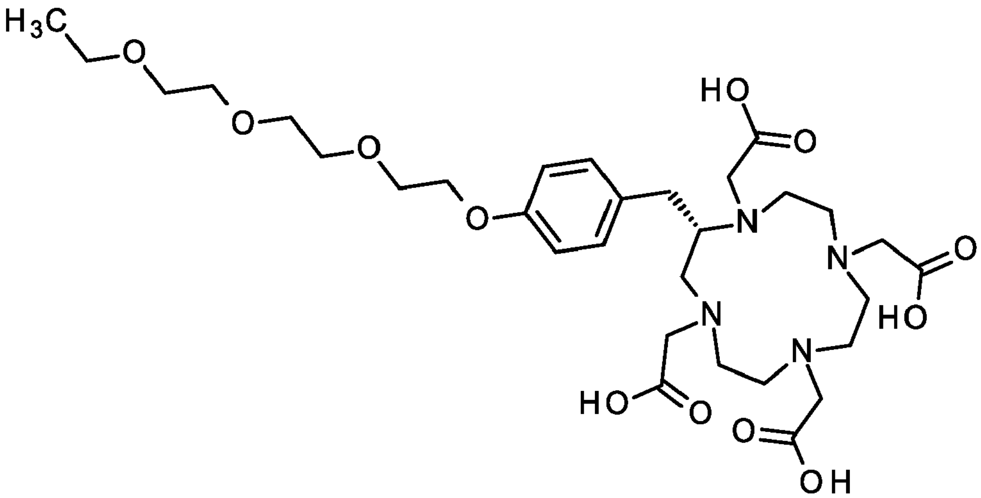

Tetra-tert-butyl-2,2',2'',2'''-[(2S)-2-(4-{2-[2-(2-ethoxyethoxy)ethoxy]ethoxy}benzyl)-1,4,7,10-tetraazacyclododecane-1,4,7,10-tetrayl]tetraacetate (568 mg, 634 μmol) was dissolved in formic acid (8.1 ml, 220 mmol) and stirred for 24h at 70°C. The reaction mixture was concentrated under reduced pressure and co-distilled twice with toluene/EtOH to yield 543 mg (89% purity, quantitative) of the title compound.

LC-MS (method 1): $R_t$ = 0.67 min; MS (ESIpos): m/z = 671 $[M+H]^+$

**Intermediate 6-1**

**methyl *N*-(*tert*-butoxycarbonyl)-3-(5-hydroxypyridin-2-yl)-L-alaninate**

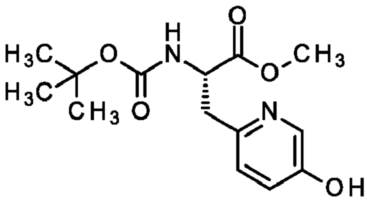

To a mixture of zinc (51.3 g, 784 mmol) in *N*,*N*-dimethylformamide (860 ml) was added iodine (9.95 g, 39.2 mmol) under nitrogen. After the reaction solution turned colorless, methyl *N*-(*tert*-butoxycarbonyl)-3-iodo-L-alaninate (86.0 g, 261 mmol) was added to the mixture. After stirring at room temperature for 1.5 hours under nitrogen. 6-bromopyridin-3-ol (CAS: 55717-45-8, 59.1 g, 340 mmol) and bis(triphenylphosphine)palladium(II)dichloride (9.17 g, 13.1 mmol) were added to above mixture. After stirring at 70 °C for 16 hours under nitrogen, the mixture was filtered. The filtrate was diluted with ethyl acetate and water. After separated, the aqueous phase was extracted with ethyl acetate. The combined organic phase was washed with brine, dried over anhydrous sodium sulfate, filtered and concentrated to give a residue. The residue was purified by column chromatography (100-200 mesh, petroleum ether: ethyl acetate = 2: 1, then 1: 1.5) to give methyl *N*-(tert-butoxycarbonyl)-3-(5-hydroxypyridin-2-yl)-L-alaninate (17.3 g, 22% yield) as yellow oil.

LC-MS (method 3): $R_t$ = 0.36 min; MS (ESIpos): m/z = 241.0 $[M-56+H]^+$

**Intermediate 6-2**

**2-(2-ethoxyethoxy)ethyl 4-methylbenzene-1-sulfonate**

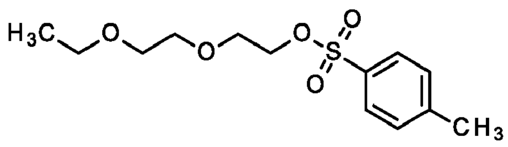

LC-MS (method 3): $R_t$ = 0.829 min; MS (ESIpos): m/z = 289.0 $[M+H]^+$.

## Intermediate 6-3

**methyl *N*-(tert-butoxycarbonyl)-3-{5-[2-(2-ethoxyethoxy)ethoxy]pyridin-2-yl}-L-alaninate**

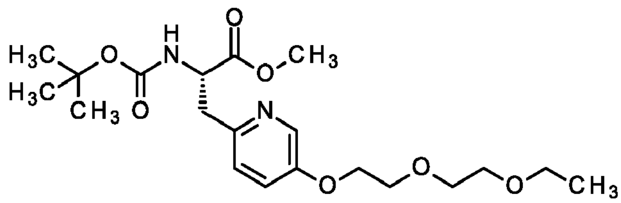

To a solution of methyl *N*-(*tert*-butoxycarbonyl)-3-(5-hydroxypyridin-2-yl)-L-alaninate (Intermediate 6-1, 17.3 g, 58.4 mmol) and 2-(2-ethoxyethoxy)ethyl 4-methylbenzene-1-sulfonate (Intermediate 6-2, 23.6 g, 81.7 mmol) in DMF (200 ml) was added potassium carbonate (16.1 g, 117 mmol) and sodium iodide (875 mg, 5.84 mmol) at room temperature. The mixture was stirred at 80 °C for 16 hours under nitrogen. The mixture was diluted with ethyl acetate and water. After separated, the aqueous phase was extracted with ethyl acetate.

The combined organic phase was washed with brine, dried over anhydrous sodium sulfate filtered and concentrated to give a residue. The residue was purified by flash column chromatography (petroleum ether / ethyl acetate, 1:0 to 1:1) to give methyl *N*-(tert-butoxycarbonyl)-3-{5-[2-(2-ethoxyethoxy)ethoxy] pyridin-2-yl}-L-alaninate (16.7 g, 69% yield) as green oil.

LC-MS (method 3): $R_t$ = 0.738 min; MS (ESIpos): m/z = 413.2 $[M+H]^+$.

## Intermediate 6-4

***tert*-butyl [(2*S*)-1-{5-[2-(2-ethoxyethoxy)ethoxy]pyridin-2-yl)-3-hydroxypropan-2-yl] carbamate**

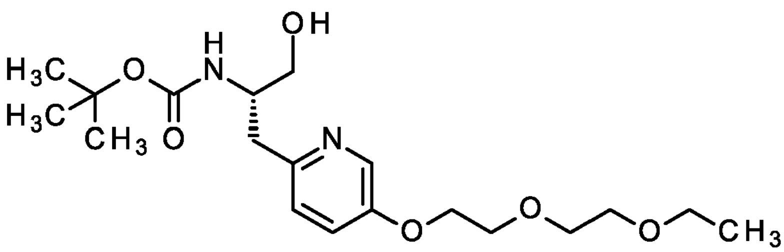

LC-MS (method 3): $R_t$ = 0.663 min; MS (ESIpos): m/z = 385.1 $[M+H]^+$.

## Intermediate 6-5

**(2*S*)-2-amino-3-{5-[2-(2-ethoxyethoxy)ethoxy]pyridin-2-yl}propan-1-ol - hydrogen chloride (1/1)**

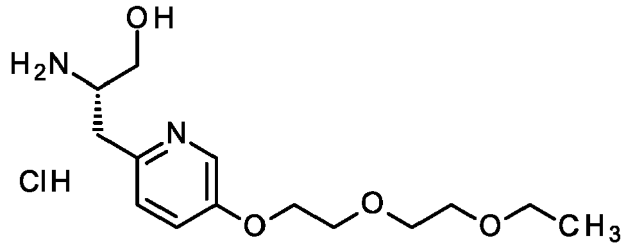

To a solution of *tert*-butyl [(2S)-1-{5-[2-(2-ethoxyethoxy)ethoxy]pyridin-2-yl}-3-hydroxypropan-2-yl]carbamate (Intermediate 6-4, 16.7 g, 43.4 mmol) in ethyl acetate (30 ml) was added hydrochloric acid (100 ml, 4.0 M in ethyl acetate, 400 mmol) at room temperature. The mixture was stirred at room temperature for 1 hour. The mixture was concentrated to give (2*S*)-2-amino-3-{5-[2-(2-ethoxyethoxy)ethoxy]pyridin-2-yl}propan-1-ol hydrochloride (1:1) (16.0 g) as brown oil.

LC-MS (Method 4): $R_t$ = 0.421 min; MS (ESIpos): m/z = 285.1 $[M+H]^+$.

__Intermediate 6-6__

**(2*S*)-3-{5-[2-(2-ethoxyethoxy)ethoxy]pyridin-2-yl}-2-{[(4-methoxyphenyl)methyl] amino}propan-1-ol**

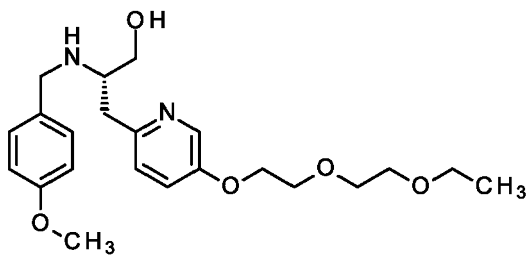

To a solution of (2*S*)-2-amino-3-{5-[2-(2-ethoxyethoxy)ethoxy]pyridin-2-yl}propan-1-ol - hydrogen chloride (1/1) (Intermediate 6-5, 16.0 g, 49.9 mmol) in methanol (160 ml) was added sodium acetate (4.09 g, 49.9 mmol) at room temperature. After stirring for 0.5 hour, acetic acid (15 ml), 4-methoxybenzaldehyde (6.1 ml, 50 mmol) and sodium cyanoborohydride (4.70 g, 74.8 mmol) were added to above mixture at room temperature. The mixture was stirred at 50°C for 16 hours under nitrogen. The mixture was quenched with saturated sodium bicarbonate solution, and then concentrated to give a residue. The residue was diluted with water, and then extracted with ethyl acetate. The combined organic phase was washed with brine, dried over anhydrous sodium sulphate filtered and concentrated to give a residue. The residue was purified by reverse phase column chromatography (Instrument: Biotage Isolera One; Column: I.D.57mm*H235mm, Welch Ultimate XB_C18 20-40 μm; 120 Å; eluent A: water (0.1% formic acid), eluent B: acetonitrile; gradient: 5%-21%,14min; 21%,13min; flow: 100 ml/min; temperature: room temperature; Detector: UV 220/254 nm) to give (2S)-3-{5-[2-(2-ethoxyethoxy)ethoxy]pyridin-2-yl}-2-[(4-methoxybenzyl)amino]propan-1-ol (9.1 g, 45% yield) as yellow oil.

LC-MS (Method 3): $R_t$ = 0.711 min; MS (ESIpos): m/z = 405.1 $[M+H]^+$.

__Intermediate 6-7__

**5-[2-(2-ethoxyethoxy)ethoxy]-2-({(2*S*)-1-[(4-methoxyphenyl)methyl]aziridine-2-yl}methyl)pyridine**

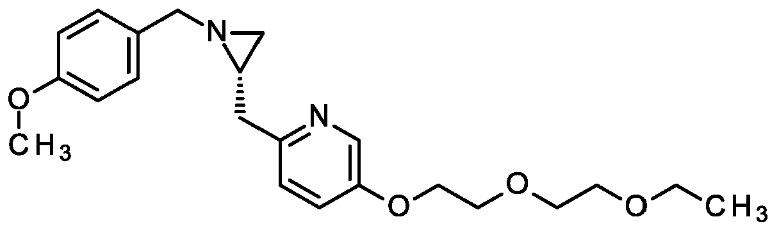

To a solution of (2*S*)-3-{5-[2-(2-ethoxyethoxy)ethoxy]pyridin-2-yl}-2-{[(4-methoxyphenyl)methyl]amino}propan-1-ol (Intermediate 6-6, 8.10 g, 20.0 mmol,) in tetrahydrofuran (80 ml) were added tert-n-butyl phosphine (7.5 ml, 30 mmol) and (E)-N1,N1,N2,N2-tetramethyldiazene-1,2-dicarboxamide (CAS: 10465-78-8, 5.17 g, 30.0 mmol) at room temperature. The mixture was stirred at room temperature for 16 hours under nitrogen. The mixture was stirred at 60 °C for 16 hours under nitrogen. The mixture was filtered. The filtrate was concentrated to give a residue. The residue was purified by flash column chromatography (silica gel, hexane ethyl acetate = 1: 0 to 1: 4) to give 6 g crude product. The crude product was purified by reversed phase column chromatography (Instrument: Agela-OCTOPUS; Column: Welch Ultimate XB_C18 I.D.95mm*H365mm,20/40 μm; 100 Å; eluent A: water (0.1% formic acid), eluent B: acetonitrile; gradient: 10%-40%,28min;40%, 10min; flow 200 ml/min; temperature: room temperature; Detector: UV 220/254 nm) to give 3.5 g crude product. The crude product was purified by flash column chromatography (100-200 mesh, petroleum ether: ethyl acetate = 1: 0 to 1: 4) to give 5-[2-(2-ethoxyethoxy)ethoxy]-2-{[(2S)-1-(4-methoxybenzyl)aziridine-2-yl]methyl}pyridine (1.0 g, 95% purity, 12% yield) as yellow oil.

LC-MS (Method 3): $R_t$ = 0.690 min; MS (ESIpos): m/z = 387.1 $[M+H]^+$.

$^1H$ NMR (400 MHz, DMSO-$d_6$): δ [ppm] = 8.15 (d, 1H), 7.22 (dd, 1H), 7.17-7.07 (m, 3H), 6.83-6.77 (m, 2H), 4.15-4.07 (m, 2H), 3.78-3.69 (m, 5H), 3.62-3.55 (m, 2H), 3.52-3.47 (m, 2H), 3.42 (q, 2H), 3.32-3.20 (m, 2H), 2.74-2.59 (m, 2H), 1.88-1.78 (m, 1H), 1.57 (d, 1H), 1.38 (d, 1H), 1.09 (t, 3H).

<u>**Cvclen Intermediate 6-1**</u>

**(2*S*)-1,7,10-tribenzyl-2-({5-[2-(2-ethoxyethoxy)ethoxy]pyridin-2-yl}methyl)-4-[(4-methoxyphenyl)methyl]-1,4,7,10-tetraazacyclododecane**

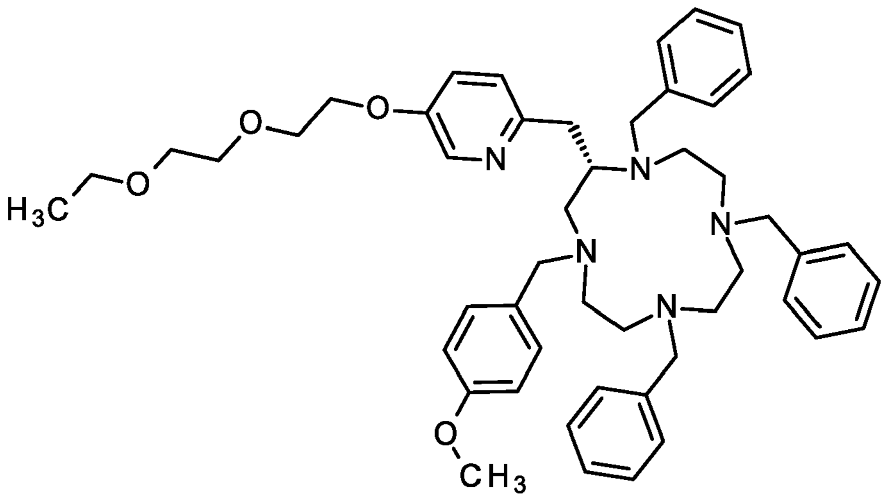

To a solution of 5-[2-(2-ethoxyethoxy)ethoxy]-2-({(2S)-1-[(4-methoxyphenyl)methyl]aziridine-2-yl}methyl)pyridine (1.00 g, 2.59 mmol) and 1-benzylaziridine (16 ml, 0.65 M, 10 mmol) (emulsion in water 0,086 g/ml) dissolved in ethanol (14 ml) was added p-toluenesulfonic acid (615 mg, 3.23 mmol) dissolved in water (310 μl) over 8h slowly at 60°C, followed by stirring at 60°C for 7h and reflux for 2h. Sodium hydroxide (192 mg) dissolved in water (310 μL) was added (pH13). A white precipitate was formed. The mixture was extracted 2 times with DCM. The organic layers were washed with brine and dried over water repellent filter and purified by column chromatography amino phase (28 g) hexane/EtOAc (20-80% EtOAc) to yield 385 mg (82% purity, 16% yield) of the title compound.

LC-MS (method 1): $R_t$ = 1.20 min; MS (ESIpos): m/z = 786 $[M+H]^+$.

**Cyclen Intermediate 6-2**

**(2*S*)-2-({5-[2-(2-ethoxyethoxy)ethoxy]pyridin-2-yl)methyl)-1,4,7,10-tetraaza cyclododecane**

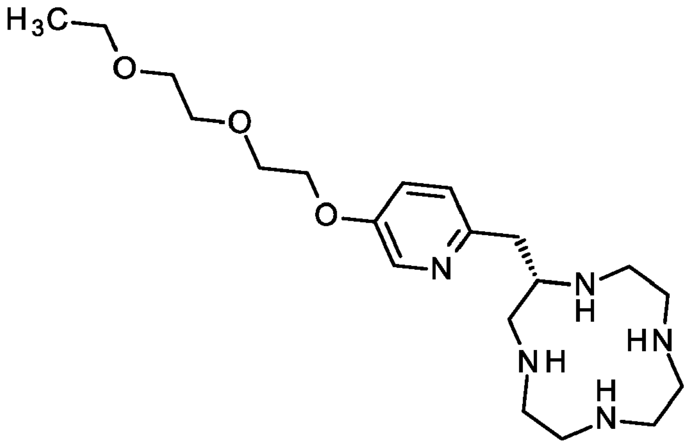

To (2*S*)-1,7,10-tribenzyl-2-({5-[2-(2-ethoxyethoxy)ethoxy]pyridin-2-yl}methyl)-4-[(4-methoxy phenyl)methyl]-1,4,7,10-tetraazacyclododecane (385 mg, 490 µmol) dissolved in EtOH (5,2 ml) and acetic acid (5,0 ml) was added palladium on activated carbon (93.8 mg, 10% purity, 88.2 µmol) and stirred under hydrogen pressure (40 bar) at 80°C for 54 h. The catalyst was removed by filtration and washed with EtOH and THF. The filtrate was concentrated under reduced pressure to yield 548 mg (28% purity, 79% yield) of the title compound.

LC-MS (method 1): $R_t$ = 0.48 min; MS (ESIpos): m/z = 396 $[M+H]^+$.

**Cyclen Intermediate 6-3**

**tetra-tert-butyl 2,2',2",2'''-[(2*S*)-2-({5-[2-(2-ethoxyethoxy)ethoxy]pyridin-2-yl}methyl)-1,4,7,10-tetraazacyclododecane-1,4,7,10-tetrayl]tetraacetate**

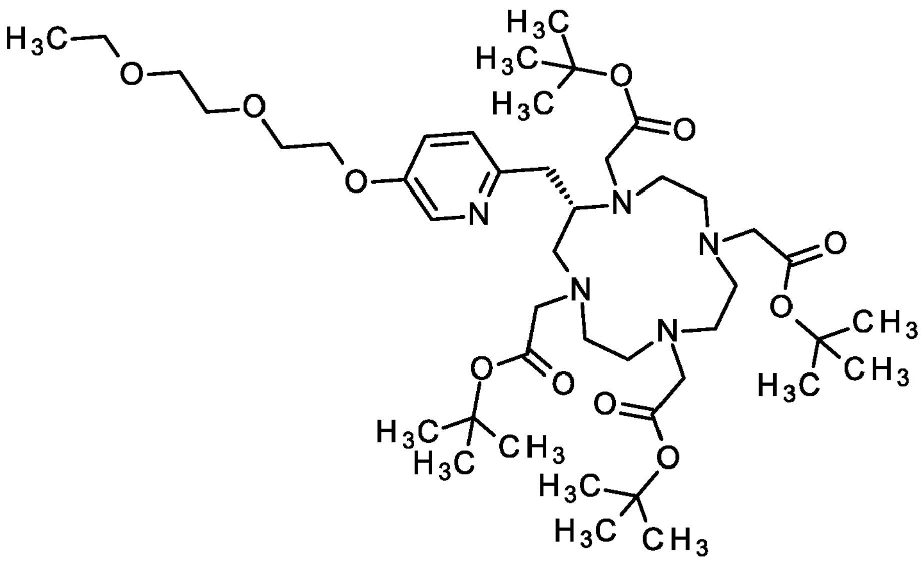

To (2S)-2-({5-[2-(2-ethoxyethoxy)ethoxy]pyridin-2-yl}methyl)-1,4,7,10-tetraazacyclododecane (548 mg, 1.39 mmol) dissolved in acetonitrile (18 ml) was added potassium carbonate (1.91 g, 13.9 mmol) stirred for 30 min at 50°C. *Tert*-butyl bromacetate (1.4 ml, 9.1 mmol) was added and stirred at RT for 18h. *Tert*-butyl methyl ether and water were

added and the phases were separated. The aqueous phase was extracted with *tert*-butyl methyl ether. The combined organic extracts were washed with aqueous sodium hydroxide solution (1M) and brine, dried and concentrated under reduced pressure to yield 1,35 g of (11% purity, 13% yield) of the title compound.

LC-MS (method 1): $R_t$ = 1.28 min; MS (ESIneg): m/z = 852 $[M+H]^-$

## Free ligand 6

**2,2',2",2'''-[(2*S*)-2-({5-[2-(2-ethoxyethoxy)ethoxy]pyridin-2-yl}methyl)-1 ,4,7,10-tetraazacyclododecane-1,4,7,10-tetrayl]tetraacetic acid**

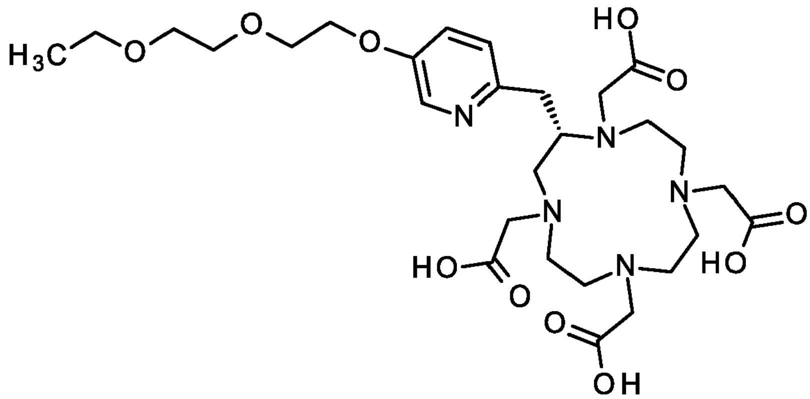

Tetra-*tert*-butyl 2,2',2",2'''-[(2*S*)-2-({5-[2-(2-ethoxyethoxy)ethoxy]pyridin-2-yl}methyl)-1,4,7,10-tetraazacyclododecane-1,4,7,10-tetrayl]tetraacetate (1.35 g, 1.58 mmol) was dissolved in formic acid (20 ml, 540 mmol) and stirred for 2 h at 70°C. The reaction mixture was concentrated under reduced pressure and co-distilled twice with toluene/EtOH to yield 1,09 g (14 % purity, 15 % yield) of the title compound,

LC-MS (method 1): $R_t$ = 0.56 min; MS (ESIpos): m/z = 628 $[M+H]^+$.

## Intermediate 7-1

**3-(4-ethoxyphenyl)-2-[(E)-(4-methoxybenzyliden)amino]propan-1-ol**

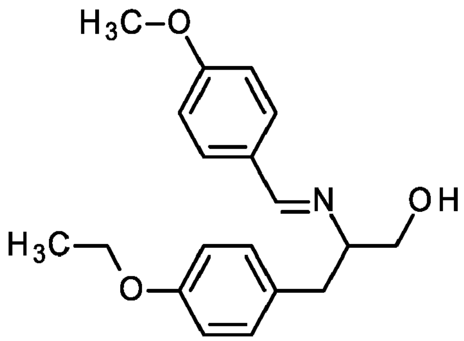

To a suspension of 2-amino-3-(4-ethoxyphenyl)propane-1-ol (15 g, 76.8 mmol) in toluene (230 ml) was added molecular sieves (0,4 nm, 7,68 g) and 4-methoxybenzaldehyd (9.3 ml, 77 mmol) and the mixture was stirred at 90°C for 1h. Molecular sieves was removed by filtration. The filtrate was concentrated under reduced pressure to yield 23.2 g (90% purity, 88% yield) of the title compound.

LC-MS (method 1): $R_t$ = 0.85 min; MS (ESIpos): m/z = 314 $[M+H]^+$.

$^1H$ NMR (400MHz, DMSO-$d_6$): δ [ppm]= 1.27 (t, 3H), 2.59 - 2.68 (m, 1H), 2.83 - 2.90 (m, 1H), 3.39 - 3.46 (m, 1H), 3.51 - 3.60 (m, 1H), 3.78 (s, 3H), 3.92 (d, 2H), 4.63 (t, 1H), 6.75 (d, 2H), 6.93 - 7.05 (dd, 4H), 7.61 (d, 2H), 7.93 - 7.95 (s, 1H).

**Intermediate 7-2**

**3-(4-ethoxyphenyl)-2-[(4-methoxybenzyl)amino]propan-1-ol**

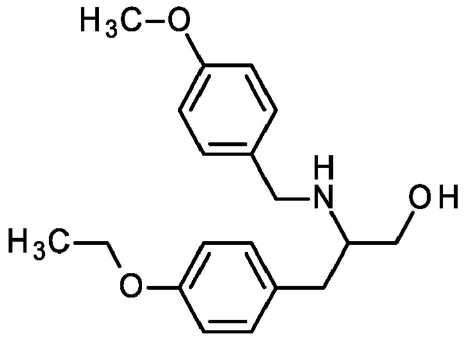

To a suspension of 3-(4-ethoxyphenyl)-2-[(*E*)-(4-methoxybenzyliden)amino]propan-1-ol (23.5 g, 75 mmol) in methanol (220 ml) was added at 0°C sodium borohydride (4.25 g, 112 mmol) in 8 portions while the mixture was stirred for 1h at 0°C. Acetone (37 ml) was added. The mixture was allowed to come to room temperature. The reaction mixture was filtered through celite and washed with methanol. The filtrate was concentrated under reduced pressure. The residue was dissolved in ethyl acetate and extracted with water. The water phase was extracted two times with ethyl acetate. The combined organic phases were concentrated under reduced pressure to yield 23,2 g (90% purity, 88% yield) of the title compound.

LC-MS (method 1): $R_t$ = 1,14 min; MS (ESIpos): m/z = 316 $[M+H]^+$.

$^1H$ NMR (400MHz, $CDCl_3$): $\delta$ [ppm]= 1.38 - 1.43 (t, 3H), 2.67 - 2.87 (m, 2H), 2.91 - 2.99 (m, 1H), 3.38 (dd, 1H), 3.63 - 3.67 (dd, 1H), 3.73 - 3.76 (d, 2H), 3.77 - 3.80 (s, 3H), 4.01 (q, 2H), 6.77 - 6.87 (dd, 4H), 7.02 - 7.11 (d, 2H), 7.13 - 7.22 (d, 2H).

**Intermediate 7-3**

**2-(4-ethoxybenzyl)-1-(4-methoxybenzyl)aziridine**

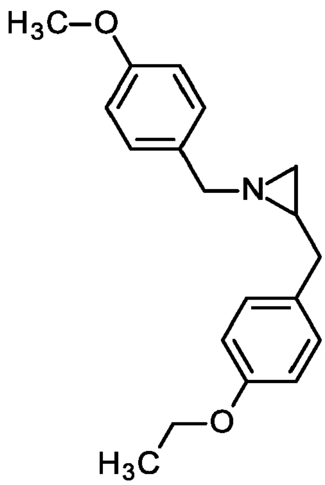

To a solution of 3-(4-ethoxyphenyl)-2-[(4-methoxybenzyl)amino]propan-1-ol (23.2 g, 73.4 mmol) in THF (290 ml) was added triphenylphosphine (27.0 g, 103 mmol) and di-*tert*-butyl-(*E*)-diazene-1,2-dicarboxylate (23.7 g, 103 mmol) at 0°C. The mixture was stirred for 22 h while it warmed to room temperature. 180ml of the solvent was removed under reduced pressure, hexane (200 ml) was added and the mixture was stirred for 1 h while a precipitate formed. The precipitate was removed by filtration. The filtrate was concentrated under reduced pressure and purified by column

chromatography (silica gel, hexane / EtOAc 30-50%) to yield 19,6 g (90% purity, 81% yield) of the title compound.

LC-MS (method 2): $R_t$ = 1.31 min; MS (ESIpos): m/z = 298 $[M+H]^+$.

$^1$H-NMR (400MHz, $CDCl_3$): $\delta$ [ppm]= 1.37 - 1.45 (m, 4H), 1.64 - 1.75 (m, 2H), 2.51 - 2.77 (dq, 2H), 3.23 - 3.50 (dd, 2H), 3.79 - 3.82 (s, 3H), 3.97 - 4.03 (q, 2H), 6.75 - 6.78 (d, 2H), 6,81-6,85 (d, 2H), 7.03 - 7.11 (d, 2H), 7.17 - 7.22 (d, 2H).

## Cyclen Intermediate 7-1

**1,7,10-tribenzyl-2-(4-ethoxybenzyl)-4-(4-methoxybenzyl)-1,4,7,10-tetraazacyclododecan**

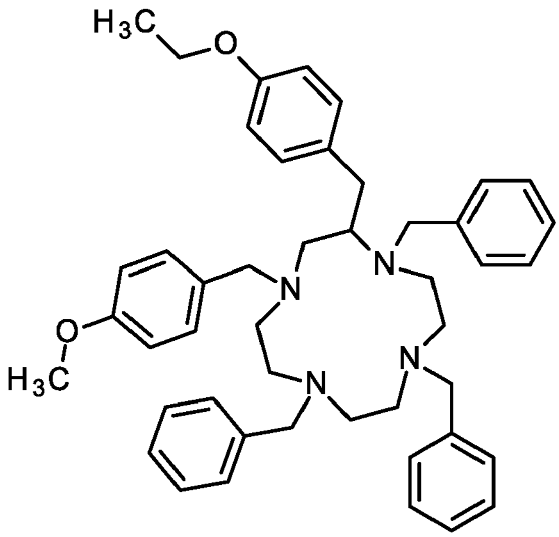

To a solution of 2-(4-ethoxybenzyl)-1-(4-methoxybenzyl)aziridine (10 g, 33.6 mmol) and 1-benzylaziridine (180 ml, 0.75 M, 130 mmol) (emulsion in water 0,1g/ml) dissolved in ethanol (180 ml) was slowly added p-toluenesulfonic acid (8.0 g, 42 mmol) in water (4 ml) over 8 h at 60°C, followed by additional stirring at 60°C for 7h and reflux for 2h. Sodium hydroxide (2.5 g) in water (5 mL) water was added (pH13). A white precipitate was formed. The mixture was extracted 2 times with DCM. The organic layers were washed with brine and dried over water repellent filter and purified by column chromatography amino phase (375g) hexane/EtOAc (0-30% EtOAc) to yield 3,00 g (90% purity, 12% yield) of the title compound.

$^1$H NMR (400 MHz, $CDCl_3$) $\delta$ [ppm]: 1.376 (1.77), 1.395 (4.03), 1.413 (2.08), 1.518 (1.32), 1.536 (2.68), 1.553 (1.39), 2.181 (7.51), 2.618 (0.44), 2.696 (0.49), 2.729 (0.41), 2.791 (3.69), 2.822 (1.85), 2.879 (0.50), 3.008 (0.58), 3.041 (0.51), 3.091 (0.56), 3.501 (0.45), 3.530 (0.79), 3.568 (3.38), 3.592 (0.75), 3.607 (0.51), 3.819 (0.67), 3.853 (0.55), 3.884 (7.17), 4.096 (0.48), 4.113 (1.35), 4.131 (1.33), 4.148 (0.48), 4.230 (0.51), 4.248 (1.51), 4.266 (1.54), 4.283 (0.51), 4.731 (0.90), 6.849 (0.77), 6.869 (1.38), 6.890 (0.72), 7.050 (0.57), 7.069 (0.54), 7.319 (0.64), 7.333 (1.40), 7.339 (1.41), 7.350 (3.27), 7.362 (3.28), 7.369 (1.91), 7.376 (2.52), 7.381 (2.31), 7.394 (16.00), 7.413 (1.33), 7.434 (1.03), 7.499 (1.32), 7.515 (0.52), 7.519 (0.62), 7.522 (0.46).

## Cvclen Intermediate 7-2

**2-(4-ethoxybenzyl)-1,4,7,10-tetraazacyclododecan**

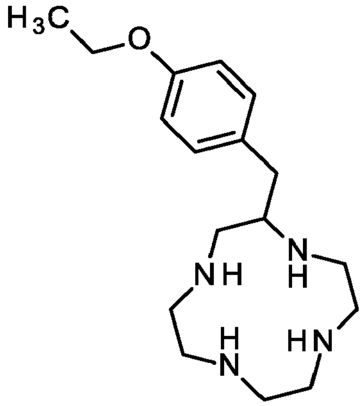

To 1,7,10-tribenzyl-2-(4-ethoxybenzyl)-4-(4-methoxybenzyl)-1,4,7,10-tetraazacyclo dodecane (2.0 g, 2.87 mmol) dissolved in EtOH (47 ml) and THF (16 ml) was added palladium on activated carbon (397 mg, 10 % purity, 373 μmol) and the mixture was stirred under hydrogen pressure (40 bar) at 80°C for 34 h. The catalyst was removed by filtration and washed with EtOH and THF. The filtrate was concentrated under reduced pressure to yield 870 mg (70% purity, 693% yield) of the title compound.

LC-MS (method 1): $R_t$ = 0.66 min; MS (ESIpos): m/z = 307 $[M+H]^+$.

**Cvclen Intermediate 7-3**

**tetra-*tert*-butyl-2,2',2",2'''-[2-(4-ethoxybenzyl)-1,4,7,10-tetraazacyclododecane-1,4,7,10-tetrayl]tetraacetate**

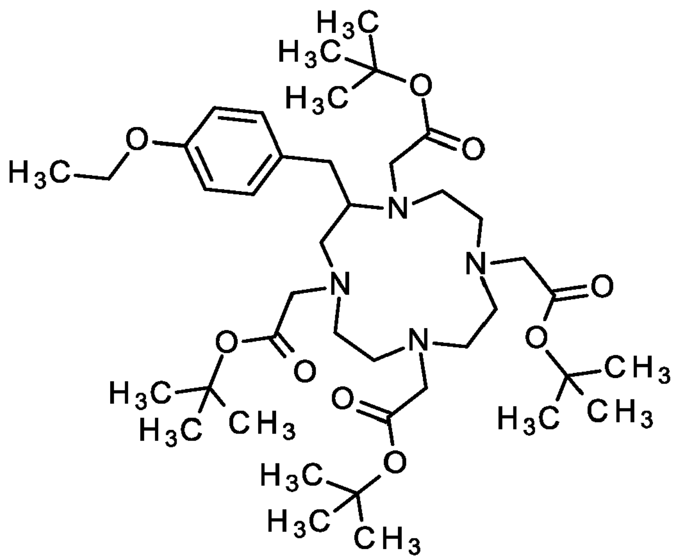

To 2-(4-ethoxybenzyl)-1,4,7,10-tetraazacyclododecane (1.04 g, 3.39 mmol) dissolved in acetonitrile (45 ml) was added potassium carbonate (2.35 g, 17 mmol) and *tert*-butyl bromacetate (2,21 ml, 14,9 mmol) and the mixture was stirred at RT for 40 h. *Tert*-butyl methyl ether and water were added and the phases were separated. The aqueous phase was extracted with *tert*-butyl methyl ether. The combined organic extract was washed with aqueous sodium hydroxide solution (1M) and brine, dried and concentrated under reduced pressure to yield 780 mg of (80% purity, 24% yield) of the title compound.

LC-MS (method 1): $R_t$ = 1.35 min; MS (ESIpos): m/z = 763 $[M+H]^+$

Free ligand 7

**2,2',2",2'''-[2-(4-ethoxybenzyl)-1,4,7,10-tetraazacyclododecane-1,4,7,10-tetrayl]tetraacetic acid**

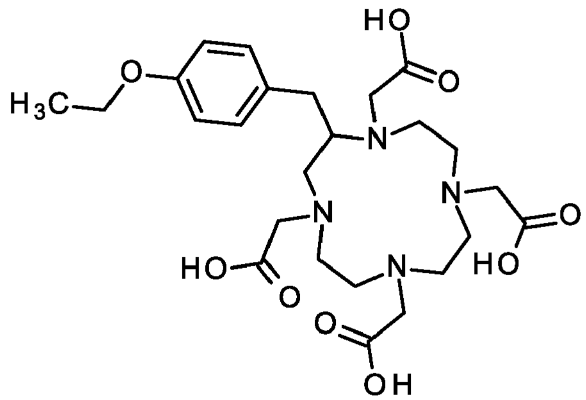

Tetra-tert-butyl-2,2',2",2'''-[2-(4-ethoxybenzyl)-1,4,7,10-tetraazacyclododecane-1,4,7,10-tetrayl]tetraacetate (780 mg, 1.02 mmol) was dissolved in formic acid (13 ml, 350 mmol) and stirred for 18h at 70°C. The reaction mixture was concentrated under reduced pressure and co-distilled twice with toluene/EtOH to yield 610 mg (85 % purity, 94 % yield) of the title compound,

LC-MS (method 1): $R_t$ = 0.60 min; MS (ESIneg): m/z = 537 $[M-H]^-$.

Intermediate 8-1

**ethyl-*N*-(tert-butoxycarbonyl)-*O*-butyl-L-tyrosinate**

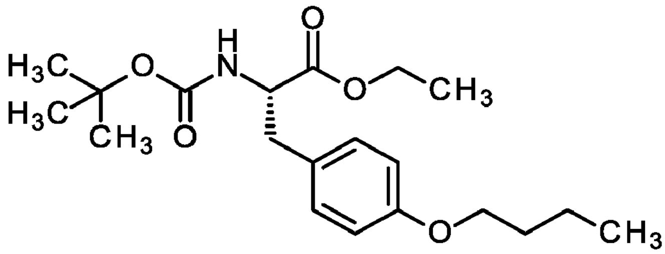

To a solution of ethyl-*N*-(*tert*-butoxycarbonyl)-L-tyrosinate (5.0 g, 16.2 mmol) in *N*,*N*-dimethyl formamide (13 ml) were added potassium carbonate (2.46 g, 17.8 mmol) and 1-iodbutane (2,2 ml, 19 mmol) at room temperature and the mixture was stirred at 40°C for 20 h. 1-lodbutane (0,92 ml, 8 mmol) was added and stirring was continued for 4 h at 40°C. Ethyl acetate and water were added and layers were separated. The aqueous phase was extracted 2 times with ethyl acetate. The combined organic extract was washed with brine, dried by filtration through a water repellent filter, concentrated under reduced pressure and purified by column chromatography (silica gel 50 g, hexane / EtOAc, 40-100%) to yield 5.21 g (95% purity, 84% yield) of the title compound

LC-MS (method 1): $R_t$ = 1.51 min; MS (ESIpos): m/z = 366 $[M+H]^+$

$^1H$ NMR (400MHz, $CDCl_3$): δ [ppm]= 0.97 (t, 3H), 1.24 (t, 3H), 1.42 (s, 9H), 1.44 - 1.52 (m, 2H), 1.70 - 1.79 (m, 2H), 2.93 - 3.09 (m, 2H), 3.93 (t, 2H), 4.09 - 4.24 (q, 2H), 4.45 - 4.55 (q, 1H), 4.95 (br d, 1H), 6.79 - 6.83 (m, 2H), 7.03 (d, 2H).

Intermediate 8-2

***tert*-Butyl-[(2S)-1-(4-butoxyphenyl)-3-hydroxypropan-2-yl]carbamate**

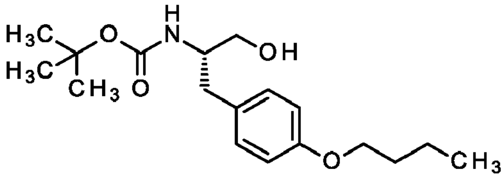

To a solution of ethyl-N-(tert-butoxycarbonyl)-O-butyl-L-tyrosinate (1000 mg, 2.74 mmol) in tetrahydrofuran (30 ml) was slowly added lithium aluminum hydride 1mol/L in THF (5.5 ml, 1.0 M, 5.5 mmol) at 0 °C and stirred at room temperature for 2 hours under nitrogen. The mixture was quenched with hydrochloric acid (2mol/L), extracted 2 times with ethyl acetate, dried by filtration through a water repellent filter and concentrated under reduced pressure to yield 970 mg (90% purity, 99% yield) of the title compound

1H-NMR (400MHz, $CDCl_3$): δ [ppm]= 0.97 (t, 3H), 1.26 (t, 1H), 1.40 - 1.44 (s, 9H), 1.45 - 1.54 (m, 2H), 1.71 - 1.79 (m, 2H), 2.05 (s, 1H), 2.76 (d, 2H), 3.51 - 3.58 (dd, 1H), 3.62 - 3.70 (dd, 1H), 3.81 (br s, 1H), 3.93 (t, 2H), 6.80 - 6.86 (m, 2H), 7.10 (d, 2H).

LC-MS (method 1): $R_t$ = 1.28 min; MS (ESIpos): m/z = 324 $[M+H]^+$

__Intermediate 8-3__

**(2S)-2-Amino-3-(4-butoxyphenyl)propan-1-ol**

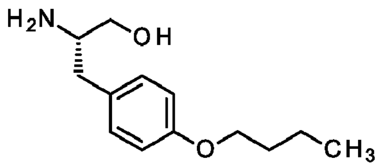

To a solution of *tert*-butyl-[(2*S*)-1-(4-butoxyphenyl)-3-hydroxypropan-2-yl]carbamate (970 mg, 3,00 mmol) in dichloromethane (10 ml) was added hydrochloric acid (4 N in dioxane) (3,7 ml, 15 mmol) at room temperature and stirred for 2 h. The mixture was concentrated under reduced pressure, diluted in DCM/EtOH 9:1 and extracted with a mixture of sodium bicarbonate solution and brine. The water phase was extracted 2 times with DCM/EtOH (9:1). The combined organic layers were dried by filtration through a water repellent filter and concentrated under reduced pressure to yield 640 mg (80% purity, 76% yield) of the title compound.

LC-MS (method 1): $R_t$ = 0.74 min; MS (ESIpos): m/z = 224 $[M+H]^+$

$^1$H-NMR (400MHz, $CDCl_3$): δ [ppm]= 0.97 (t, 3H), 1.39 - 1.44 (m, 1H), 1.44 - 1.58 (m, 2H), 1.69 - 1.80 (m, 2H), 2.35 (br s, 3H), 2.53 (dd, 1H), 2.70 - 2.77 (dd, 1H), 3.08 - 3.17 (m, 1H), 3.36 - 3.45 (dd, 1H), 3.64 (dd, 1H), 3.88 - 3.98 (t, 2H), 6.79 - 6.88 (m, 2H), 7.04 - 7.14 (m, 2H).

__Intermediate 8-4__

**(2*S*)-3-(4-butoxyphenyl)-2-[(*E*)-(4-methoxybenzyliden)amino]propan-1-ol**

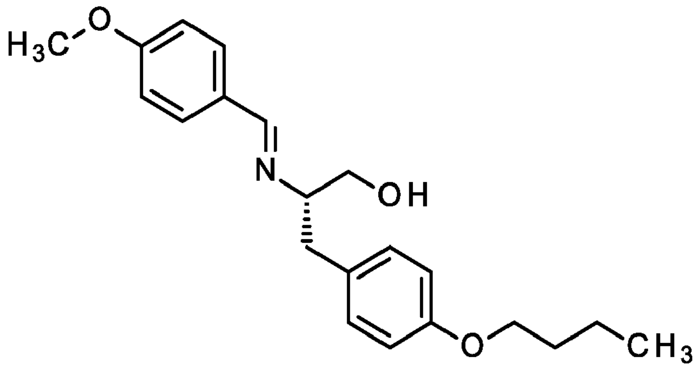

To a solution of (2*S*)-2-amino-3-(4-butoxyphenyl)propan-1-ol (640 mg, 2.87 mmol) in toluene (8,4ml) was added molecular sieve 0,4 nm (287mg) and 4-methoxybenzaldehyde (350 µl, 2.9 mmol) and stirred for 4 h at 90°C. Molecular sieve was removed by filtration, the filtrate was concentrated under reduced pressure to yield 1,02g (90% purity, 94% yield) of the title compound.

$^{1}$H NMR (400 MHz, DMSO-$d_6$): δ [ppm]= 0.90 (t, 3H), 1.39-1.45 (m, 2H), 1.59-1.68 (m, 2H), 2.63 (dd, 1H), 2.86 (dd, 1H), 3.39-3.46 (m, 1H), 3.52 - 3.58 (m, 1H), 3.78 (s, 3H), 3.87 (t, 2H), 4.62 (t, 1H), 6.75 (d, 2H), 6.96 (d, 2H), 7.02 (d, 2H), 7.61 (d, 2H), 7.95 (s, 1H).

**Intermediate 8-5**

**(2*S*)-3-(4-butoxyphenyl)-2-[(4-methoxybenzyl)amino]propan-1-ol**

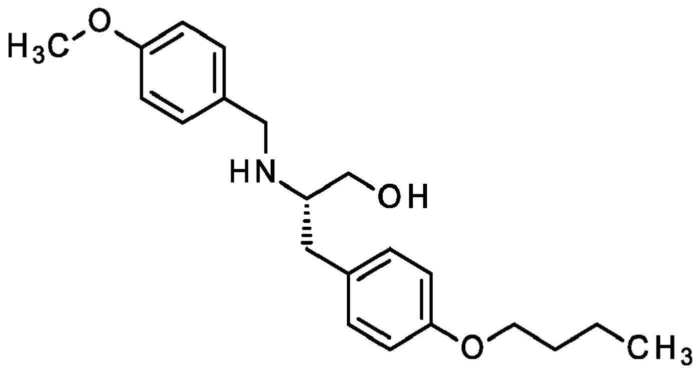

To a solution of (2*S*)-3-(4-butoxyphenyl)-2-[(*E*)-(4-methoxybenzyliden)amino]propan-1-ol (1.02 g, 2.99 mmol) in methanol (8,8 ml) was added sodium borohydride (170 mg, 4.48 mmol) in 3 portions at 0°C and stirred for 1h. Methanol was added to make the reaction mixture stirrable. Acetone (1.6 ml) was added and the mixture was warmed to room temperature. The mixture was filtered over Celite, washed with methanol and concentrated under reduced pressure. The residue was dissolved in ethyl acetate and extracted with water. The water phase was extracted two times with ethyl acetate. The combined organic phases were concentrated under reduced pressure to yield 810 mg (70% purity, 55% yield) of the title compound.

LC-MS (method 1): $R_t$ = 0.97 min; MS (ESIpos): m/z = 344 $[M+H]^+$

$^{1}$H NMR (400 MHz, $CDCl_3$) δ [ppm]: 0.965 (4.36), 0.977 (0.84), 0.984 (9.91), 0.995 (0.50), 1.002 (4.76), 1.177 (0.53), 1.269 (0.87), 1.441 (1.47), 1.471 (1.20), 1.477 (0.43), 1.490 (1.93), 1.495 (0.86), 1.509 (1.87), 1.513 (0.80), 1.528 (1.20), 1.734 (0.71), 1.750 (1.64), 1.766 (1.26), 1.770 (1.81), 1.780 (0.60), 1.787 (1.44), 1.804 (0.52), 2.055 (1.30), 2.087 (2.50), 2.089 (3.63), 2.717 (0.50), 2.735 (0.60), 2.751 (0.76), 2.768 (0.86), 2.793 (0.72), 2.810 (0.86), 2.950 (0.43), 2.959 (0.51), 2.964 (0.48), 2.973 (0.45), 3.373 (0.56), 3.387 (0.54), 3.401 (0.67), 3.414 (0.62), 3.645 (0.71), 3.654 (0.70), 3.673 (0.63), 3.682 (0.59), 3.746 (0.60), 3.760 (2.63), 3.768 (1.23), 3.791 (16.00), 3.801 (4.22), 3.809 (0.86), 3.812 (0.73), 3.814 (0.70), 3.822 (5.76), 3.827 (1.14), 3.931 (2.68), 3.948 (5.07), 3.964 (2.37), 4.634 (1.53), 5.049 (0.83), 6.810 (0.89), 6.820 (3.50), 6.826 (1.50), 6.831 (1.41), 6.838 (4.11), 6.841 (4.80), 6.848 (0.85), 6.855 (1.31), 6.860 (3.44), 6.866 (0.46), 6.891 (0.47), 6.896 (0.82), 6.912 (0.64), 6.917 (0.83), 7.037 (0.68), 7.045 (0.50), 7.052 (2.85), 7.058 (1.37), 7.074 (2.29), 7.122 (0.62), 7.143 (0.52), 7.177 (2.14), 7.199 (1.90), 7.297 (0.89), 7.319 (0.85).

Optical rotation:$[\alpha]_D$ = -5.03° +/- 0.3° (c = 5.4 mg/ml, 20°C, 589 nm, $CHCl_3$).

## Intermediate 8-6

**(2S)-2-(4-butoxybenzyl)-1-(4-methoxybenzyl)aziridine**

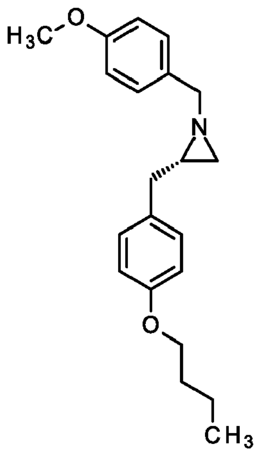

To a solution of (2*S*)-3-(4-Butoxyphenyl)-2-[(4-methoxybenzyl)amino]propan-1-ol (810 mg, 2.36 mmol) in THF (9.2 ml) was added triphenylphosphine (866 mg, 3.30 mmol) and di-*tert*-butyl-(*E*)-diazene-1,2-dicarboxylate (760 mg, 3.30 mmol) at 0°C. The mixture was than stirred 22 h while it warmed to room temperature. One half of the solvent was removed under reduced pressure, hexane (20 ml) was added and the mixture was stirred for 2 h while a precipitate formed. The precipitate was removed by filtration. The filtrate was concentrated under reduced pressure and purified by column chromatography (silica gel, hexane / EtOAc 0 - 40%) to yield 426 mg (85% purity, 47% yield) of the title compound.

LC-MS (method 2): $R_t$ = 1,48 min; MS (ESIpos): m/z = 326 $[M+H]^+$

$^1$H-NMR (400 MHz, $CDCl_3$): $\delta$ [ppm]= 0.97 (t, 3H), 1.40 - 1.54 (m, 3H), 1.63 - 1.79 (m, 4H), 2.55 (dd, 1H), 2.73 (dd, 1H), 3.35 (dd, 2H), 3.80 (s, 3H), 3.93 (t, 2H), 6.77 (d, 2H), 6.82 (d, 2H), 7.07 (d, 2H), 7.20 (d, 2H).

Optical rotation:$[\alpha]_D$ = -27.1° +/- 0.7° (c = 3.0 mg/ml, 20°C, 589 nm, $CHCl_3$).

## Cyclen Intermediate 8-1

**(2*S*)-1,7,10-tribenzyl-2-(4-butoxybenzyl)-4-(4-methoxybenzyl)-1,4,7,10-tetraazacyclododecan**

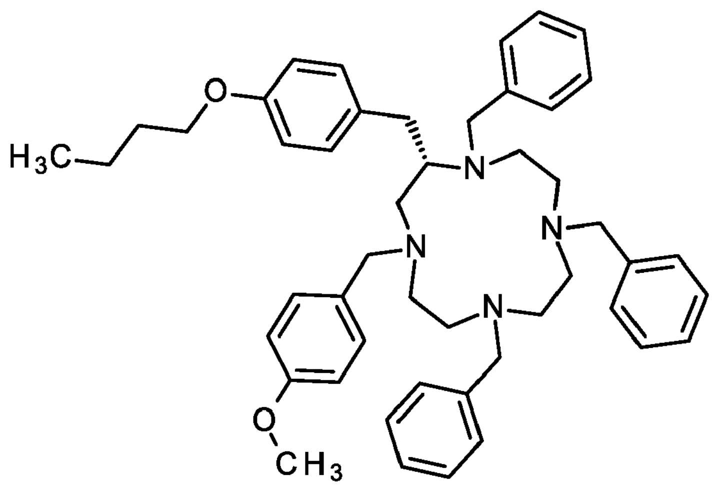

To a solution of (2S)-2-(4-Butoxybenzyl)-1-(4-methoxybenzyl)aziridine (1.85 g, 5.68 mmol) and 1-benzylaziridine (35 ml, 0.65 M, 23 mmol) (emulsion in water 0,087g/ml) dissolved in ethanol (30 ml) was added p-toluenesulfonic acid (1.35 g, 7.11 mmol) dissolved in water (680 μl) over 8h slowly at 60°C, followed by stirring at 60°C for 7h and reflux for 2h. Sodium hydroxide (423 mg) dissolved in 850 μL water was added (pH13). A white precipitate was formed. The mixture was extracted 2 times with DCM. The organic layers were washed with brine and dried over water repellent filter and purified by column chromatography amino phase functionalized silica gel 110g, hexane / EtOAc, 0-30%) to yield 455 mg (76% purity, 8% yield) of the title compound.

**Cyclen Intermediate 8-2**

**(2*S*)-2-(4-butoxybenzyl)-1,4,7,10-tetraazacyclododecan**

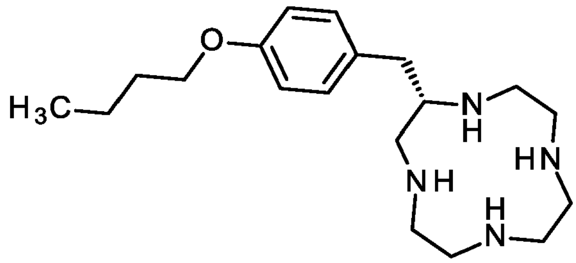

To (2*S*)-1,7,10-tribenzyl-2-(4-butoxybenzyl)-4-(4-methoxybenzyl)-1,4,7,10-tetraazacyclo dodecane (455 mg, 628 μmol) dissolved in EtOH (6.6 ml) and acetic acid (6,5 ml) was added palladium on activated carbon (86,8 mg, 10% purity, 81.6 μmol) and the mixture was stirred under hydrogen pressure (40 bar) at 80°C for 40 h. The catalyst was removed by filtration and washed with EtOH and THF. The filtrate was concentrated under reduced pressure to yield 387 mg (80% purity, quantitative) of the title compound.

LC-MS (method 1): $R_t$ = 0.74 min; MS (ESIpos): m/z = 335 $[M+H]^+$.

**Cyclen Intermediate 8-3 tetra-*tert*-butyl-2,2',2",2'''-[(2*S*)-2-(4-butoxybenzyl)-1,4,7,10-tetraazacyclododecane-1,4,7,10-tetrayl]tetraacetate**

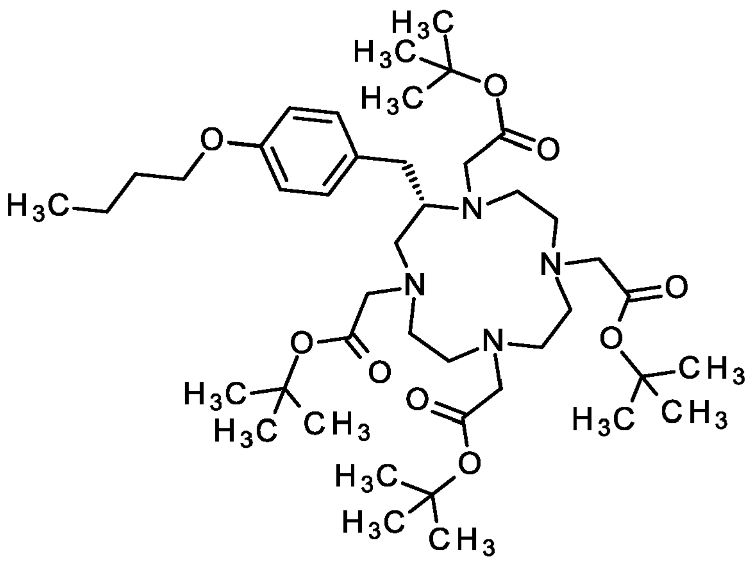

To (2S)-2-(4-butoxybenzyl)-1,4,7,10-tetraazacyclododecane (380 mg, 1.14 mmol) dissolved in acetonitrile (15 ml) was added potassium carbonate (1.57 g, 11.4 mmol) and the mixture was stirred for 30 min at 50°C. *Tert*-butyl bromoacetate (840 μl, 5.7 mmol) was added and stirring at RT was continued for 18h. *Tert*-butyl methyl ether and water

were added and the phases were separated. The aqueous phase was extracted with *tert*-butyl methyl ether. The combined organic extracts were washed with aqueous sodium hydroxide solution (1M) and brine, dried and concentrated under reduced pressure to yield 950 mg of (67% purity, 71% yield) of the title compound.

LC-MS (method 1): $R_t$ = 1.46 min; MS (ESIpos): m/z = 792 $[M+H]^+$.

**Free ligand 8**

**2,2',2",2'''-[(2S)-2-(4-butoxybenzyl)-1,4,7,10-tetraazacyclododecane-1,4,7,10-tetrayl]tetraacetic acid**

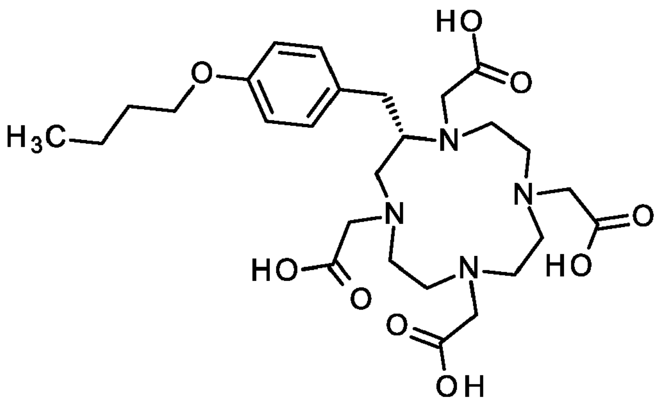

Tetra-*tert*-butyl-2,2',2",2'''-[(2*S*)-2-(4-butoxybenzyl)-1,4,7,10-tetraazacyclododecane-1,4,7,10-tetrayl]tetraacetate (950 mg, 1.20 mmol) was dissolved in formic acid (15 ml, 410 mmol) and stirred for 3 h at 70°C and 16 h at room temperature. The reaction mixture was concentrated under reduced pressure and co-distilled twice with toluene/EtOH to yield 652 mg (52% purity, 50% yield) of the title compound,

LC-MS (method 1): $R_t$ = 0.79 min; MS (ESIpos): m/z = 567 $[M+H]^+$.

**Synthesis of Examples**

**Example 1**

**Gadolinium-2,2',2"-[(2*S*)-10-(carboxymethyl)-2-(4-{2-[2-(2-methoxyethoxy)-ethoxy] ethoxy}benzyl)-1,4,7,10-tetraazacyclododecane-1,4,7-triyl]triacetate**

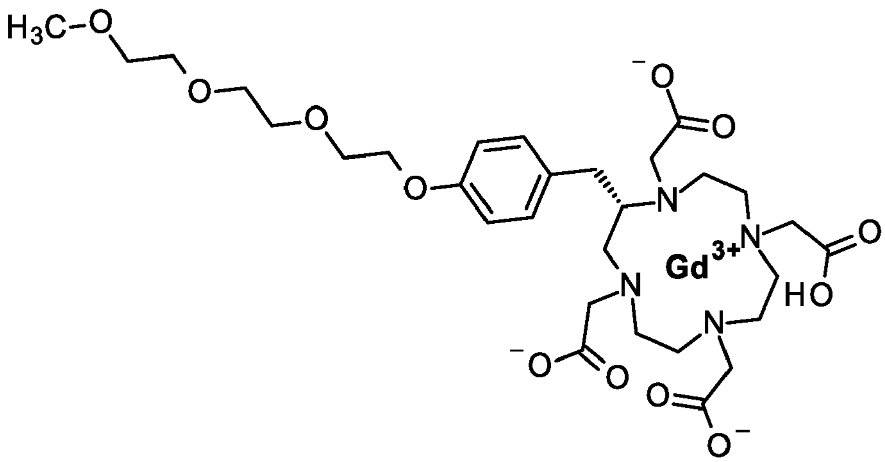

To 2,2',2",2'''-[(2*S*)-2-(4-{2-[2-(2-methoxyethoxy)ethoxy]ethoxy}benzyl)-1,4,7,10-tetraaza cyclododecane-1,4,7,10-tetrayl]tetraacetic acid (free ligand 1, 2.19 g, 3.33 mmol) suspended in water (54 ml) was added gadolinium

oxide (543mg, 1,50 mmol)and pH5 was adjusted by addition of aqueous sodium hydroxide (2 M) and stirred at 100°C for 2h. Chelex® was added and the mixture was stirred at RT for 21h, while the pH value was constantly adjusted to 5 by addition of hydrochloric acid (2 M). The mixture was filtered and purified by column chromatography (Biotage®Sfär C18 D, water / acetonitrile, 0-100%) to yield 50,9 mg (98% purity, 2% yield) of the title compound.

LC-MS (method 1): $R_t$ = 0.64 min; MS (ESIneg): m/z = 810 $[M-H]^-$.

## Example 2

**Gadolinium-2,2',2"-[(2*S*)-10-(carboxymethyl)-2-{4-[2-(2-ethoxyethoxy)ethoxy]benzyl}-1,4,7,10-tetraazacyclododecane-1,4,7-triyl]triacetate**

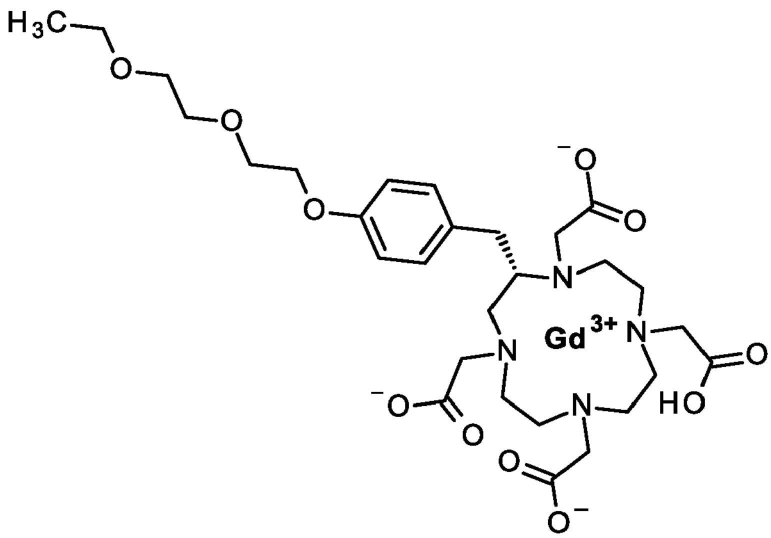

To a suspension of 2,2',2",2"'-[(2S)-2-{4-[2-(2-ethoxyethoxy)ethoxy]benzyl}-1,4,7,10-tetraaza cyclododecane-1,4,7,10-tetrayl]tetraacetic acid (free ligand 2, 760 mg, 1.21 mmol) in water (20 ml) was added gadolinium oxide (198 mg, 546 μmol). The pH was adjusted to pH 5 by addition of aqueous sodium hydroxide (2 M) and the mixture was stirred at 100°C for 2h. Chelex® was added and the mixture was stirred at RT for 16h. The mixture was filtered and purified by column chromatography (Biotage®Sfär C18 D, water / acetonitrile, 0-100%) to yield 31.9 mg (96% purity, 3% yield) of the title compound.

LC-MS (method 1): $R_t$ = 0.66 min; MS (ESIpos): m/z = 782 $[M+H]^+$.

## Example 3

**Gadolinium-2,2',2"-[(2*R*)-10-(carboxymethyl)-2-(4-(2-[2-(2-methoxyethoxy)ethoxy] ethoxy}benzyl)-1,4,7,10-tetraazacyclododecane-1,4,7-triyl]triacetate**

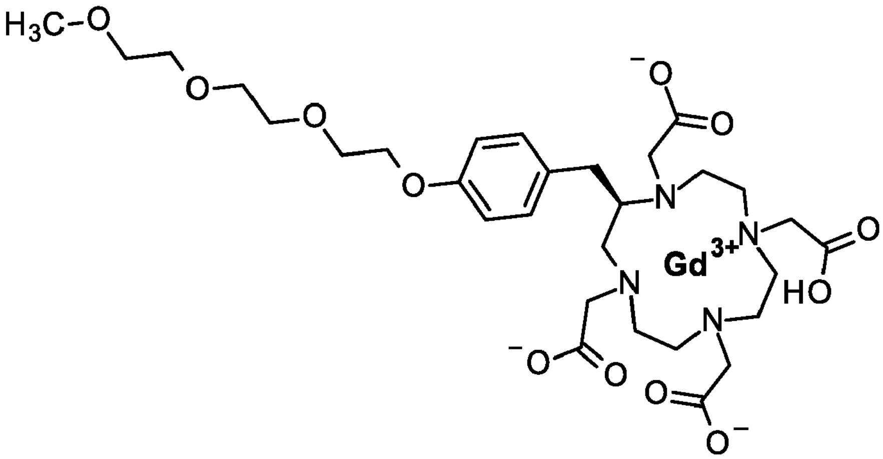

To 2,2',2'',2'''-[(2*R*)-2-(4-{2-[2-(2-methoxyethoxy)ethoxy]ethoxy}benzyl)-1,4,7,10-tetrazacyclo dodecane-1,4,7,10-tetrayl]tetra acetic acid (free ligand 3, 2.78 g, 4.23 mmol) in water (68 ml) was added gadolinium oxide (691 mg, 1.90 mmol) and pH was adjusted to pH5 by addition of aqueous sodium hydroxide (2 M) and the mixture was stirred at 100°C for 2 h. Chelex® was added and the mixture was stirred at RT for 16 h. The mixture was filtered and purified by column chromatography (Biotage®Sfär C18 D, water/ acetonitrile 0-100%) to yield 84 mg (96% purity, 3% yield) of the title compound.

LC-MS (method 1): $R_t$ = 0.61 min; MS (ESIpos): m/z = 812 $[M+H]^+$.

## Example 4

**Gadolinium-2,2',2''-{(2*S*)-10-(carboxymethy))-2-[4-(2-ethoxyethoxy)benzy)]-1,4,7,10-tetraazacyclododecane-1,4,7-triyl}triacetate**

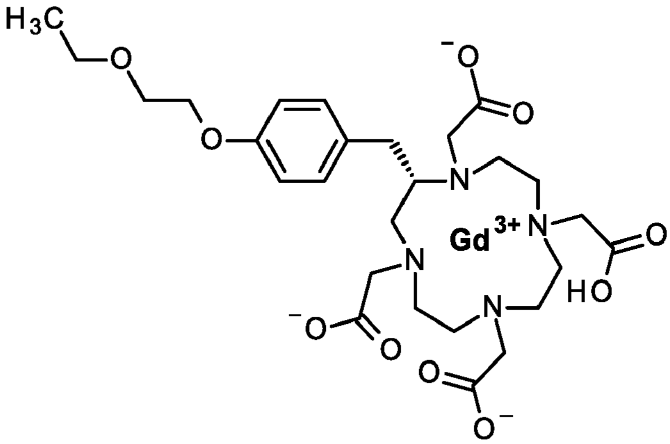

To 2,2',2'',2'''-{(2*S*)-2-[4-(2-ethoxyethoxy)benzyl]-1,4,7,10-tetraazacyclododecane-1,4,7,10-tetrayl}tetra acetic acid (free ligand 4, 1.09 g, 1.87 mmol) in water (30 ml) was added gadolinium oxide (305 mg, 842 µmol) and pH5 was adjusted by addition of aqueous sodium hydroxide (2 M) and stirred at 100°C for 1h. Chelex® was added and the mixture was stirred at RT for 16 h. The mixture was filtered and purified by column chromatography (Biotage®Sfär C18 D, water / acetonitrile, 0-100%) to yield 185 mg (95% purity, 13% yield) of the title compound.

LC-MS (method 1): $R_t$ = 0.63 min; MS (ESIpos): m/z = 737 $[M+H]^+$.

**Example 5**

**Gadolinium-2,2',2"-[(2*S*)-10-(carboxymethyl)-2-(4-{2-[2-(2-ethoxyethoxy)ethoxy]ethoxy} benzyl)-1,4,7,10-tetraazacyclododecane-1,4,7-triyl]triacetate**

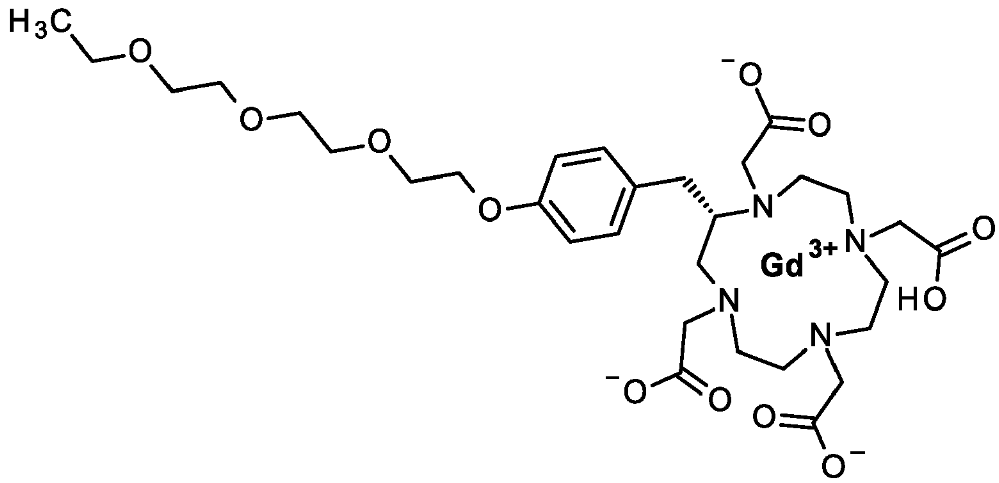

To 2,2',2",2'''-[(2*S*)-2-(4-{2-[2-(2-Ethoxyethoxy)ethoxy]ethoxy}benzyl)-1,4,7,10-tetraazacyclo dodecane-1,4,7,10-tetrayl]tetraacetic acid (free ligand 5, 540 mg, 805 μmol) suspended in water (13 ml) was added gadolinium oxide (131 mg, 362 μmol) and the pH was adjusted to pH5 by addition of aqueous sodium hydroxide (2M) and the mixture was stirred at 100°C for 1h. Chelex® was added and the mixture was stirring at RT was continued for 16h. The mixture was filtered and purified by column chromatography (Biotage®Sfär C18 D, water / acetonitrile, 0-100%) to yield 169 mg (98% purity, 25% yield) of the title compound.

LC-MS (method 1): $R_t$ = 0.66 min; MS (ESIpos): m/z = 825 $[M+H]^+$.

**Example 6**

**Gadolinium 2,2',2"-[(2S)-10-(carboxymethyl)-2-({5-[2-(2-ethoxyethoxy)ethoxy]pyridin-2-yl}methyl)-1,4,7,10-tetraazacyclododecane-1,4,7-triyl]triacetate**

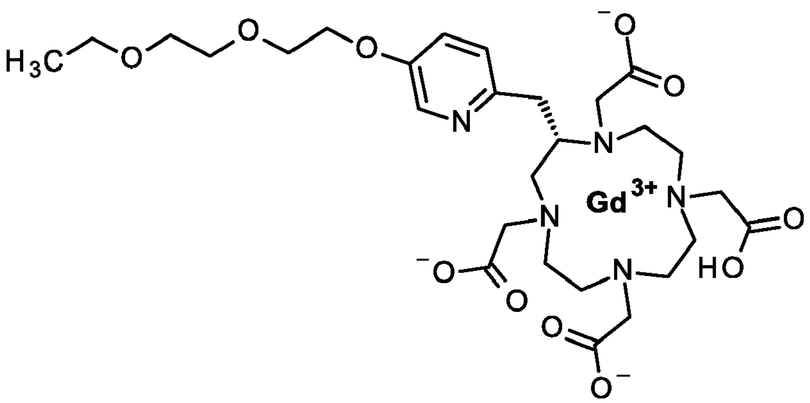

To a suspension of 2,2',2",2'''-[(2*S*)-2-({5-[2-(2-ethoxyethoxy)ethoxy]pyridin-2-yl}methyl)-1,4,7,10-tetraazacyclododecane-1,4,7,10-tetrayl]tetraacetic acid (free ligand 6, 1.09 g, 1.74 mmol) in water (28 ml) was added gadolinium oxide (283 mg, 781 μmol). The pH was adjusted from pH2 to pH5 by addition of aqueous sodium hydroxide (2 M). The reaction mixture was stirred at 100°C for 1 h. Chelex® was added and the mixture was stirred at RT overnight. The mixture was filtered and purified by column chromatography (Biotage®Sfär C18 D, 30 g, water / acetonitrile 0-100%) to

yield 39 mg (96% purity, 3% yield) of the title compound.

LC-MS (method 1): $R_t$ = 0.51 min; MS (ESIpos): m/z = 783 $[M+H]^+$.

**Example 7**

**Gadolinium-2,2',2"-[10-(carboxymethyl)-2-(4-ethoxybenzyl)-1,4,7,10-tetraazacyclododecane-1,4,7-triyl]triacetate**

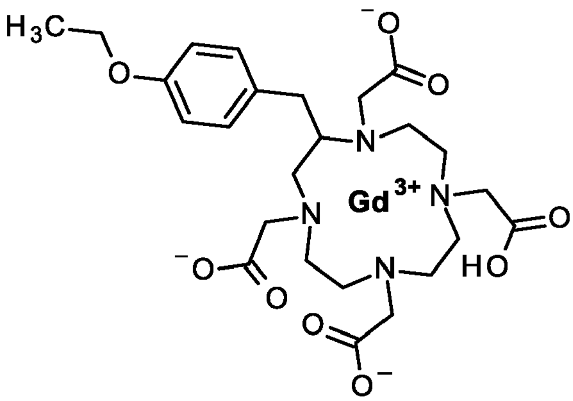

To a suspension of 2,2',2",2'''-[2-(4-ethoxybenzyl)-1,4,7,10-tetraazacyclododecane-1,4,7,10-tetrayl]tetraacetic acid (free ligand 7, 600 mg, 1.11 mmol) in water (18 ml) was added gadolinium oxide (182 mg, 501 μmol). The pH was adjusted from pH 2 to pH 5 by addition of aqueous sodium hydroxide (2 M). The reaction mixture was stirred at 100°C for 2h. Chelex® was added and the mixture was stirred at RT for 3 days. The mixture was filtered and purified by column chromatography (Biotage®Sfär C18 D, 12g, water/ acetonitrile 0-100%) to yield 263 mg (90% purity, 31% yield) of the title compound as a acemic mixture of rdiastereomers.

LC-MS (method 1): peak 1 $R_t$ = 0.64 min; MS (ESIpos): m/z = 694 $[M+H]^+$ and peak 2 $R_t$ = 0.69 min; MS (ESIpos): m/z = 694 $[M+H]^+$.

**Example 8**

**Gadolinium-2,2',2"-[10-(carboxymethyl)-2-(4-ethoxybenzyl)-1,4,7,10-tetraazacyclododecane-1,4,7-triyl]triacetate (racemic diastereomer 1)**

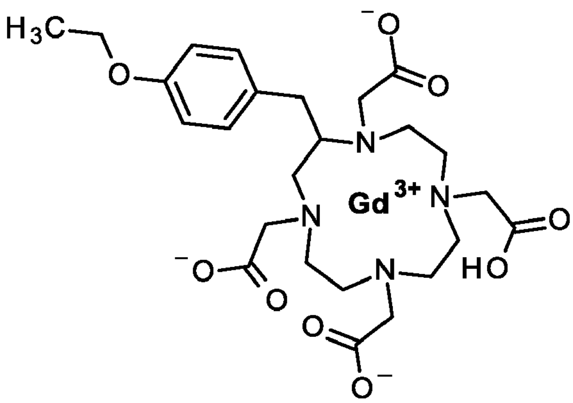

The title compound from example 7 (experiment was repeated in analogy with 776 mg 2,2',2",2'''-[2-(4-ethoxybenzyl)-1,4,7,10-tetraazacyclododecane-1,4,7,10-tetrayl]tetraacetic acid and 235 mg gadolinium oxide) was separated into racemic diastereomers by preparative HPLC (method 6, gradient: 0.00-0.50 min 10%, B, 0.50-6.00 min 10-50% B) to give title compound (diastereomer 1, 11 mg at $R_t$ = 1.2 - 1.6 min) and diastereomer 2 (60 mg at $R_t$ = 0.7 - 1.1 min,

see example 9).

LC-MS (method 1): $R_t$ = 0.69 min; MS (ESIpos): m/z = 694 $[M+H]^+$.

## Example 9

**Gadolinium-2,2',2"-[10-(carboxymethyl)-2-(4-ethoxybenzyl)-1,4,7,10-tetraazacyclododecane-1,4,7-triyl]triacetate (racemic diastereomer 2)**

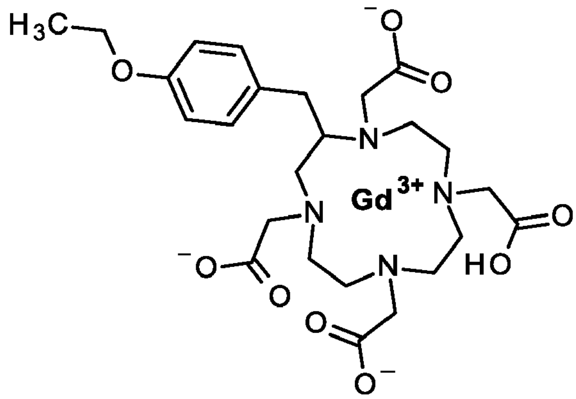

For the preparation of the title compound as racemic mixture of diastereomers see example 7. Separation of diastereomers by preparative HPLC (method see example 8) gave 60 mg of the title compound (diastereomer 2 at $R_t$ = 0.7 - 1.1 min).

LC-MS (method 1): $R_t$ = 0.64 min; MS (ESIneg): m/z = 692 $[M-H]^-$.

## Example 10

**Gadolinium-2,2',2"-[(2S)-10-(carboxymethyl)-2-(4-ethoxybenzyl)-1,4,7,10-tetraazacyclododecane-1,4,7-triyl]triacetate (stereoisomer 1)**

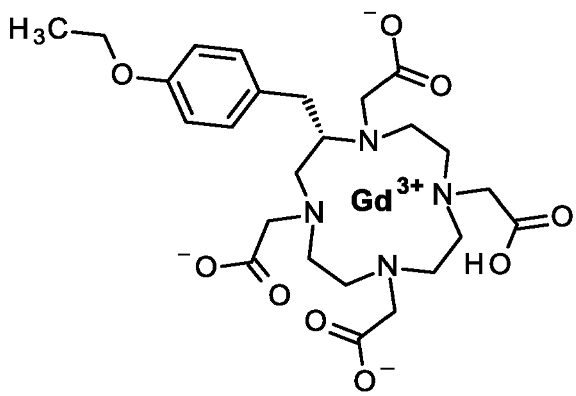

To a suspension of 2,2',2",2'''-[(2*S*)-2-(4-ethoxybenzyl)-1,4,7,10-tetraazacyclododecane-1,4,7,10-tetrayl]tetraacetic acid (1.45 g, 2.7 mmol, prepared in analogy to ligand 7 starting from (2S)-2-amino-3-(4-ethoxyphenyl)propane-1-ol CAS: 200267-69-2,) in water (43 ml) was added gadolinium oxide (440 mg, 1.2 mmol). The pH was adjusted from pH 2 to pH 5 by addition of aqueous sodium hydroxide (2M). The reaction mixture was stirred at 100°C for 2 h. Chelex ® was added and the mixture was stirred at RT for 3 days while the pH was keep at pH 5.5 by addition of aqueous HCl (2M). The mixture was filtered and purified by column chromatography (Biotage®Sfär C18 D, 60 g, water / acetonitrile 0-100% followed by Biotage®Sfär C18 D, 12 g, water / acetonitrile 0-40%) to give title compound (stereoisomer 1, 8.3 mg , 93% purity yield 0.43%) and stereoisomer 2 (65 mg, see example 11).

LC-MS (method 1): $R_t$ = 0.69 min; MS (ESIpos): m/z = 694 $[M+H]^+$

**Example 11**

**Gadolinium-2,2',2"-[(2*S*)-10-(carboxymethyl)-2-(4-ethoxybenzyl)-1,4,7,10-tetraazacyclododecane-1,4,7-triyl]triacetate (stereoisomer 2)**

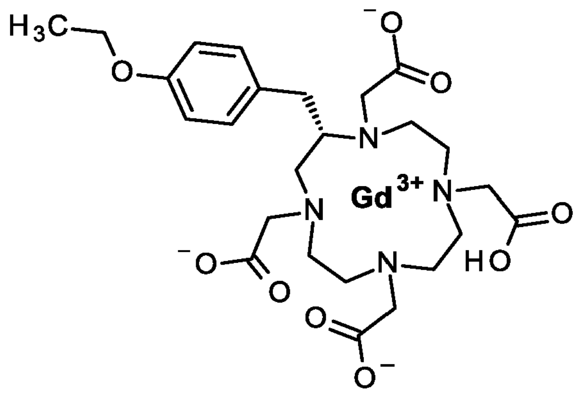

For the preparation of the title compound as mixture of stereoisomers and their separation see example 10. Separation stereoisomers by by column chromatography on Biotage®Sfär C18 gave 65 mg of the title compound (stereoisomer 2, 90% purity, 3.1% yield).

LC-MS (method 1): $R_t$ = 0.64 min; MS (ESIneg): m/z = 692 $[M-H]^-$.

Optical rotation:$[\alpha]_D$ = 21.9° +/- 0.7° (c = 3.5 mg/ml, 20°C, 589 nm, water).

**Example 12**

**Gadolinium-2,2',2"-[(2S)-2-(4-butoxybenzyl)-10-(carboxymethyl)-1,4,7,10-tetraaza cyclododecane-1,4,7-triyl]triacetate (stereoisomer 1)**

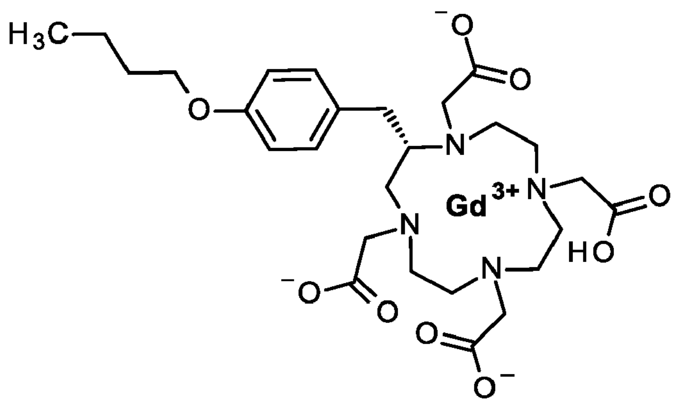

To a suspension of 2,2',2",2'''-[(2S)-2-(4-butoxybenzyl)-1,4,7,10-tetraazacyclododecane-1,4,7,10-tetrayl]tetraacetic acid (free ligand 8, 640 mg, 1.13 mmol) in water (18 ml) was added gadolinium oxide (184 mg, 508 μmol). The pH was adjusted from pH 2 to pH 5 by addition of aqueous sodium hydroxide (2 M). The reaction mixture was stirred at 100°C for 3 h. Chelex® was added and the mixture was stirred at room temperature over night while the pH was keep at pH 5.5 by addition of aqueous HCl (2M). The mixture was filtered and purified by by column chromatography (Biotage®Sfär C18 D, 30 g, water / acetonitrile 0-50% to give title compound (stereoisomer 1, purity, 11,2% yield)) and stereoisomer 2 (65 mg, see example 13).

LC-MS (method 1): $R_t$ = 0.81 min; MS (ESIpos): m/z = 722 $[M+H]^+$.

Optical rotation:$[\alpha]_D$ = 19.2° +/- 0.5° (c = 4.5 mg/ml, 20°C, 589 nm, water).

**Example 13**

**Gadolinium-2,2',2"-[(2S)-2-(4-butoxybenzyl)-10-(carboxymethyl)-1,4,7,10-tetraaza cyclododecane-1,4,7-triyl]triacetate (stereoisomer 2)**

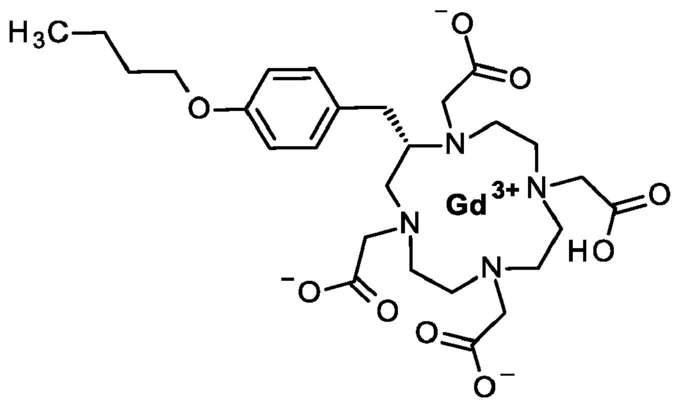

For the preparation of the title compound as mixture of stereoisomers and their separation see example 10. Separation stereoisomers by by column chromatography on Biotage®Sfär C18 gave 27.8 mg of the title compound (stereoisomer 2, 80% purity, 3% yield).

LC-MS (method 1): $R_t$ = 0.82 min; MS (ESIpos): m/z = 722 $[M+H]^+$

**EXPERIMENTAL SECTION - PHYSCOC and BIOLOGICAL ASSAYS**

**In vitro and in vivo characterization of example compounds**

Examples were tested in selected assays one or more times. When tested more than once, data are reported as either average values or as median values, wherein

- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from assays represent average values or median values calculated utilizing data sets obtained from testing of one or more synthetic batch.

**Example A**

**Water solubility**

The exploratory water solubility of the compounds is determined at room temperature (20°C) in 0.5 mL buffer solution (10 mM Tris-HCl, pH 7.4) in the microcentrifuge tubes (Eppendorf, 2.0 mL safe-lock caps). The solid compounds were added stepwise to the buffer solution. More substance is added once all the suspended material is dissolved until an equilibrium between undissolved and dissolved substance is reached. The suspension was mixed using a shaker (Heidolph Reax 2000) and treated 5 min in an ultrasound bath (Bandelin, Sonorex Super RK255H). The Gadolinium concentration of the test substance in the clear solution is measured by ICP-MS. The results are summarized in Table 1.

**Table 1:** Solubilities of compounds in aqueous buffer at 20°C (pH 7.4).

| **Example No** | **Solubility** [mol/L] |
|---|---|
| 1 | > 0.201 |
| 2 | > 0.055 |
| 3 | > 0.166 |
| 4 | > 0.738 |
| 5 | > 0.284 |
| 7 | > 0.403 |
| 9 | > 0.145 |
| 10 | > 0.023 |
| 11 | > 0.146 |
| 12 | 0.211 |
| 13 | > 0.043 |
| RC1 | > 0.250 |
| n.d. not determined | |

**Example B**

**Chemical stability**

Examples were separately dissolved in 10 mM Tris-HCI buffer, pH 7.4 at a final concentration of 1 mmol Gd/L. The solution was autoclaved three times at 1 bar, 121°C for 20 min. Before and after each autoclaving step an aliquot was removed, frozen at -20°C for later analytical analyses by HPLC-ICP-MS (Gd157) to determine the integrity of the compound.

Examples of used HPLC methods: Agilent 1290 Infinity II LC, Agilent ICP-MS 7900, Column: Waters BEH Acquity C18 UPLC, 1.7 μm, 2.1 x 50 mm. Solvent A: 20 mmol/L formic acid in water. Solvent B: MeOH. Gradient from 2% B to 98% B within 2.5 min, flow 0.8 mL/min or Solvent B: MeCN, isocratic 14% B, flow: 0.6 mL/min. Detection by ICP-MS, tuned to $^{157}Gd$ (m/z) and $^{175}Lu$ (internal standard). Before measuring via ICP the samples were mixed with 50% MeCN (+ 0.1% HNO3 + 0.5mM DTPA + 25nM Lu as IS). The chromatograms, displaying the intensity of the detected Gd, were visually compared. The compounds were valuated stable if changes in the chromatograms before and after autoclaving are < 1% (Figure 1).

**Example C**

**Complex Stability (Transmetallation)**

The used method is described elsewhere (Laurent S et al., Stability of MRI paramagnetic contrast media: a proton relaxometric protocol for transmetallation assessment. Invest Radiol. 2001 Feb;36(2):115-22). In brief: A solution containing 2.5 mmol/L of test compound and 2.5 mmol/L $ZnCl_2$ in 26 mM phosphate buffer (KH2PO4 Merck; 1.09439.1000, #HC748513, pH 7,00), was incubated at 37°C over a period of at least 3 days. If test compounds are instable under these conditions, transmetallation during this test would lead to the formation of the $Zn^{2+}$ complex of the test item, while $Gd^{3+}$ is released and precipitated as highly insoluble $GdPO_4$. Since $Zn^{2+}$ does not influence the T1 relaxation time and $Gd^{3+}$ is withdrawn from the solution by precipitation, this ongoing exchange reaction would lead to increasing T1 relaxation times of the solution over time (measured at 1.41 T).

After 3d of incubation T1 relaxation time was observed. All investigated compounds showed lower $Gd^{3+}$ dissociation of the Gd complex under these stress conditions compared to the linear reference compounds (RC1 and RC2). Stbailities of T1 relaxation rates ($R1_n/R1_0$) in % after 72h incubation are summarized in Table 2.

**Table 2:** Transmetallation stability [%] of paramagnetic contrast agents over 72 hours (2.5 mM $Zn^{2+}$, 26 mM phosphate buffer, 37°C, pH 7.4).

| Example No | Stability [%] |
|---|---|
| 2 | 95 |
| 5 | 96 |
| 7 | 98 |
| RC1 | 44 |
| RC2 | 14 |
| RC3 | stable |

**Example D**

**Relaxivity measurements at 1.4 T**

Relaxivity measurements at 1.41 T were performed using a MiniSpec mq60 spectrometer (Bruker Analytik, Karlsruhe, Germany) operating at a resonance frequency of 60 MHz and a temperature of 37°C. The $T_1$ relaxation times were determined using the standard inversion recovery (IR) method with a fixed relaxation delay of at least $5 \times T_1$. The variable inversion time (TI) was calculated automatically by the standard software of the MiniSpec mq60 (8 steps). The $T_2$ measurements were performed by using a Carr-Purcell-Meiboom-Gill (CPMG) pulse sequence, applying a relaxation delay of at least $5 \times T_1$.

Each relaxivity measurement was performed using three different Gd concentrations (3 concentrations between 0.05 and 2 mM). The $T_1$ and $T_2$ relaxation times of the example compounds 1 to 10 were measured in different media for example in water and human plasma. Human plasma preparation: For each experiment fresh blood was taken from a volunteer using 10 mL citrate-tubes (Sarstedt S-Monovette 02.1067.001, 10 mL, Citrate). The 10 mL citrate-tubes were carefully inverted 10 times to mix blood and anticoagulant and centrifuged for 15min at 1811 g at RT (Eppendorf, Centrifuge 5810R).

The relaxivities $r_i$ (where i=1, 2) were calculated based on the measured relaxation rates $R_i$ in water and plasma:

$$r_i = (R_i - R_{i(0)}) / C_{Gd}$$

where $R_{i(0)}$ represent the relaxation rate of the respective solvent and $C_{Gd}$ the concentration of the compound normalized to the Gadolinium. The Gadolinium concentrations of the investigated solutions were verified by Inductively Coupled Plasma Mass Spectrometry (ICP-MS Agilent 7500a, Waldbronn, Germany). The determined relaxivity values are summarized in Table 3.

**Table 3:** Relaxivities of investigated compounds in water and human plasma at 1.41 T and relaxivities of Reference compounds 1-3 (RC1-RC3) at 1.5 T in water and bovine plasma. All values are measured at 37°C and normalized to Gd and given in L $mmol^{-1}$ $s^{-1}$.

| Example No | $r_1$ water* | $r_2$ water* | $r_1$ human plasma* | $r_2$ human plasma* |
|---|---|---|---|---|
| 1 | 5.1 | 6.0 | 6.6 | 8.4 |
| 2 | 4.9 | 5.8 | 5.9 | 7.8 |
| 3 | 5.0 | 5.9 | 6.8 | 8.9 |
| 4 | 4.8 | 5.8 | 6.6 | 8.9 |
| 5 | 5.1 | 5.9 | 5.7 | 7.2 |
| 6 | 5.3 | 6.3 | 5.9 | 7.4 |
| 7 | 4.5 | 5.3 | 6.2 | 8.4 |
| 8 | 4.7 | 5.5 | 6.5 | 8.7 |
| 9 | 4.3 | 5.1 | 5.6 | 8.2 |

(continued)

| Example No | $r_1$ water* | $r_2$ water* | $r_1$ human plasma* | $r_2$ human plasma* |
|---|---|---|---|---|
| 10 | 4.8 | 5.6 | 6.4 | 8.5 |
| 11 | 4.4 | 5.2 | 6.2 | 8.7 |
| 12 | 4.7 | 5.6 | 8.8 | 16.0 |
| 13 | 5.3 | 9.1 | 9.8 | 16.1 |
| RC1^ | 4.7 | 5.1 | 6.9 | 8.7 |
| RC2^ | 4.0 | 4.3 | 6.3 | 8.7 |
| RC3^ | 3.3 | 3.9 | 5.2 | 6.1 |
| * values are depicted in L $mmol^{-1}$ $s^{-1}$<br>^ Relaxivities from reference compounds from Rohrer et. al. at 1.5 T (Invest. Radiol. 2005; 40, 11: 715-724) and in bovine plasma (Kreaber GmbH, Pharmaceutical Raw Material, Ellerbek, Germany) instead of human plasma | | | | |

**Example E Uptake into freshly isolated rat hepatocytes**

Freshly isolated rat hepatocytes were received as described before (Papeleu P. et al. (2006) Isolation of Rat Hepatocytes. In: Phillips I.R., Shephard E.A. (eds) Cytochrome P450 Protocols. Methods in Molecular Biology, vol 320. Humana Press, Totowa, NJ. https://doi.org/10.1385/1-59259-998-2:229). The rat hepatocytes were incubated ($2.5 \cdot 10^5$ cells/well) for 2.5 to 4h in collagen coated 24-well plates (Fa. Beckton Dickinson Biocoat, cell environments, Collagen I Cellware) in Williams E-medium (Fa. Sigma W1878) in an incubator (37°C, 5% $CO_2$ and 95% oxygen). After incubation the adherent hepatocytes were washed once with 37°C Hepes carbonate transport buffer (500 µL/well). The test items and RC1 were prepared from stock solutions (180 to 500 mM concentration) in Hepes carbonate transport buffer at pH 7.4 to reach a final concentration of 100 µmol Gd/L. The specific uptake of the test item or RC1 into rat hepatocytes via OATP1 B1 and OATP1 B3 was challenged using a known OATP1B1/1B3 inhibitor (Rifampicine, Fa. Sigma R3501, test item and inhibitor 1:1 each 100 µM final concentration). The prepared rat hepatocyte plates were incubated with test solutions (500 µL/well) at 37°C for 10 min using a thermoshaker (Fa. Grant Bio, PHMP). Triplicates were performed for each test item. After incubating the cells were washed once with cold PBS buffer (Fa. Gibco, Dulbeccos PBS (+)). After washing the intact hepatocytes were lysed by addition of 500 µL cold 0.01 % Triton-X 100 in Millipore water for 1h. The strongly hypo-osmolar water led to cell membrane rupture and the release of the intracellular Gd-compound. After 1h 500 µL of 10 nM Terbium ICP-MS standard was added in 2% nitric acid and 0.01% Triton 100 before the Gadolinium concentrations in the lysate were measured using ICP-MS (Agilent 8900, singleQuad, integr. Time 0.3 s, no gas, 5 replicates, Gd m/z 157, Tb m/z 159). As a reference standard RC1 is used in the same well plate. The hepatocyte uptake of the test compound is calculated relative to RC1 (resulting in the unit % of RC1). The results different are summarized in the Table 4.

**Table 4:** Uptake of compounds into freshly isolated rat hepatocytes in % compared to reference compound 1 (RC1, Gd-EOB-DTPA).

| Example No | Uptake rat hepatocytes [% of RC1] | Inhibition Rifampicine [% inhibition] |
|---|---|---|
| 1 | 47 | 90 |
| 2 | 65 | 93 |
| 3 | 49 | 83 |
| 4 | 67 | 94 |
| 5 | 57 | 89 |
| 6 | 47 | 91 |
| 7 | 73 | 96 |
| 8 | 143 | 96 |
| 9 | 42 | 93 |
| 10 | 104 | 96 |

(continued)

| Example No | Uptake rat hepatocytes [% of RC1] | Inhibition Rifampicine [% inhibition] |
|---|---|---|
| 11 | 49 | 96 |
| 12 | 110 | 96 |
| 13 | 137 | 86 |
| RC1^ | 100 | 97 |
| RC2^ | 9 | 79 |
| n.d. not determined | | |

**Example F Uptake into stable transfected OATP1B1 HEK 293 cells**

Human stable transfected OATP1B1 human embryonic kidney 293 cells (OATP1B1 HEK 293, SLCO1B1, Entrez Gene ID: 10599) were used (Leonhardt M et al. Drug Metab Dispos. 2010 Jul;38(7):1024-8). The following culture medium was used: 500 mL DMEM (Dulbecco's Modified Eagle Medium - high Glucose, D6429), 50 mL FCS (fetal bovine serum, Fa. Sigma, F7524), 5.8 mL G418 (Geneticin, Fa. Gibco 10131-027) and 5 mL Pen-Strep. (Penicillin-Streptomycin, Fa. Gibco, Ref 15140-122). Cryopreserved cells were shortly thawed in water bath and diluted in 10 mL warm culture medium (37°C). After medium change the cells were grown for 3d in a 25 $cm^2$ cell culture flask. After 3 to 5d the cells were transferred into 75 $cm^2$ flasks. For the splitting the cells were incubated with 1.5 mL trypsin (Trypsin-EDTA (1x) 0,05%, Ref 25300-054 100ml, Fa. Gibco) for 3-5min in the incubator (37°C, 5% $CO_2$ and 95% oxygen). After centrifugation and washing with medium the cells were counted (50 μL cell suspension and 50 μL trypan blue (Trypan Blue Solution, 93595-50ml, Fa. Fluka). Cells were cultured ($1\times10^5$ cells/well) in poly-D-lysine coated 24-well plates (Fa. Corning, Ref: 354414) in 1.5 mL medium per well. After 48h in the incubator the medium was changed and 1.5 mL/well sodium butyrate containing medium were added (40 mL culture medium plus 400 μL Na-Butyrate, Fa. Sigma Aldrich B5887-5G, 250 mg in 2.27 mL MilliQ water). After 24h in the incubator the medium was vacuumed off and 1.5 mL transport buffer was added and incubated for 10min. The transport buffer contains the following ingredients: 500 mL HBSS (Hank's balanced salt solution, Hyclone, SH30268.01), 5 mL HEPES buffer (4-(2-hydroxyethyl)-1-piperazineethanesulfonic acid, Gibco 15630-056) and 3.9 mL glucose (Sigma, G8270-100, 90 g/200mL MilliQ water). All test items were prepared in transport buffer at a final concentration of 100 μM from stock solutions (180 to 500 mM concentration). The OATP1B1 HEK 293 cells were incubated with test solutions (test item in transport buffer 500 μL) for 40min on a thermoshaker without shaking (PHMP, Fa. Grant Bio). Triplicates were performed for each test item. After incubation the cells were washed with 2 mL of cold PBS (+) buffer (Dulbeccos PBS +, Fa. Gibco). The OATP1B1 HEK 293 cells were lysed by addition of 500 μL cold 0.01 % Triton-X 100 in Millipore water for 1h. The strongly hypo-osmolar water led to cell membrane rupture and the release of the intracellular Gd-compound. After 1h 500 μL of 1 nM Terbium ICP-MS standard was added in 2% nitric acid and 0.01% Triton 100. Before the Gadolinium concentrations in the lysate were measured using ICP-MS (Agilent 8900, singleQuad, integr. Time 0.3 s, no gas, 5 replicates, Gd m/z 157, Tb m/z 159) the samples were centrifuged at 4000 rpm for 2min. As a reference standard Gd-EOB-DTPA (gadoxetate) is used in the same well plate. The OATP1B1 HEK 293 uptake of the test compound is calculated relative to gadoxetate (resulting in the unit % of RC1). The results are summarized in Table 5.

**Table 5:** Uptake of compounds into stably transfected OATP1B1-HEK 293 cells in % compared to reference compound 1 (RC1, Gd-EOB-DTPA).

| Example No | Uptake into OATP1B1-HEK 293 cells [% of RC1] | Inhibition Rifampicine [% inhibition] |
|---|---|---|
| 1 | 143 | 21 |
| 2 | 282 | 0 |
| 3 | 120 | 31 |
| 4 | 65 | 29 |
| 5 | 78 | 41 |
| 6 | 174 | 2 |
| 7 | 103 | 78 |

(continued)

| Example No | Uptake into OATP1B1-HEK 293 cells [% of RC1] | Inhibition Rifampicine [% inhibition] |
|---|---|---|
| 8 | 208 | 75 |
| 9 | 60 | 23 |
| 10 | 212 | 73 |
| 11 | 96 | 64 |
| 12 | 119 | 81 |
| 13 | 847 | 88 |
| RC1^ | 100 | 81 |
| n.d. not determined | | |

**Example G Uptake into stable transfected OATP1B3 HEK 293 cells**

Human transfected OATP1B3 human embryonic kidney 293 cells (OATP1B3 HEK 293, SLCO1B3, Entrez Gene ID: 28234) were used (Leonhardt M et al. Drug Metab Dispos. 2010 Jul;38(7):1024-8). The following culture medium was used: 500 mL DMEM (Dulbecco's Modified Eagle Medium - high Glucose, D6429), 50 mL FCS (fetal bovine serum, Fa. Sigma, F7524), 5.8 mL G418 (Geneticin, Fa. Gibco 10131-027) and 5 mL Pen-Strep. (Penicillin-Streptomycin, Fa. Gibco, Ref 15140-122). Cryopreserved cells were shortly thawed in water bath and diluted in 10 mL warm culture medium (37°C). After medium change the cells were grown for 3d in a 25 $cm^2$ cell culture flask. After 3 to 5d the cells were transferred into 75 $cm^2$ flasks. For the splitting of cells these were incubated with 1.5 mL trypsin (Trypsin-EDTA (1x) 0,05%, Ref 25300-054 100ml, Fa. Gibco) for 3-5 min in the incubator (37°C, 5% $CO_2$ and 95% oxygen). After centrifugation and washing with medium the cells were counted (50 μL cell suspension and 50 μL trypan blue (Trypan Blue Solution, 93595-50ml, Fa. Fluka). Cells were cultured ($1\times10^5$ cells/well) in poly-D-lysine coated 24-well plates (Fa. Corning, Ref: 354414) in 1.5 mL medium per well. After 48 hours in the incubator the medium was changed and 1.5 mL/well sodium butyrate containing medium were added (40 mL culture medium plus 400 μL Na-Butyrate, Fa. Sigma Aldrich B5887-5G, 250 mg in 2.27 mL MilliQ water). After 24 hours in the incubator the medium was vacuumed off and 1.5 mL transport buffer was added and incubated for 10 min. The transport buffer contains the following ingredients: 500 mL HBSS (Hank's balanced salt solution, Hyclone, SH30268.01), 5 mL HEPES buffer (4-(2-hydroxyethyl)-1-piperazine-ethanesulfonic acid, Gibco 15630-056) and 3.9 mL glucose (Sigma, G8270-100, 90 g/200mL MilliQ water). All test items were prepared in transport buffer at a final concentration of 100 μM from stock solutions (180 to 500 mM concentration). The OATP1B3 HEK 293 cells were incubated with test solutions (test item in transport buffer 500 μL) for 40min on a thermoshaker without shaking (PHMP, Fa. Grant Bio). Triplicates were performed for each test item. After incubation the cells were washed with 2 mL of cold PBS (+) buffer (Dulbeccos PBS +, Fa. Gibco). The OATP1B3 HEK 293 cells were lysed by addition of 500 μL cold 0.01 % Triton-X 100 in Millipore water for 1h. The strongly hypo-osmolar water led to cell membrane rupture and the release of the intracellular Gd-compound. After 1 hour 500 μL of 1 nM Terbium ICP-MS standard was added in 2% nitric acid and 0.01% Triton 100. Before the Gadolinium concentrations in the lysate were measured using ICP-MS (Agilent 8900, singleQuad, integr. Time 0.3 s, no gas, 5 replicates, Gd m/z 157, Tb m/z 159) the samples were centrifuged at 4000 rpm for 2 min. As a reference standard Gd-EOB-DTPA (gadoxetate) is used in the same well plate. The OATP1B3 HEK 293 uptake of the test compound is calculated relative to gadoxetate (resulting in the unit % of RC1). The results are summarized in Table 6.

**Table 6:** Uptake of compounds into stably transfected OATP1B3-HEK 293 cells in % compared to reference compound 1 (RC1, Gd-EOB-DTPA).

| Example No | Uptake into OATP1B3-HEK 293 cells [% of RC1] | Inhibition Rifampicine [% inhibition] |
|---|---|---|
| 1 | 79 | 92 |
| 2 | 121 | 86 |
| 3 | 28 | 84 |
| 4 | 103 | 97 |

(continued)

| Example No | Uptake into OATP1B3-HEK 293 cells [% of RC1] | Inhibition Rifampicine [% inhibition] |
|---|---|---|
| 5 | 100 | 96 |
| 6 | 32 | 65 |
| 7 | 36 | 95 |
| 8 | 94 | 99 |
| 9 | 13 | 94 |
| 10 | 80 | 96 |
| 11 | 32 | 90 |
| 12 | 66 | 98 |
| 13 | 383 | 98 |
| RC1^ | 100 | 99 |

**Example H**

**Partition coefficient (logP butanol/buffer)**

The partition coefficient P (log P= log($C_{butanol}$/$C_{buffer}$)) was determined in buffered aqueous solution (10 μM of compound in 0.5 mL 50 mM Tris-HCl saturated with Butanol, pH 7.4) and 1-butanol 1+1 and the mixture was shaken for 2h at room temperature (n=3). The Gd-concentrations in each phase. $C_{butanol}$ and $C_{buffer}$ were measured by ICP-MS (The Chemistry of Contrast Agents in Medical Magnetic Resonance Imaging" 2nd Ed. Merbach AS, Helm L, Toth E, eds. Hoboken, NJ: Wiley 2013).

**Table 7:** Partition coefficient P (log P= log(butanol/buffer, pH 7.4))

| Example No | Log P |
|---|---|
| 1 | -2.12 |
| 2 | -1.52 |
| 3 | -2.05 |
| 4 | -1.57 |
| 5 | -1.74 |
| 6 | -1.96 |
| 7 | -1.32 |
| 8 | -1.33 |
| 9 | -1.36 |
| 10 | -1.34 |
| 11 | -1.37 |
| 12 | -0.45 |
| RC1^ | -2.12 |
| RC2^ | -2.07 |

**Example I NOAEL**

The no-observed-adverse-effect level (NOAEL) was tested in mice using a modified 4-level up procedure with

increasing doses of the test items. The observed NOAEL denotes the level of exposure, at which no adverse effects were observed (Dorato et al. Regul Toxicol Pharmacol. 2005 Aug;42(3):265-74). The test items were applicated as intravenous bolus injection via the tail vein. The starting dose was 0.2 mmol/kg bw (8-fold of the clinical dose of RC1), and the dose for the next level was increased to 0.5 mmol Gd/kg bw if the animals showed no adverse effects and decreased if the mice showed any adverse effects. The next level was 1.5 mmol Gd/kg bw. At the highest dose level of 2.5 mmol Gd/kg bw 3 animals were injected. Symptoms were observed continuously during the whole experiment (2h after contrast agent administration). Animals were euthanized in deep anesthesia 48h post injection (xylazine hydrochloride 20 mg/mL, Rompun 2%, Bayer Vital GmbH, Leverkusen and ketamine hydrochloride 100 mg/mL, Ketavet, Pfizer, Pharmacia GmbH, Berlin). Macroscopic analysis was performed in all mice immediately after death. Blood was collected by puncture of vena cava and ALT, AST, GGT, GLDH, creatinine and BUN were analyzed (300 μL serum). The NOAEL levels are summarized in Table in relation to their lipophilicity (logP values) in Figure 2.

**Table 8:** Examples of no-observed-adverse-effect level (NOAEL)

| Example No | NOAEL [mmol Gd/kg bw] |
|---|---|
| 4 | 2.50 |
| 7 | 1.50 |

**Example O Contrast-Enhanced Liver MRI in healthy mice**

Dynamic liver contrast-enhanced magnetic resonance imaging (CE-MRI) was performed in healthy mice. The liver MRI signal intensity change over time was compared to RC1 (Primovist). The studies were performed at a 4.7T preclinical MRI scanner equipped with a dedicated transmit-receive mouse body volume coil (Bruker Biospec 70/20, Bruker BioSpin, Ettlingen, Germany). Studies were done using female NMRI mice (-25 g, n=3, Charles River Kisslegg). The animals were anesthetized using a mixture of isoflurane (initial 3%, then 2 %), oxygen gas (ca. 0.5 L/min) and nitrous oxide (flow ca. 1 L/min). CE-MRI was done using a T1-weighted FLASH sequence with retrospective respiratory gating. Different imaging slices (kidney, liver and muscle) were investigated. The acquisition time per image was 32 seconds and 110 repetitions were performed in each study. After 4 repetitions the example compound (0.025 mmol/kg bw), reference compound 1 (RC1, Primovist, 0.025 mmol/kg bw) or reference compound 2 (RC2, Multihance, 0.05 mmol/kg bw) was injected. Three different regions-of interest within the liver were evaluated. The relative signal enhancement over time was compared to that of RC1 and RC2, as very well known liver specific MRI market products. Representative images of the liver before and ~10min after administration are depicted in Figure 2.

**Example P CT phantom study**

The x-ray attenuation was investigated with computed tomography (CT). Therefore, 6 different test samples (example 4, 5, 6, 2, 12 and RC1) were formulated at a concentration of 10 mmol Gd/L. One additional test sample contains water and another additional sample contains an X-ray contrast media (RC4) formulated at a concentration of 10 mmol Iodine /L. The 8 samples were placed in water phantom. CT imaging was performed using a Dual Source CT Scanner (Somatom Force, Siemens Healthcare, Erlangen, Germany) operating in single source mode. The scan parameters were 120 kV tube voltage, 200 mAs, 0.5s rotation time and image reconstruction as performed using a Br36 kernel, 1 mm slice thickness and a field of view of 240 $mm^2$. CT imaging showed a high and very similar x-ray signal enhancement of all 6 Gd containing test samples. The signal enhancement was higher than that of RC4 formulated at identical molar attenuating element (iodine) concentration. The images demonstrated a high contrast between the 6 test samples and the reference water sample (Figure 3)

**Reference Compounds**

**Reference compound 1**
Primovist® (Gd-EOB-DTPA, gadoxetate disodium, Bayer AG, Germany)

**Reference compound 2**
MultiHance® (Gd-BOPTA, gadobenic acid, Bracco, Italy)

**Reference compound 3**
Gadovist®/ Gadavist® (Gd-DO3A-butro!, gadobutrol, Bayer AG, Germany)

**Reference compound 4**
Ultravist 300® (iopromide, Bayer AG, Germany)

## Claims

1. A compound of general formula (I),

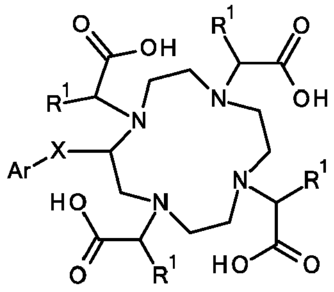

(I) ,

in which:

Ar represents a group selected from

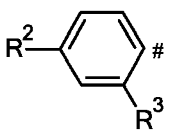 , 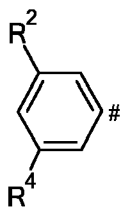 , 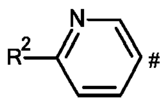 and 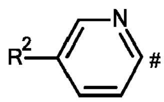 ,

wherein # indicates the point of attachment to X,

X represents a group selected from $CH_2$, $(CH_2)2$, $(CH_2)3$, $(CH_2)_4$ and $*\text{-}(CH_2)_2\text{-}O\text{-}CH_2\text{-}^{\#}$, wherein * indicates the point of attachment to Ar and $^{\#}$ indicates the point of attachment to the acetic acid moiety;
$R^1$ represents a hydrogen atom or a group selected from $C_1$-$C_3$-alkyl, $-CH_2OH$, $-(CH_2)_2OH$ and $-CH_2OCH_3$;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-;

and stereoisomers, tautomers, N-oxides, hydrates, solvates, or salts thereof, or mixtures of same.

2. The compound according to claim 1, wherein

Ar represents a group selected from

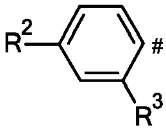, 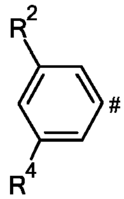, 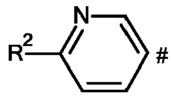 and 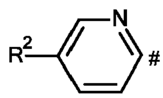

wherein # indicates the point of attachment to X,

X represents a group selected from $CH_2$, $(CH_2)2$, $(CH_2)3$, $(CH_2)_4$ and *-$(CH_2)_2$-O-$CH_2$-#, wherein * indicates the point of attachment to Ar and # indicates the point of attachment to the acetic acid moiety;
$R^1$ represents a hydrogen atom or a group selected from $C_1$-$C_3$-alkyl, -$CH_2OH$, -$(CH_2)_2OH$ and -$CH_2OCH_3$;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ represents a hydrogen atom
$R^4$ represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-;

and stereoisomers, tautomers, N-oxides, hydrates, solvates, or salts thereof, or mixtures of same.

**3.** The compound according to any of claims 1 or 2, wherein

Ar represents a group selected from

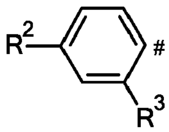 and 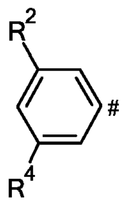,

wherein # indicates the point of attachment to X,

X represents a group selected from $CH_2$, $(CH_2)_2$, $(CH_2)_3$, $(CH_2)_4$ and *-$(CH_2)_2$-O-$CH_2$-#, wherein * indicates the point of attachment to Ar and # indicates the point of attachment to the acetic acid moiety;
$R^1$ represents a hydrogen atom or a group selected from $C_1$-$C_3$-alkyl, -$CH_2OH$, -$(CH_2)_2OH$ and -$CH_2OCH_3$;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

**4.** The compound according to any of claims 1 to 3, wherein

Ar represents a group selected from

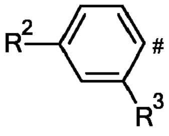 and 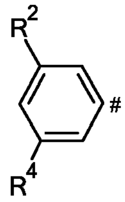 ,

wherein # indicates the point of attachment to X,

X represents a group selected from $CH_2$ and $(CH_2)_2$;
$R^1$ represents a hydrogen atom or a $-CH_2OH$ group;
$R^2$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom;
$R^3$ and $R^4$ represent, independently from each other, a hydrogen atom;

and stereoisomers, tautomers, N-oxides, hydrates, solvates, or salts thereof, or mixtures of same.

**5.** The compound according to any of claims 1 to 4, wherein

Ar represents a group selected from

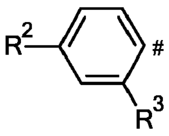 and 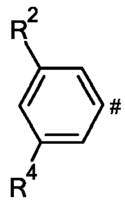 ,

wherein # indicates the point of attachment to X,

X represents a group selected from $CH_2$ and $(CH_2)2$;
$R^1$ represents a hydrogen atom or a $-CH_2OH$ group;
$R^2$ represents a group selected from $(H_3C-CH_2O)-(CH_2)_2-O-$, $(H_3C-CH_2O)-(CH_2)_2-O-(CH_2)_2-O-$ and $(H_3C-CH_2O)-(CH_2)_2-O-(CH_2)_2-O-(CH_2)_2-O-$;
$R^3$ and $R^4$ represent, independently from each other, a hydrogen atom;

and stereoisomers, tautomers, N-oxides, hydrates, solvates, or salts thereof, or mixtures of same.

**6.** The compound of general formula (I) according to any one of claims 1 to 5 in the form of a complex with $Gd^{3+}$, or a stereoisomer, a tautomer, an N-oxide, a hydrate, a solvate, or a salt thereof, or a mixture of same.

**7.** A compound according to claim 6 selected from the group consisting of

Gadolinium-2,2',2"-[(2S)-10-(carboxymethyl)-2-(4-{2-[2-(2-methoxyethoxy)ethoxy] ethoxy}benzyl)-1,4,7,10-tetraazacyclododecane-1,4,7-triyl]triacetate,
Gadolinium-2,2',2"-[(2*S*)-10-(carboxymethyl)-2-{4-[2-(2-ethoxyethoxy)ethoxy]benzyl}-1,4,7,10-tetraazacyclododecane-1,4,7-triyl]triacetate,
Gadolinium-2,2',2"-[(2*R*)-10-(carboxymethyl)-2-(4-{2-[2-(2-methoxyethoxy)ethoxy] ethoxy}benzyl)-1,4,7,10-tetraazacyclododecane-1,4,7-triyl]triacetate,
Gadolinium-2,2',2"-{(2S)-10-(carboxymethyl)-2-[4-(2-ethoxyethoxy)benzyl]-1,4,7,10-tetraazacyclododecane-1,4,7-triyl}triacetate,
Gadolinium-2,2',2"-[(2S)-10-(carboxymethyl)-2-(4-{2-[2-(2-ethoxyethoxy)ethoxy]ethoxy} benzyl)-1,4,7,10-tetraazacyclododecane-1,4,7-triyl]triacetate,
Gadolinium 2,2',2"-[(2S)-10-(carboxymethyl)-2-({5-[2-(2-ethoxyethoxy)ethoxy]pyridin-2-yl}methyl)-1,4,7,10-tetraazacyclododecane-1,4,7-triyl]triacetate,
Gadolinium-2,2',2"-[10-(carboxymethyl)-2-(4-ethoxybenzyl)-1,4,7,10-tetraazacyclododecane-1,4,7-triyl]triacetate,
Gadolinium-2,2',2"-[10-(carboxymethyl)-2-(4-ethoxybenzyl)-1,4,7,10-tetraazacyclododecane-1,4,7-triyl]triacetate racemic diasereomer 1,
Gadolinium-2,2',2"-[10-(carboxymethyl)-2-(4-ethoxybenzyl)-1,4,7,10-tetraazacyclododecane-1,4,7-triyl]triacetate racemic diasereomer 2,
Gadolinium-2,2',2"-[(2S)-10-(carboxymethyl)-2-(4-ethoxybenzyl)-1,4,7,10-tetraazacyclododecane-1,4,7-triyl]triacetate stereoisomer 1,
Gadolinium-2,2',2"-[(2S)-10-(carboxymethyl)-2-(4-ethoxybenzyl)-1,4,7,10-tetraazacyclododecane-1,4,7-triyl]triacetate stereoisomer 2,
Gadolinium-2,2',2"-[(2S)-2-(4-butoxybenzyl)-10-(carboxymethyl)-1,4,7,10-tetraaza cyclododecane-1,4,7-triyl]triacetate stereoisomer 1 and
Gadolinium-2,2',2"-[(2S)-2-(4-butoxybenzyl)-10-(carboxymethyl)-1,4,7,10-tetraaza cyclododecane-1,4,7-triyl]triacetate stereoisomer 2

or a stereoisomer, a tautomer, an N-oxide, a hydrate, a solvate, or a salt thereof, or a mixture of same.

**8.** The compound of general formula (I) according to any one of claims 6 or 7 in the form of a sodium ($Na^+$) salt of a complex with $Gd^{3+}$, or a stereoisomer, a tautomer, an N-oxide, a hydrate or a solvate thereof, or a mixture of same.

**9.** The compound of general formula (I) according to any one of claims 1 to 5 in the form of a metal complex with a metal ion suitable for computed tomography, or a stereoisomer, a tautomer, an N-oxide, a hydrate, a solvate, or a salt thereof, or a mixture of same.

**10.** The compound of general formula (I) according to any one of claims 1 to 5 or 9 in the form of a metal complex with a metal ion suitable for computed tomography, wherein the metal ion suitable for computed tomography is a metal ion with a k-edge energy in the range of from 33 to 91 keV and/or a metal ion having at least the x-ray attenuation of iodine in the energy range of medical x-ray imaging.

**11.** The compound of general formula (I) according to any one of claims 1 to 5 or 9 in the form of a metal complex with a metal ion suitable for computed tomography, wherein the metal ion suitable for computed tomography is a lanthanide or selected from the group consisting of Bi, W, Hf and Ta.

**12.** A method of preparing a compound of general formula (I) in the form of a complex with $Gd^{3+}$ according to any of claims 6 or 7, said method comprising the step of allowing a compound of general formula (I)

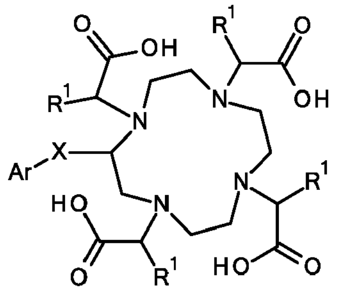

(I) ,

or a stereoisomer, tautomer, N-oxide, hydrate, solvate, or salt thereof, or mixtures of same in which Ar, X, $R^1$, $R^2$, $R^3$ and $R^4$ are as defined for the compounds of general formula (I), according to any one of claims 1 to 5, to react with a Gadolinium (III) salt,
thereby giving a compound of general formula (I), in which Ar, X, $R^1$, $R^2$, $R^3$ and $R^4$ are as defined for the compounds of general formula (I), according to any one of claims 1 to 5, in the form of a complex with $Gd^{3+}$.

**13.** Use of a compound of any one of claims 1 to 11 for diagnostic imaging.

**14.** Use of a compound of any one of claims 1 to 11 for computed tomography or magnetic resonance imaging.

**15.** Use of a compound of any one of claims 1 to 11 for magnetic resonance imaging, preferably of the vascular, renal or of the hepatobiliary system or of the gastrointestinal tract, more preferably for magnetic resonance imaging of the liver.

**16.** The compounds according to any one of claims 1 to 11 for use in diagnostic imaging.

**17.** The compounds according to any one of claims 1 to 11 for use in computed tomography or magnetic resonance imaging.

**18.** The compounds according to any one of claims 1 to 11 for use in magnetic resonance imaging, preferably of the vascular, renal or of the hepatobiliary system or of the gastrointestinal tract, more preferably for magnetic resonance imaging of the liver.

**19.** Use of the compounds according to any one of claims 1 to 11 or mixtures thereof for the manufacture of diagnostic agents.

**20.** Use of the compounds according to any one of claims 1 to 11 or mixtures thereof for the manufacture of contrast agents for computed tomography or magnetic resonance imaging, preferably for the manufacture of contrast agents for magnetic resonance imaging.

**21.** A method of imaging body tissue in a patient, comprising the steps of administering to the patient an effective amount of one or more compounds according to any one of claims 6 to 11 in a pharmaceutically acceptable carrier, and subjecting the patient to diagnostic imaging, preferably magnetic resonance imaging.

**22.** Use of a compound of general formula (I)

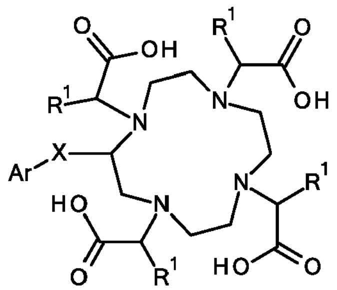

(I) ,

or a stereoisomer, tautomer, N-oxide, hydrate, solvate, or salt thereof, or mixtures of same in which Ar, X, $R^1$, $R^2$, $R^3$ and $R^4$ are as defined for the compounds of general formula (I), according to any one of claims 1 to 5, for the preparation of a compound of general formula (I) in the form of a complex with $Gd^{3+}$ according to any one of claims 6 or 7.

Figure 1

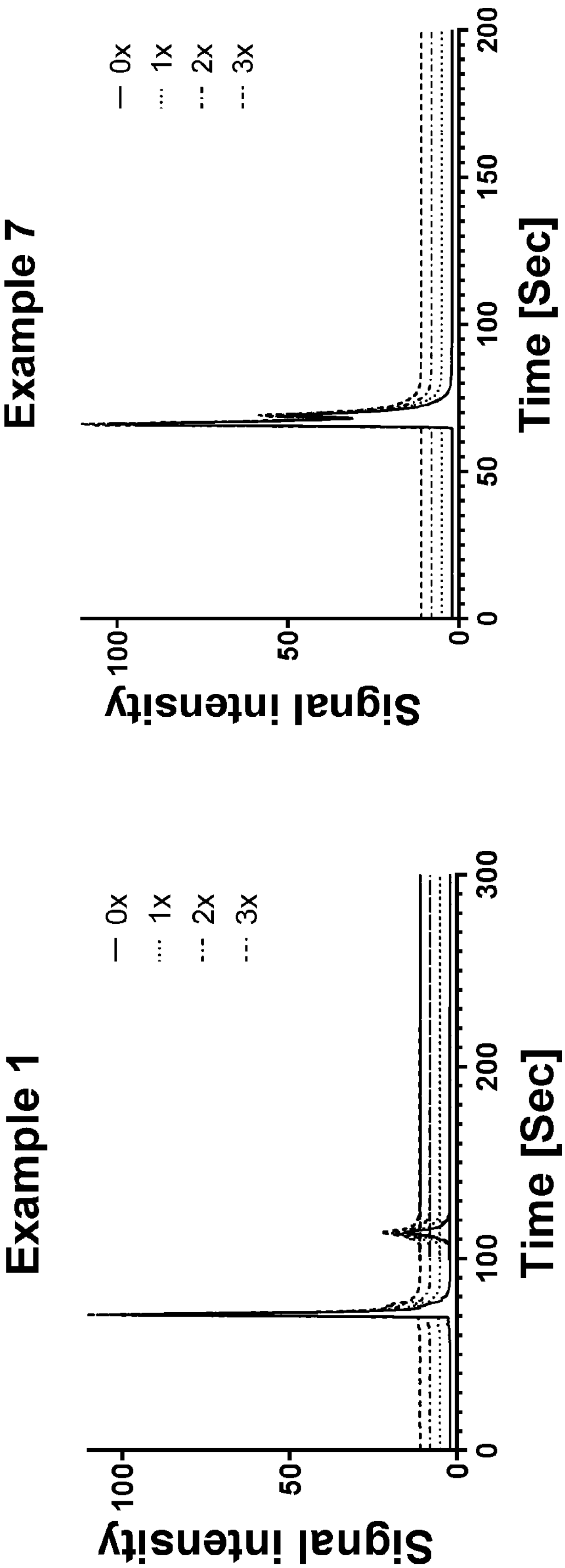
Example 7
Signal intensity
100
50
0
0
50
100
150
200
Time [Sec]
0x
1x
2x
3x
Example 1
Signal intensity
100
50
0
0
100
200
300
Time [Sec]
0x
1x
2x
3x

Figure 2

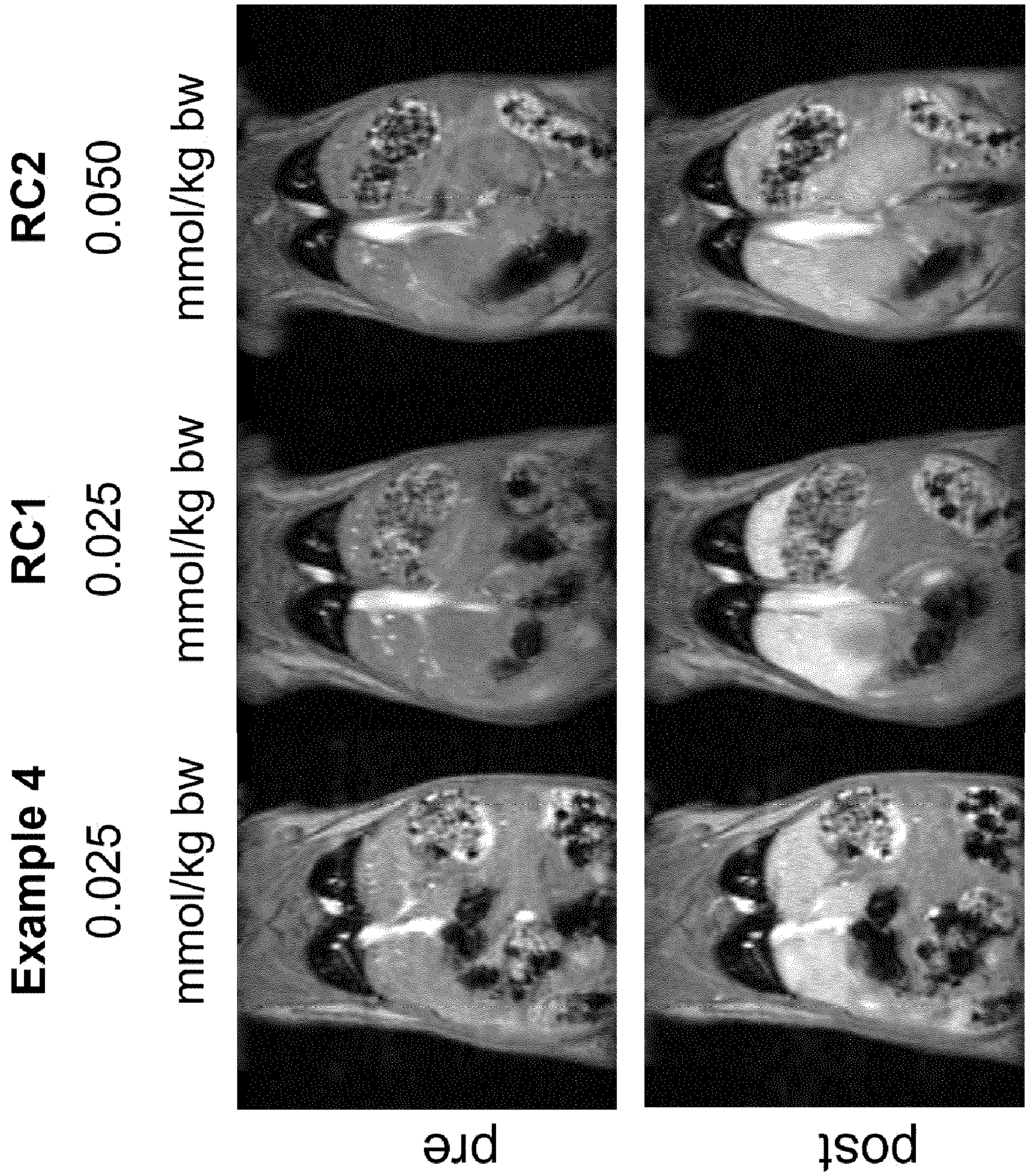
Example 4
0.025
mmol/kg bw
RC1
0.025
mmol/kg bw
RC2
0.050
mmol/kg bw
pre
post

Figure 3

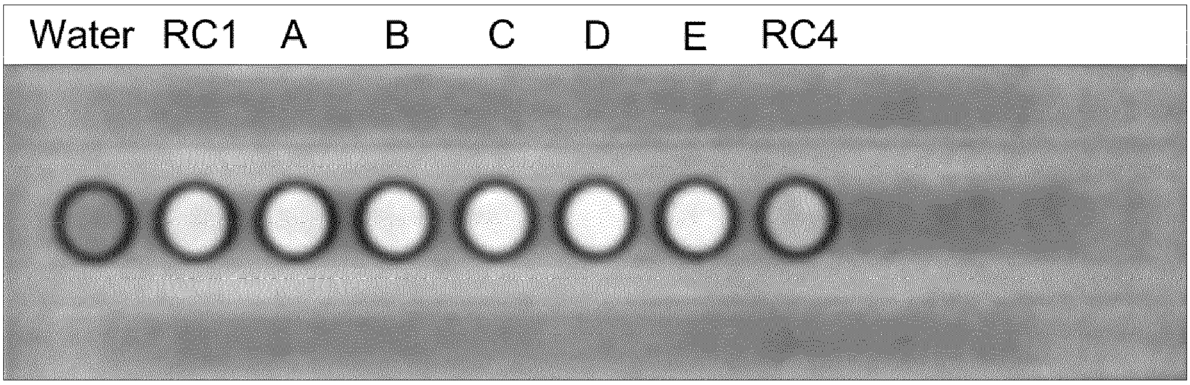

Europäisches Patentamt
European Patent Office
Office européen des brevets

# EUROPEAN SEARCH REPORT

Application Number
EP 22 19 4915

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>A | WO 2018/051197 A1 (UNIV HONG KONG POLYTECHNIC [CN])<br>22 March 2018 (2018-03-22)<br>* claims 1, 6,7,8 *<br>* figure 39; compounds 39g, 40e *<br>----- | 1-4,6, 8-22<br><br><br>5,7 | INV.<br>C07D257/02<br>C07F5/00<br>A61K51/04 |
| | | | TECHNICAL FIELDS SEARCHED (IPC) |
| | | | C07D<br>C07F<br>A61K |

The present search report has been drawn up for all claims

1

| Place of search | Date of completion of the search | Examiner |
|---|---|---|
| Munich | 23 January 2023 | Gutke, Hans-Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 19 4915

This annex lists the patent family members relating to the patent documents cited in the above-mentioned European search report. The members are as contained in the European Patent Office EDP file on
The European Patent Office is in no way liable for these particulars which are merely given for the purpose of information.

23-01-2023

| Patent document cited in search report | Publication date | Patent family member(s) | Publication date |
|---|---|---|---|
| WO 2018051197 A1 | 22-03-2018 | CN 109963839 A | 02-07-2019 |
| | | EP 3515896 A1 | 31-07-2019 |
| | | JP 2019529439 A | 17-10-2019 |
| | | US 2021283278 A1 | 16-09-2021 |
| | | WO 2018051197 A1 | 22-03-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 199532741 A
- WO 2001082795 A
- WO 2007009638 A
- WO 2004006965 A
- WO 1997032862 A
- WO 1999005145 A
- WO 1996016677 A
- WO 1995028392 A
- WO 2013083535 A
- EP 405704 A
- US 5560903 A
- WO 2019051197 A
- WO 9731905 A


### Non-patent literature cited in the description


- **LAUFFER RB et al.** Paramagnetic metal complexes as water proton relaxation agents for NMR imaging: theory and design. *Chem Rev.,* 1987, vol. 87 (5), 901-27
- **CARAVAN P et al.** Gadolinium(III) chelates as MRI contrast agents: structure, dynamics, and applications. *Chem Rev.,* 1999, vol. 99 (9), 2293-352
- **SEALE MK et al.** *Radiographics,* 2009, vol. 29, 1725-1748
- **FIDLER J et al.** *Hepatology,* 2011, vol. 53 (2), 678-82
- **PARK Y et al.** *Korean J Radiol,* 2010, vol. 11 (4), 433-40
- **BLUEMKE DA et al.** *Radiology,* 2005, vol. 237, 89-98
- **RINGE KI et al.** *American Journal of Roentgenology,* 2010, vol. 195, 13-28
- **LEONHARDT M et al.** *Drug Metab Dispos.,* July 2010, vol. 38 (7), 1024-8
- **WEINMANN HJ et al.** *Magn Reson Med,* 1991, vol. 22, 233-237
- **ROHRER M et al.** *Invest Radiol.,* November 2005, vol. 40 (11), 715-24
- **KAHN J et al.** *Radiology.,* 2017, vol. 282 (3), 708-716
- **FRENZEL et al.** *Invest Radiol.,* December 2008, vol. 43 (12), 817-28
- Historical development of x-ray contrast media for urography and angiography. **CLAUSS W ; SPECK U et al.** Computed tomography. Springer, 1996, 1-11
- **NOWAK et al.** *Med Phys.,* 03 December 2011, vol. 8 (12), 6469-82
- Detection of Focal Liver Lesions: CT of the Hepatobiliary System with Gadoxetic Acid Disodium. *Radiology,* 1997, vol. 202, 399-405
- **S. M. BERGE et al.** Pharmaceutical Salts. *J. Pharm. Sci.,* 1977, vol. 66, 1-19
- **L. DAI ; J. ZHANG ; Y. CHEN ; L.E. MACKENZIE ; R. PAL ; G.-L. LAW.** *Inorg. Chem.,* 2019, vol. 58, 12506-12510
- **Q. YUAN ; P. XUE ; M. FANG ; E. FU ; C. WU.** *Synthetic Communications,* 2003, vol. 33 (11), 1911-1916
- **T.W. GREENE ; P.G.M. WUTS.** Protective Groups in Organic Synthesis. Wiley, 1999
- *CHEMICAL ABSTRACTS,* 72594-77-5
- *CHEMICAL ABSTRACTS,* 112-35-6
- *CHEMICAL ABSTRACTS,* 16853-85-3
- *CHEMICAL ABSTRACTS,* 7647-01-0
- *CHEMICAL ABSTRACTS,* 123-11-5
- *CHEMICAL ABSTRACTS,* 16940-66-2
- *CHEMICAL ABSTRACTS,* 603-35-0
- *CHEMICAL ABSTRACTS,* 870-50-8
- *CHEMICAL ABSTRACTS,* 6192-52-5
- *CHEMICAL ABSTRACTS,* 7440-05-3
- *CHEMICAL ABSTRACTS,* 584-08-7
- *CHEMICAL ABSTRACTS,* 64-18-6
- *CHEMICAL ABSTRACTS,* 55717-45-8
- *CHEMICAL ABSTRACTS,* 10465-78-8
- *CHEMICAL ABSTRACTS,* 200267-69-2
- **LAURENT S et al.** Stability of MRI paramagnetic contrast media: a proton relaxometric protocol for transmetallation assessment. *Invest Radiol.,* February 2001, vol. 36 (2), 115-22

- **ROHRER.** *Invest. Radiol.,* 2005, vol. 40 (11), 715-724
- Isolation of Rat Hepatocytes. **PAPELEU P. et al.** Cytochrome P450 Protocols. Methods in Molecular Biology. Humana Press, 2006, vol. 320
- The Chemistry of Contrast Agents in Medical Magnetic Resonance Imaging. Wiley, 2013
- **DORATO et al.** *Regul Toxicol Pharmacol.,* August 2005, vol. 42 (3), 265-74